(19) Europäisches Patentamt
European Patent Office
Office européen des brevets

(11) EP 3 902 021 A1

(12) EUROPEAN PATENT APPLICATION
published in accordance with Art. 153(4) EPC

(43) Date of publication:
27.10.2021 Bulletin 2021/43

(21) Application number: 19899765.2

(22) Date of filing: 13.12.2019

(51) Int Cl.:
H01L 35/24 (2006.01) H01L 35/22 (2006.01)
H01L 51/00 (2006.01) H01L 51/30 (2006.01)

(86) International application number:
PCT/JP2019/048905

(87) International publication number:
WO 2020/129836 (25.06.2020 Gazette 2020/26)

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
KH MA MD TN

(30) Priority: 18.12.2018 JP 2018236680
27.06.2019 JP 2019120137
17.10.2019 JP 2019190190

(71) Applicant: Toyo Ink SC Holdings Co., Ltd.
Tokyo 104-8377 (JP)

(72) Inventors:
- IWATA, Tohru
Tokyo 104-8377 (JP)
- KURAUCHI, Keisuke
Tokyo 104-8377 (JP)
- ANDO, Rui
Tokyo 104-8377 (JP)

(74) Representative: SSM Sandmair
Patentanwälte Rechtsanwalt
Partnerschaft mbB
Joseph-Wild-Straße 20
81829 München (DE)

# (54) THERMOELECTRIC CONVERSION MATERIAL, AND THERMOELECTRIC CONVERSION ELEMENT PREPARED THEREWITH

(57) A thermoelectric conversion material containing an electrically conductive material (A) and an organic compound (B) that are in a relationship satisfying the following formula (1): 0 eV ≤ | (HOMO of the organic compound (B)) - (HOMO of the electrically conductive material (A)) | ≤ 1.64 eV.

Figure 1

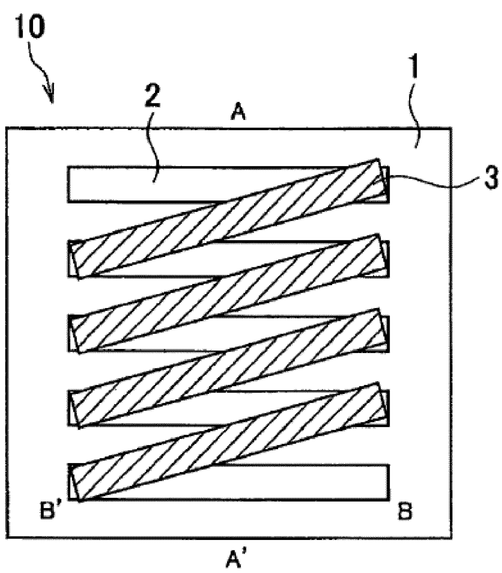




Printed by Jouve, 75001 PARIS (FR)

## Description

TECHNICAL FIELD

Embodiments of the present invention relate to a thermoelectric conversion material and a thermoelectric conversion element using the thermoelectric conversion material.

BACKGROUND ART

Thermoelectric conversion materials capable of converting thermal energy and electric energy to each other are used in thermoelectric conversion elements such as thermoelectric power generation elements and Peltier elements. Thermoelectric conversion elements are elements for converting heat into electric power and are configured with a combinations of semiconductors or a combination of metals. Typical thermoelectric conversion elements are classified into p-type semiconductor elements, n-type semiconductor elements and elements in which a p-type semiconductor and an n-type semiconductor are combined. Thermoelectric conversion elements utilize the Seebeck effect that develops electromotive force when heat is applied so that a temperature difference is generated between both ends of a semiconductor. In order to obtain a larger potential difference, in general, a p-type semiconductor and an n-type semiconductor are used in combination as materials for thermoelectric conversion elements.

Thermoelectric conversion elements are used as thermoelectric modules which are formed by combining a large number of elements into a plate shape or a cylinder shape. The thermal energy can be directly converted into electric power, and thermoelectric conversion elements can be used, for example, as power source in wristwatches operated by body temperature, power sources for power generation for ground use and powers source for power generation for artificial satellites. The performance of the thermoelectric conversion element depends on the performance of the thermoelectric conversion material, the durability of the module, and the like.

As described in Non-Patent Document 1, a dimensionless thermoelectric figure of merit (ZT) is used as an index representing the performance of a thermoelectric conversion material. A power factor PF ($=S^2 \cdot \sigma$) may be used as an index representing the performance of a thermoelectric conversion material.

The dimensionless thermoelectric figure of merit "ZT" is expressed by the following expression (A).

$$ZT=(S^2 \cdot \sigma \cdot T)/\kappa \qquad (A)$$

Here, S is the Seebeck coefficient (V/K), $\sigma$ is the electrical conductivity (S· m), T is the absolute temperature (K), and $\kappa$ is the thermal conductivity (W/(m·K)). The thermal conductivity $\kappa$ is expressed by the following expression (B).

$$\kappa=\alpha \cdot \rho \cdot C \qquad (B)$$

Here, $\alpha$ is the thermal diffusivity ($m^2/s$), $\rho$ is the density ($kg/m^3$), and C is the specific heat capacity (J/(kg· K)).

Accordingly, in order to improve the performance of the thermoelectric conversion (hereafter, may also be referred to as thermoelectric properties), it is important to improve the Seebeck coefficient or the electrical conductivity, and also to lower the thermal conductivity.

As typical thermoelectric conversion materials, inorganic materials such as bismuth-tellurium based (Bi-Te based) materials as materials for the temperature of from the ordinary temperature to 500 K, lead-tellurium based (Pb-Te based) materials as materials for the temperature of from the ordinary temperature to 800 K, and silicon-germanium based (Si-Ge based) materials as materials for the temperature of from the ordinary temperature to 1000 K are known.

However, a thermoelectric conversion material containing such an inorganic material may often contain a rare element, and thus may often be costly, or may contain a hazardous material. Further, since inorganic materials have poor workability, they complicate the manufacturing process. Therefore, the manufacturing energy and the manufacturing cost for the thermoelectric conversion material containing an inorganic material increase, and a wide use of such thermoelectric conversion material is difficult to be achieved. Further, since inorganic materials are rigid, it is difficult to form a flexible thermoelectric conversion element which can be installed in a shape other than a plane.

On the other hand, a thermoelectric conversion element using an organic material instead of a conventional inorganic material has been studied. Since organic materials have excellent moldability, and have flexibility superior to that of inorganic materials, organic materials have high versatility in a temperature range where the organic material itself does not decompose. In addition, since printing technology and the like can be easily utilized, organic materials are more advantageous than inorganic materials in terms of the manufacturing energy and the manufacturing cost.

For example, Patent Document 1 discloses a thermoelectric material which is obtained by including, in the

material, together with carbon nanotubes (CNT), a polymer dispersant having an organic coloring matter skeleton attached thereto, and which has good CNT dispersibility, is suitable for a coating method, and exhibits excellent thermoelectromotive force. Patent Document 2 discloses a thermoelectric conversion material in which a porphyrin skeleton and a substituent containing an alkyl group are bonded, and which has a high Seebeck coefficient. However, in the thermoelectric conversion element of Patent Document 1, the polymer chain of the polymer dispersant inhibits the interaction with the CNT, and sufficient performance is not obtained. Further, the thermoelectric conversion element disclosed in Patent Document 2 has a low electrical conductivity of $10^{-8}$ to $10^{-7}$ S/cm and a practical value as a thermoelectric conversion element cannot be obtained.

CITATION LIST

PATENT DOCUMENTS

Patent Document 1: WO 2015/050113
Patent Document 2: WO 2015/129877

NON-PATENT DOCUMENTS

Non-Patent Document 1: "Handbook of Thermoelectric Conversion Technology (first edition)" by Takenobu Kajikawa, NTS Inc., p. 19

SUMMARY OF INVENTION

TECHNICAL PROBLEM

It is an object of the present invention to provide a thermoelectric conversion material that achieves compatibility between a Seebeck coefficient and electroconductive properties and exhibits a high power factor. It is also an object of the present invention to provide a thermoelectric conversion element that exhibits excellent thermoelectric performance, using the material.

SOLUTION TO PROBLEM

As a result of intensive research, the inventors of the present invention discovered that the problem was able to be solved using a thermoelectric conversion material described below, thus enabling the inventors to complete the present invention.

That is, an embodiment of the present invention relates to a thermoelectric conversion material containing at least one electrically conductive material selected from the group consisting of a carbon material, a metal material and an electrically conductive polymer, and an organic compound that is different from the electrically conductive material, wherein the electrically conductive material and the organic compound satisfy the following expression (1).

Expression (1)

$$0\ \text{eV} \leq |\text{(HOMO of the organic compound)} - \text{(HOMO of the electrically conductive material)}| \leq 1.64\ \text{eV}$$

In Expression (1), HOMO represents an energy level of a highest occupied molecular orbital, provided that in a case in which the electrically conductive material is a metal material, the HOMO of the conductive material represents a Fermi level of the electrically conductive material.

Another embodiment of the present invention relates to a thermoelectric conversion material containing an electrically conductive material, a first organic compound that is different from the electrically conductive material, and a second organic compound that is different from the electrically conductive material and that is different from the first organic compound, wherein the thermoelectric conversion material satisfies all of the following (1) to (3).

(1) 0 < ((HOMO of the first organic compound) - (HOMO of the electrically conductive material)) × ((HOMO of the second organic compound) - (HOMO of the electrically conductive material))

(2) | (HOMO of the first organic compound) - (HOMO of the electrically conductive material) | < | (HOMO of the second organic compound) - (HOMO of the electrically conductive material) |
(3) An adsorptivity of the first organic compound to the electrically conductive material is greater than an adsorptivity of the second organic compound to the electrically conductive material.

In (1) to (3) above, HOMO represents an energy level of a highest occupied molecular orbital, provided that in a case in which the electrically conductive material is a metal material, the HOMO of the electrically conductive material represents a Fermi level of the electrically conductive material.

Another embodiment of the present invention relates to a thermoelectric conversion element having a thermoelectric conversion film containing the thermoelectric conversion material as described above and an electrode, wherein the thermoelectric conversion film and the electrode are electrically connected to each other.

ADVANTAGEOUS EFFECTS OF INVENTION

An embodiments of the present invention is able to provide a thermoelectric conversion material that achieves compatibility between a Seebeck coefficient and electroconductive properties. An embodiments of the present invention is able to provide a thermoelectric conversion element that exhibits excellent thermoelectric performance using the material.

BRIEF DESCRIPTION OF DRAWINGS

FIG. 1 is a schematic diagram showing a structure of an example of a thermoelectric conversion element.
FIG. 2 is a schematic diagram illustrating a method of measuring the electromotive force of a thermoelectric conversion element.

DESCRIPTION OF EMBODIMENTS

Embodiments of the present invention are as follows.

[1] A thermoelectric conversion material containing at least one electrically conductive material selected from the group consisting of a carbon material, a metal material and an electrically conductive polymer, and an organic compound that is different from the electrically conductive material, wherein the electrically conductive material and the organic compound satisfy the following expression (1).

Expression (1)

$$0\ \text{eV} \leq |\ (\text{HOMO of the organic compound}) - (\text{HOMO of the electrically conductive material})\ | \leq 1.64\ \text{eV}$$

In expression (1), HOMO represents an energy level of a highest occupied molecular orbital, provided that in a case in which the electrically conductive material is a metal material, the HOMO of the electrically conductive material represents a Fermi level of the electrically conductive material.
[2] The thermoelectric conversion material according to [1], wherein the HOMO of the electrically conductive material is an energy level higher than the HOMO of the organic compound.
[3] The thermoelectric conversion material according to [1] or [2], wherein an amount of the organic compound is not more than 400% by mass relative a total mass of the electrically conductive material.
[4] The thermoelectric conversion material according to any one of [1] to [3], wherein the organic compound is a compound having any one selected from the group consisting of a perylene skeleton, a pyrrolopyrrole skeleton, a thiazolothiazole skeleton, an oxazolothiazole skeleton, an oxazolooxazole skeleton, a benzobisthiazole skeleton, a benzobisoxazole skeleton, a thiazolobenzoxazole skeleton, a thioxanthone skeleton, a phenothiazine skeleton, and a phenanthroline skeleton, provided that the organic compound is different from a compound having a perylene carbodiimide skeleton and is different from a compound represented by the following structural formula X.

Structural Formula X

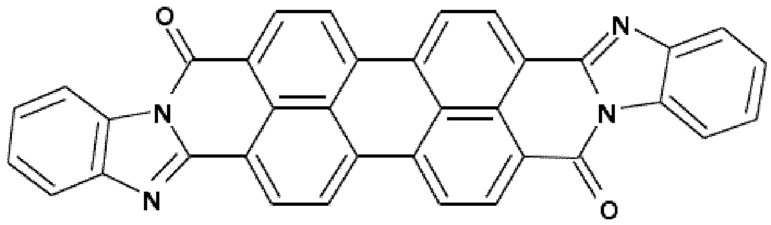

[5] The thermoelectric conversion material according to any one of [1] to [4], wherein the organic compound is a compound represented by any one selected from the group consisting of the following general formulae (1) to (8).

General Formula (1)

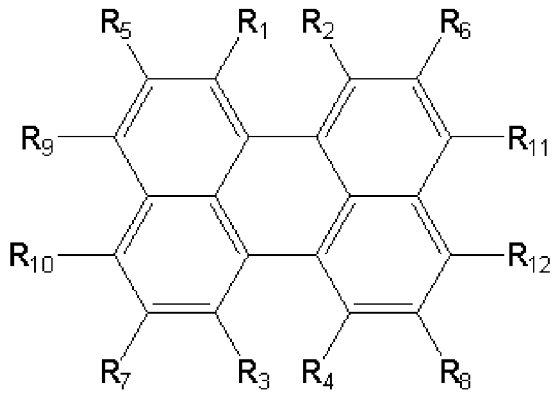

In general formula (1), each of $R_1$ to $R_{12}$ independently represents any one selected from the group consisting of a hydrogen atom, a halogen atom, a cyano group, a nitro group, a substituted or unsubstituted alkyl group, a substituted or unsubstituted alkoxy group, a substituted or unsubstituted aryloxy group, a substituted or unsubstituted alkylthio group, a substituted or unsubstituted arylthio group, a substituted or unsubstituted aryl group, a substituted or unsubstituted heterocyclic group, and a substituted or unsubstituted amino group, and adjacent two groups of $R_1$ to $R_{12}$ may be bonded to each other to form a ring.

General Formula (2)

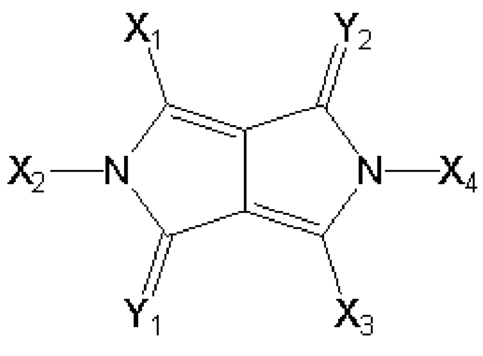

In general formula (2), each of $X_1$ to $X_4$ independently represents any one selected from the group consisting of a hydrogen atom, a substituted or unsubstituted alkyl group, a substituted or unsubstituted aryl group, and a substituted or unsubstituted heterocyclic group, and each of $Y_1$ and $Y_2$ independently represents any one selected from the group consisting of an oxygen atom, a sulfur atom, and a dicyanomethylene group.

General Formula (3)

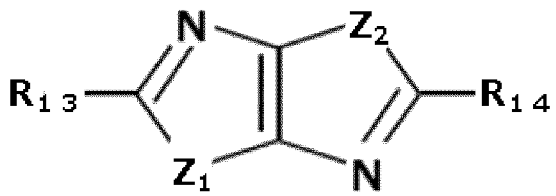

In general formula (3), each of $Z_1$ and $Z_2$ independently represents any one selected from the group consisting of an oxygen atom and a sulfur atom, and each of $R_{13}$ and $R_{14}$ independently represents any one selected from the group consisting of a hydrogen atom, a halogen atom, a cyano group, a nitro group, a carboxyl group, a substituted or unsubstituted alkyl group, a substituted or unsubstituted alkoxy group, a substituted or unsubstituted aryloxy group, a substituted or unsubstituted alkylthio group, a substituted or unsubstituted arylthio group, a substituted or unsubstituted aryl group, a substituted or unsubstituted heterocyclic group, and a substituted or unsubstituted amino group.

General Formula (4)

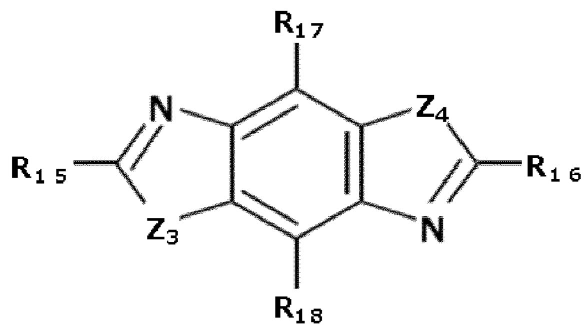

In general formula (4), each of $Z_3$ and $Z_4$ independently represents any one selected from the group consisting of an oxygen atom and a sulfur atom. Each of $R_{15}$ to $R_{18}$ independently represents any selected from the group consisting of a hydrogen atom, a halogen atom, a cyano group, a nitro group, a carboxyl group, a substituted or unsubstituted alkyl group, a substituted or unsubstituted alkoxy group, a substituted or unsubstituted aryloxy group, a substituted or unsubstituted alkylthio group, a substituted or unsubstituted arylthio group, a substituted or unsubstituted aryl group, a substituted or unsubstituted heterocyclic group, and a substituted or unsubstituted amino group.

General Formula (5)

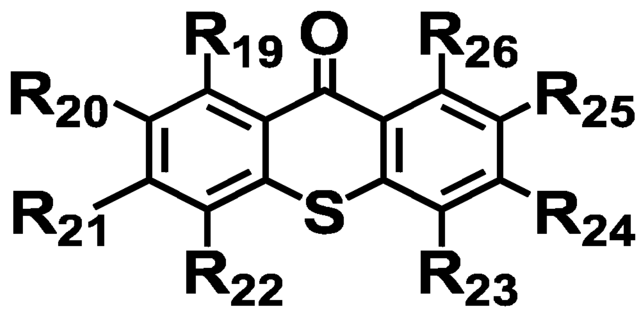

In general formula (5), each of $R_{19}$ to $R_{26}$ independently represents any one selected from the group consisting of a hydrogen atom, a halogen atom, a hydroxyl group, a sulfanyl group, a cyano group, a nitro group, a carboxyl group, an alkoxycarbonyl group, an acyl group, a substituted or unsubstituted alkyl group, a substituted or unsubstituted alkoxy group, a substituted or unsubstituted aryloxy group, a substituted or unsubstituted alkylthio group, a substituted or unsubstituted arylthio group, a substituted or unsubstituted aryl group, a substituted or unsubstituted heterocyclic group, a substituted or unsubstituted amino group, and a substituted or unsubstituted acyloxy group, provided that at least one of $R_{19}$ to $R_{26}$ is other than a hydrogen atom.

General Formula (6)

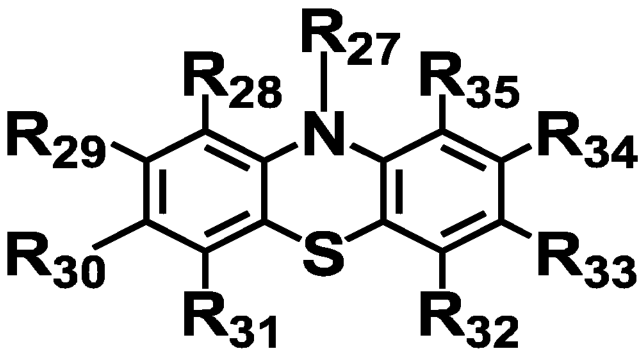

General Formula (7)

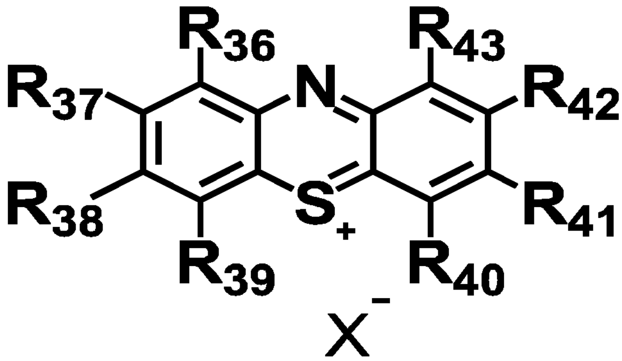

In general formula (6), $R_{27}$ represents any one selected from the group consisting of a hydrogen atom, a substituted or unsubstituted alkyl group, a substituted or unsubstituted aryl group, and a substituted or unsubstituted heterocyclic group. In general formulae (6) and (7), each of $R_{28}$ to $R_{43}$ independently represents any one selected from the group consisting of a hydrogen atom, a halogen atom, a hydroxyl group, a cyano group, a nitro group, a carboxyl group, a substituted or unsubstituted alkyl group, a substituted or unsubstituted alkoxy group, a substituted or unsubstituted aryloxy group, a substituted or unsubstituted alkylthio group, a substituted or unsubstituted arylthio group, a substituted or unsubstituted aryl group, a substituted or unsubstituted heterocyclic group, and a substituted or unsubstituted amino group. In general formula (7), $X^-$ represents an anion.

General Formula (8)

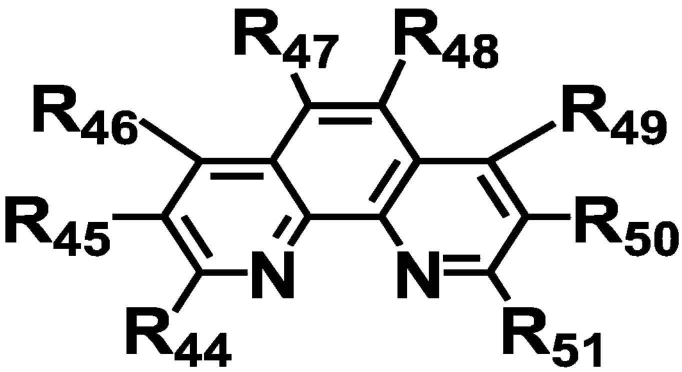

In general formula (8), each of $R_{44}$ to $R_{51}$ independently represents any one selected from the group consisting of a hydrogen atom, a halogen atom, a hydroxyl group, a cyano group, a nitro group, a carboxyl group, a sodium sulfonato group, a substituted or unsubstituted alkyl group, a substituted or unsubstituted alkoxy group, a substituted or unsubstituted aryloxy group, a substituted or unsubstituted alkylthio group, a substituted or unsubstituted arylthio group, a substituted or unsubstituted aryl group, a substituted or unsubstituted heterocyclic group, and a substituted or unsubstituted amino group. Adjacent two groups of $R_{44}$ to $R_{51}$ may be bonded to each other to form a ring.

[6] The thermoelectric conversion material according to any one of [1] to [5], wherein the electrically conductive material contains at least one selected from the group consisting of a carbon nanotube, Ketjen black, a graphene nanoplate, and graphene.

[7] The thermoelectric conversion material according to any one of [1] to [6], wherein the electrically conductive material is a carbon nanotube.

[8] A thermoelectric conversion material containing an electrically conductive material, a first organic compound that is different from the electrically conductive material, and a second organic compound that is different from the electrically conductive material and that is different from the first organic material, wherein the thermoelectric conversion material satisfies all of the following (1) to (3).

(1) 0 < ((HOMO of the first organic compound) - (HOMO of the electrically conductive material)) × ((HOMO of the second organic compound) - (HOMO of the electrically conductive material))
(2) | (HOMO of the first organic compound) - (HOMO of the electrically conductive material) | < | (HOMO of the second organic compound) - (HOMO of the electrically conductive material) |
(3) An adsorptivity of the first organic compound to the electrically conductive material is greater than an adsorptivity of the second organic compound to the electrically conductive material.

In (1) to (3) above, HOMO represents an energy level of a highest occupied orbital, provided that in a case in which the electrically conductive material is a metal material, the HOMO of the electrically conductive material represents a Fermi level of the electrically conductive material.

[9] The thermoelectric conversion material according to [8], wherein the electrically conductive material comprises a carbon material.

[10] The thermoelectric conversion material according to [9], wherein the carbon material comprises a carbon nanotube.

[11] A thermoelectric conversion element including a thermoelectric conversion film containing the thermoelectric conversion material according to any one of [1] to [10], and an electrode, wherein the thermoelectric conversion film and the electrode are electrically connected to each other.

(First Embodiment)

The thermoelectric conversion material of this embodiment contains an electrically conductive material (A) and an organic compound (B) for which the value | (HOMO of the organic compound (B)) - (HOMO of the electrically conductive material (A)) | (hereafter also referred to as ΔHOMO) is small. Using such a specific combination, compatibility between a high Seebeck coefficient and the electroconductive properties can be achieved, and excellent thermoelectric performance can be exhibited. This is because holes (careers) efficiently move from the organic compound having a small thermal excitation energy to the electrically conductive material, and these holes move within the electrically conductive material, thereby achieving a high Seebeck coefficient and a high electrical conductivity are achieved.

<Electrically Conductive Material (A)>

The electrically conductive material (A) contributes to improvement in electroconductive properties. The electroconductive properties can be improved by increasing the amount of the electrically conductive material (A).

The electrically conductive material (A) is not particularly limited as long as it is a material having electroconductive properties (a carbon material, a metal material, an electrically conductive polymer, and the like). Examples of the carbon materials include graphite, carbon nanotubes, carbon black (examples thereof including Ketjen black), and graphene (examples thereof including graphene nanoplates). Examples of the metal materials include metal powders such as a powder of, for example, gold, silver, copper, nickel, chromium, palladium, rhodium, ruthenium, indium, silicon, aluminum, tungsten, molybdenum, germanium, gallium, platinum or the like, ZnSe, CdS, InP, GaN, SiC, SiGe, alloys thereof, and composite powders thereof. Examples further include microparticles including a core and a material that is different from the core material and that coats the core, and specific examples include silver-coated copper powders in which copper is used as a core and the surface thereof is coated with silver. Examples further include metal oxide powders such as a powder of, for example, silver oxide, indium oxide, tin oxide, zinc oxide, ruthenium oxide, ITO (tin-doped indium oxide), AZO (aluminum-doped zinc oxide), GZO (gallium-doped zinc oxide) or the like, and a powder in which the surface is coated with any of these metal oxides. Examples of the electrically conductive polymers include PEDOT/PSS (a composite composed of poly(3,4-ethylenedioxythiophene) and polystyrene sulfonate), polyaniline, polyacetylene, polypyrrole, polythiophene, and polyparaphenylene.

One electrically conductive material may be used singly, or two or more electrically conductive materials may

be used in combination.

The shape of the electrically conductive material (A) is not particularly limited, and for example, an irregular shape, an aggregated shape, a scaly shape, a microcrystalline shape, a spherical shape, a flaky shape, a wire shape, or the like can be appropriately used.

From the viewpoint of the compatibility between the Seebeck coefficient and the electrical conductivity, the electrically conductive material (A) preferably contains a carbon material. The carbon material is preferably a carbon nanotube, carbon black or graphene, more preferably a carbon nanotube, and particularly preferably a single-walled carbon nanotube.

With respect to the graphite, examples of graphite having a scaly shape include CMX, UP-5, UP-10, UP-20, UP-35N, CSSP, CSPE, CSP, CP, CB-150, CB-100, ACP, ACP-1000, ACB-50, ACB-100, ACB-150, SP-10, SP-20, J-SP, SP-270, HOP, GR-60, LEP, F#1, F#2 and F#3 manufactured by Nippon Graphite Industries, Co. Ltd., BF-3AK, FBF, BF-15AK, CBR, CPB-6S, CPB-3, 96L, 96L-3, K-3, SC-120, SC-60, HLP, CP-150 and SB-1 manufactured by Chuetsu Graphite Works Co., Ltd., EC1500, EC1000, EC500, EC300, EC100 and EC50 manufactured by Ito Graphite Co., Ltd., and 10099M and PB-99 manufactured by Nishimura Graphite Co., Ltd. Examples of natural graphite having a spherical shape include CGC-20, CGC-50, CGB-20 and CGB-50 manufactured by Nippon Graphite Industries, Co., Ltd. Examples of earthy graphite include Blue P, AP, AOP and P#1 manufactured by Nippon Graphite Industries, Co., Ltd., and APR, K-5, AP-2000, AP-6, 300F and 150F manufactured by Chuetsu Graphite Works Co., Ltd. Examples of artificial graphite include PAG-60, PAG-80, PAG-120, PAG-5, HAG-10W and HAG-150 manufactured by Nippon Graphite Industries, Co., Ltd., G-4AK, G-6S, G-3G-150, G-30, G-80, G-50, SMF, EMF, SFF, SFF-80B, SS-100, BSP-15AK, BSP-100AK and WF-15C manufactured by Chuetsu Graphite Works Co., Ltd., and SGP-100, SGP-50, SGP-25, SGP-15, SGP-5, SGP-1, SGO-100, SGO-50, SGO-25, SGO-15, SGO-5, SGO-1, SGX-100, SGX-50, SGX-25, SGX-15, SGX-5 and SGX-1 manufactured by SEC Carbon, Limited.

Examples of electrically conductive carbon fibers and examples of carbon nanotubes include vapor-phase carbon fibers such as VGCF manufactured by Showa Denko K.K., EC1.5 and EC1.5-P manufactured by Meijo Nano Carbon Co., Ltd., TUBALL manufactured by Kusumoto Chemicals, Ltd., single-walled carbon nanotubes such as ZEONANO manufactured by Zeon Nano Technology, Co., Ltd., FloTube 9000, FloTube 7000 and FloTube 2000 manufactured by CNano, NC 7000 manufactured by Nanocyl, and 100T and 200P manufactured by Knano.

Examples of carbon blacks include furnace blacks, such as TOKABLACK #4300, TOKABLACK #4400, TOKABLACK #4500 and TOKABLACK #5500 manufactured by Tokai Carbon Co., Ltd., PRINTEX L manufactured by Degussa, Raven 7000, Raven 5750, Raven 5250, Raven 5000 ULTRA III, Raven 5000 ULTRA, Conductex SC ULTRA, Conductex 975 ULTRA, PUERBLACK 100, PUERBLACK 115 and PUERBLACK 205 manufactured by Columbian, #2350, #2400B, #2600B, #3050B, #3030B, #3230B, #3350B, #3400B and #5400B manufactured by Mitsubishi Chemical Corporation, MONARCH 1400, MONARCH 1300, MONARCH 900, Vulcan XC-72R and Black Pearls 2000 manufactured by Cabot, Ensaco 250G, Ensaco 260G, Ensaco 350G and Super P-Li manufactured by TIMCAL; Ketjen blacks, such as EC-300J and EC-600JD manufactured by Lion Corporation; and acetylene blacks, such as Denka Black, Denka Black HS-100 and Denka Black FX-35 manufactured by Denka Company Limited. The carbon black is not particularly limited to these products, and one carbon black may be used singly, or two or more carbon blacks may be used in combination.

<Organic Compound (B)>

The organic compound (B) is not particularly limited as long as it satisfies the following expression (1) and is different from the electrically conductive material (A). The organic compound (B) can be selected from known organic compounds.

Expression (1)

$$0\ \text{eV} \leq |(\text{HOMO of the organic compound (B)}) - (\text{HOMO of the electrically conductive material (A)})| \leq 1.64\ \text{eV}$$

In expression (1), HOMO represents the energy level of the highest occupied orbital, provided that in a case in which the electrically conductive material (A) is a metal material, the HOMO of the electrically conductive material (A) represents the Fermi level of the electrically conductive material (A).

The mechanism of thermoelectric conversion is suggested as follows.

A hole is generated in the thermally excited organic compound (B), and moves to the electrically conductive material (A), and a potential difference is generated in the electrically conductive material (A), causing an electric current to flows. Therefore, when the electrically conductive material (A) and the organic compound (B) satisfy the expression (1), the value of HOMO of the organic compound (B) and the value of HOMO of the electrically conductive material (A)

become close to each other, and the hole movement efficiently occurs from the organic compound (B) to the electrically conductive material (A). Accordingly, the potential difference in the electrically conductive material (A) becomes larger, and the Seebeck coefficient is improved.

When the HOMO of the electrically conductive material (A) is an energy level higher than the HOMO of the organic compound (B), the hole movement from the organic compound (B) to the electrically conductive material (A) occurs more efficiently. Therefore, the HOMO of the electrically conductive material (A) is preferably an energy level higher than the HOMO of the organic compound (B).

The efficiency of the hole movement in the above described mechanism is related to the intermolecular distance between the electrically conductive material (A) and the organic compound (B), and the intermolecular distance is preferably closer, that is, the affinity between the two is preferably excellent. For example, for those having a $\pi$ plane, such as CNT, compounds containing an aromatic ring, a heterocyclic ring, or an acidic functional group are preferable, and, for metals such as silver, compounds containing an acidic functional group, a basic functional group, a heterocyclic ring, or a metal coordination skeleton are preferable.

As described above, the organic compound (B) functions to generate holes (careers) upon thermal excitation. Therefore, a material which is easily thermally excited, that is, a material in which the band gap (the energy difference between HOMO-LUMO (highest occupied orbital - lowest empty orbital)) is small is preferable. The band gap of the organic compound (B) is preferably not more than 2.5 eV, more preferably not more than 1.5 eV, and particularly preferably 1.0 eV or lower.

The organic compound (B) contributes to the improvement of the Seebeck coefficient in the thermoelectric conversion material. The Seebeck coefficient can be improved by increasing the amount of the organic compound (B). However, in a case in which the amount of the organic compound (B) increases, the insulation properties increase, meaning the electroconductive properties decrease. Accordingly, from the viewpoint of the compatibility between the Seebeck coefficient and the electrical conductivity, the amount of the organic compound (B), relative to the total mass of the electrically conductive material (A), is preferably not more than 400% by mass, more preferably not more than 200% by mass, still more preferably from 3 to 120% by mass, and particularly preferably from 5 to 100% by mass.

From the viewpoint of promoting surface adsorption on the electrically conductive material (A) and uniformity, and further increasing the molecular ratio, the molecular weight of the organic compound (B) is preferably small, and the mass-average molecular weight (Mw) of the organic compound (B) is preferably not more than 2,000, and more preferably 1,000 or less.

The organic compound (B) satisfying the condition as described above is preferably a compound having any one of a perylene skeleton, a pyrrolopyrrole skeleton, a thiazolothiazole skeleton, an oxazolothiazole skeleton, an oxazolooxazole skeleton, a benzobisthiazole skeleton, a benzobisoxazole skeleton, a thiazolobenzoxazole skeleton, a thioxanthone skeleton, a phenothiazine skeleton, and a phenanthroline skeleton, and is preferably a compound represented by any one of the following general formulae (1) to (8); provided that, the organic compound (B) is different from a compound having a perylene carbodiimide skeleton and is different from a compound represented by the structural formula X as described above.

General Formula (1)

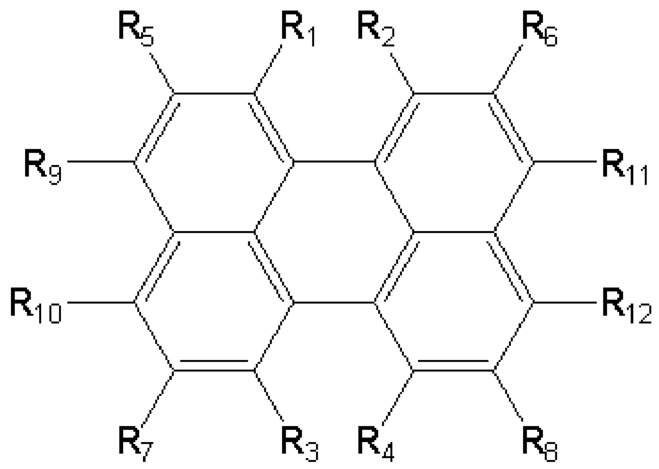

In general formula (1), each of $R_1$ to $R_{12}$ is independently any one selected from the group consisting of a hydrogen atom, a halogen atom, a cyano group, a nitro group, a substituted or unsubstituted alkyl group, a substituted or unsubstituted alkoxy group, a substituted or unsubstituted aryloxy group, a substituted or unsubstituted alkylthio group,

a substituted or unsubstituted arylthio group, a substituted or unsubstituted aryl group, a substituted or unsubstituted heterocyclic group, and a substituted or unsubstituted amino group, and adjacent two groups of $R_1$ to $R_{12}$ may be bonded to each other to form a ring.

General Formula (2)

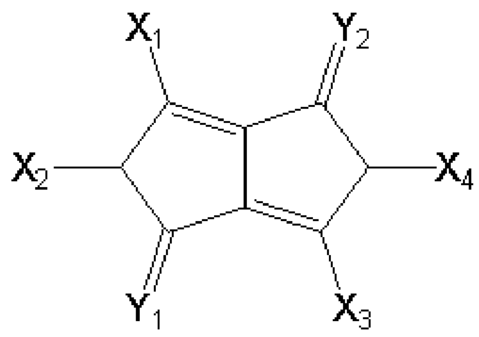

In general formula (2), each of $X_1$ to $X_4$ independently represents any one selected from the group consisting of a hydrogen atom, a substituted or unsubstituted alkyl group, a substituted or unsubstituted aryl group, and a substituted or unsubstituted heterocyclic group, and each of $Y_1$ and $Y_2$ independently represents any one selected from the group consisting of an oxygen atom, a sulfur atom, and a dicyanomethylene group.

General Formula (3)

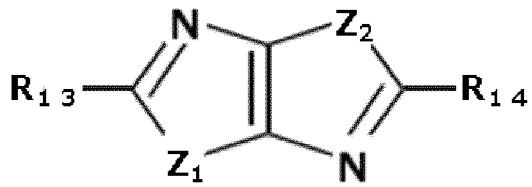

In general formula (3), each of $Z_1$ and $Z_2$ independently represents any one selected from the group consisting of an oxygen atom and a sulfur atom, and each of $R_{13}$ and $R_{14}$ independently represents any one selected from the group consisting of a hydrogen atom, a halogen atom, a cyano group, a nitro group, a carboxyl group, a substituted or unsubstituted alkyl group, a substituted or unsubstituted alkoxy group, a substituted or unsubstituted aryloxy group, a substituted or unsubstituted alkylthio group, a substituted or unsubstituted arylthio group, a substituted or unsubstituted aryl group, a substituted or unsubstituted heterocyclic group, and a substituted or unsubstituted amino group.

General Formula (4)

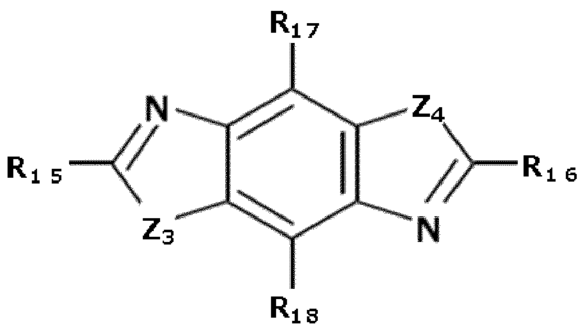

In general formula (4), each of $Z_3$ and $Z_4$ independently represents any one selected from the group consisting of an oxygen atom and a sulfur atom. Each of $R_{15}$ to $R_{18}$ independently represents any one selected from the group consisting of a hydrogen atom, a halogen atom, a cyano group, a nitro group, a carboxyl group, a substituted or unsubstituted alkyl group, a substituted or unsubstituted alkoxy group, a substituted or unsubstituted aryloxy group, a substituted or unsubstituted alkylthio group, a substituted or unsubstituted arylthio group, a substituted or unsubstituted aryl group,

a substituted or unsubstituted heterocyclic group, and a substituted or unsubstituted amino group.

General Formula (5)

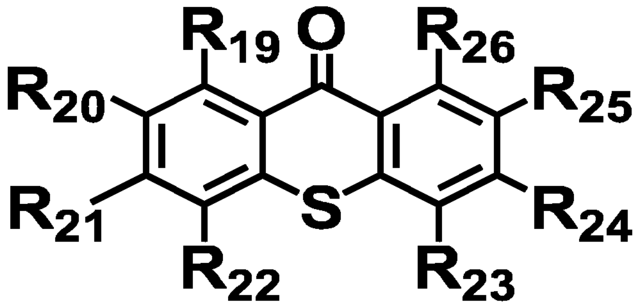

In general formula (5), each of $R_{19}$ to $R_{26}$ independently represents any one selected from the group consisting of a hydrogen atom, a halogen atom, a hydroxyl group, a sulfanyl group, a cyano group, a nitro group, a carboxyl group, an alkoxycarbonyl group, an acyl group, a substituted or unsubstituted alkyl group, a substituted or unsubstituted alkoxy group, a substituted or unsubstituted aryloxy group, a substituted or unsubstituted alkylthio group, a substituted or unsubstituted arylthio group, a substituted or unsubstituted aryl group, a substituted or unsubstituted heterocyclic group, a substituted or unsubstituted amino group, and a substituted or unsubstituted acyloxy group, provided that at least one of $R_{19}$ to $R_{26}$ is other than a hydrogen atom.

General Formula (6)

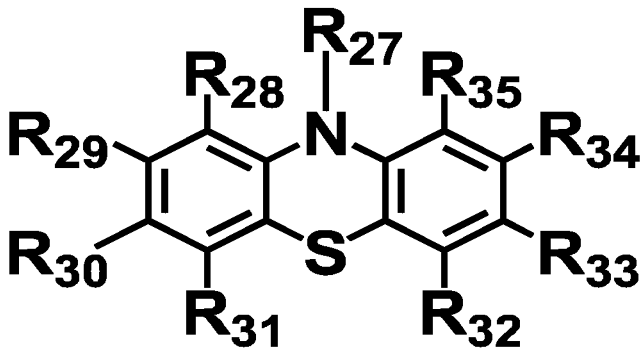

General Formula (7)

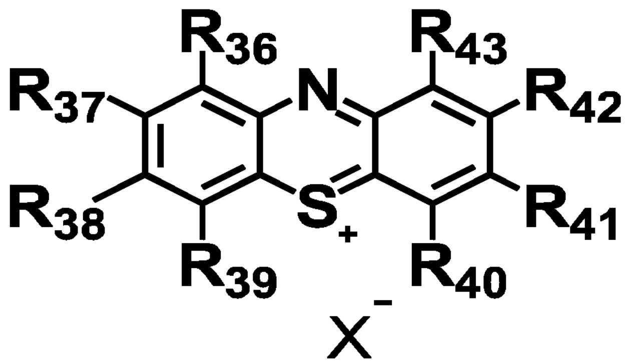

In general formula (6), $R_{27}$ represents any one selected from the group consisting of a hydrogen atom, a substituted or unsubstituted alkyl group, a substituted or unsubstituted aryl group, and a substituted or unsubstituted heterocyclic group. In general formulae (6) and (7), each of $R_{28}$ to $R_{43}$ independently represents any one selected from the group consisting of a hydrogen atom, a halogen atom, a hydroxyl group, a cyano group, a nitro group, a carboxyl group, a substituted or unsubstituted alkyl group, a substituted or unsubstituted alkoxy group, a substituted or unsubstituted aryloxy group, a substituted or unsubstituted alkylthio group, a substituted or unsubstituted arylthio group, a substituted or unsubstituted aryl group, a substituted or unsubstituted heterocyclic group, and a substituted or unsubstituted amino group. In general formula (7), $X^-$ represents an anion.

General Formula (8)

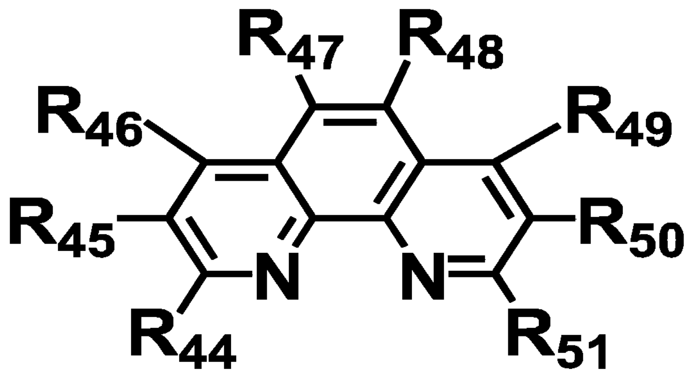

In general formula (8), each of $R_{44}$ to $R_{51}$ independently represents any one selected from the group consisting of a hydrogen atom, a halogen atom, a hydroxyl group, a cyano group, a nitro group, a carboxyl group, a sodium sulfonato group, a substituted or unsubstituted alkyl group, a substituted or unsubstituted alkoxy group, a substituted or unsubstituted aryloxy group, a substituted or unsubstituted alkylthio group, a substituted or unsubstituted arylthio group, a substituted or unsubstituted aryl group, a substituted or unsubstituted heterocyclic group, and a substituted or unsubstituted amino group. Adjacent two groups of $R_{44}$ to $R_{51}$ may be bonded to each other to form a ring.

Here, $R_1$ to $R_{51}$ in general formulae (1) to (8) are described.

Examples of the halogen atom include a fluorine atom, a chlorine atom, a bromine atom, and an iodine atom.

Examples of the substituted or unsubstituted alkyl groups include unsubstituted alkyl groups having 1 to 30 carbon atoms, such as a methyl group, an ethyl group, a propyl group, a butyl group, a sec-butyl group, a tert-butyl group, a pentyl group, a hexyl group, a heptyl group, an octyl group, and a stearyl group; substituted alkyl groups having 1 to 30 carbon atoms, such as a 2-phenylisopropyl group, a trichloromethyl group, a trifluoromethyl group, a benzyl group, an α-phenoxybenzyl group, an α,α-dimethylbenzyl group, an α,α-methylphenylbenzyl group, an α,α-bis(trifluoromethyl)benzyl group, a triphenylmethyl group, and an α-benzyloxybenzyl group; and unsubstituted cycloalkyl groups, such as a cyclopentyl group and a cyclohexyl group.

Examples of the substituted or unsubstituted alkoxy groups include unsubstituted alkoxy groups having 1 to 20 carbon atoms, such as a methoxy group, an ethoxy group, a propoxy group, a butoxy group, a tert-butoxy group, an octyloxy group, and a tert-octyloxy group; and substituted alkoxy groups having 1 to 20 carbon atom, such as a 3,3,3-trifluoroethoxy group and a benzyloxy group.

Examples of the substituted or unsubstituted aryloxy groups include unsubstituted aryloxy groups having 6 to 20 carbon atoms, such as a phenoxy group, a 1-naphthyloxy group, a 2-naphthyloxy group, and a 9-anthryloxy group; and substituted aryloxy groups having 6 to 20 carbon atoms, such as a 4-tert-butylphenoxy group, a 4-nitrophenoxy group, a 3-fluorophenoxy group, a pentafluorophenoxy group, and a 3-trifluoromethylphenoxy group.

Examples of the substituted or unsubstituted alkylthio groups include unsubstituted alkylthio groups having 1 to 20 carbon atoms, such as a methylthio group, an ethylthio group, a tert-butylthio group, a hexylthio group, and an octylthio group; and substituted alkylthio groups having 1 to 20 carbon atoms, such as a 1,1,1-tetrafluoroethylthio group, a benzylthio group, and a trifluoromethylthio group.

Examples of the substituted or unsubstituted arylthio groups include unsubstituted arylthio groups having 6 to 20 carbon atoms, such as a phenylthio group; and substituted arylthio groups having 6 to 20 carbon atoms, such as a 2-methylphenylthio group, a 4-tert-butylphenylthio group, a 3-fluorophenylthio group, a pentafluorophenylthio group, and a 3-trifluoromethylphenylthio group.

Examples of the substituted or unsubstituted aryl groups include unsubstituted aryl groups having 6 to 30 carbon atoms, such as a phenyl group, o-tolyl group, m-tolyl group, p-tolyl group, 2,4-xylyl group, p-coumenyl group, mesityl group, 1-naphthyl group, 2-naphthyl group, 1-anthryl group, 9-phenanthryl group, 1-acenaphthyl group, 2-azulenyl group, 1-pyrenyl group, and 2-triphenylenyl group; and substituted aryl groups having 6 to 30 carbon atoms, such as a p-cyanophenyl group, a p-diphenylaminophenyl group, a p-styrylphenyl group, a 4-[(2-tolyl)ethenyl]phenyl group, and a 4-[(2,2-ditolyl)ethenyl]phenyl group.

Examples of the substituted or unsubstituted heterocyclic groups include unsubstituted aromatic heterocyclic groups having 3 to 20 carbon atoms, such as a 2-furyl group, a 3-furyl group, a 2-thienyl group, a 3-thienyl group, a 1-pyrrolyl group, a 2-pyrrolyl group, a 3-pyrrolyl group, a 2-pyridyl group, a 3-pyridyl group, a 4-pyridyl group, a 2-pyrazyl group, a 2-oxazolyl group, a 3-isoxazolyl group, a 2-thiazolyl group, a 3-isothiazolyl group, a 2-imidazolyl group, a 3-pyrazolyl group, a 2-quinolyl group, a 3-quinolyl group, a 4-quinolyl group, a 5-quinolyl group, a 6-quinolyl group, a 7-quinolyl group, a 8-quinolyl group, a 1-isoquinolyl group , a 2-quinoxalinyl group, a 2-benzofuryl group, a 2-benzothienyl group, an N-indolyl group, an N-carbazolyl group, and an N-acridinyl group; and substituted aromatic heterocyclic groups having 3 to 20 carbon atoms, such as a 2-(5-phenyl)furyl group, a 2-(5-phenyl)thienyl group, and a 2-(3-cyano)pyridyl group.

Examples of the substituted or unsubstituted amino groups include an unsubstituted amino group ($NH_2$); and substituted amino groups having 1 to 30 carbon atoms, such as an N-methylamino group, an N-ethylamino group, an N,N-diethylamino group, an N,N-diisopropylamino group, an N,N-dibutylamino group, an N-benzylamino group, an N,N-dibenzylamino group, an N-phenylamino group, an N-phenyl-N-methylamino group, an N,N-diphenylamino group, an N,N-bis(m-tolyl)amino group, an N,N-bis(p-tolyl)amino group, an N,N-bis(p-biphenylyl)amino group, a bis[4-(4-methyl)biphenyl]amino group, an N-p-biphenylyl-N-phenylamino group, an N-$\alpha$-naphthyl-N-phenylamino group, an N-$\beta$-naphthyl-N-phenylamino group, an N-phenanthryl-N-phenylamino group, an N,N-bis(m-fluorophenyl)amino group, an N,N-bis(3-(9-phenyl)carbazole)amino group, an N,N-bis(p-cyanophenyl)amino group and a bis [4($\alpha$,$\alpha$'-dimethylbenzyl)phenyl] amino group.

Examples of the substituted or unsubstituted acyloxy groups include acyloxy groups having 1 to 30 carbon atoms, such as an acetoxy group and a benzoyloxy group.

From the viewpoint of the performance and practicability of the material, the organic compound (B) preferably has a small band gap as described above and has an excellent affinity to, for example, a solvent as described below. From the above viewpoint, $R_1$ to R $_{12}$ in general formula (1) are preferably a hydrogen atom, a cyano group, a nitro group, a substituted or unsubstituted alkyl group, a substituted or unsubstituted aryl group, a substituted or unsubstituted aromatic heterocyclic group, a substituted or unsubstituted amino group; more preferably a hydrogen atom, a cyano group, a substituted or unsubstituted aryl group, a substituted or unsubstituted aromatic heterocyclic group, or a substituted or unsubstituted amino group; and particularly preferably a hydrogen atom or a substituted amino group.

Among these, at least one of $R_9$ to $R_{12}$ is preferably a substituted or unsubstituted amino group, more preferably an amino group substituted with an aryl group or a heterocyclic group, and particularly preferably an amino group substituted with an aromatic heterocyclic group such as a carbazolyl group.

Next, $X_1$ to $X_4$, $Y_1$ and $Y_2$ in general formula (2) are described.

With respect to the substituted or unsubstituted alkyl groups, the substituted or unsubstituted aryl groups, and the substituted or unsubstituted heterocyclic groups, the above descriptions may be employed.

From the viewpoint of the band gap reduction and the affinity to the electrically conductive material (A), it is preferable that each of substituents $X_1$ and $X_3$ is independently a substituted or unsubstituted aryl group, or a substituted or unsubstituted heterocyclic group, which are expected to undergo $\pi$-conjugated expansion.

From the viewpoint of the affinity to, for example, a solvent, it is preferable that each of substituents $X_2$ and $X_4$ is independently a hydrogen atom or a substituted or unsubstituted alkyl group.

$Y_1$ and $Y_2$ are preferably an oxygen atom or a sulfur atom, and particularly preferably an oxygen atom.

Examples of $X^-$ in general formula (7) include a formate ion, an acetate ion, a propionate ion, a malate ion, a maleate ion, a succinate ion, a glycolate ion, a lactate ion, a tartrate ion, a 1,3,4,5-tetrahydroxycyclohexanecarboxylate (quinate) ion, a benzoate ion, a chlorine ion, a bromine ion, an iodine ion, an alkyl sulfate anion, an alkyl sulfonate anion, a tetracyanob orate anion, dicyanamide, a thiocyanate anion, a hydrogen sulfide anion, a nitrate anion, a cyanide ion, an oxalate borate anion, a phosphate anion, a phosphonate anion, a phosphinate anion, an alkylphosphate anion, an alkylphosphonate anion, an alkylphosphinate anion, a tetraphenylborate, a nonaflate anion, a bis(trifluoromethylsulfonyl)imide anion, a hexafluorophosphate anion, a tetrafluoroborate anion, a trifluoroacetate anion, a fluoroalkyl phosphate anion, a trifluoromethanesulfonate anion, and a tetrafluoroborate ion.

Specific examples of the compounds represented by general formula (1), (2), (3) or (4) are shown in Table 1 to 38 below, and specific examples of the compounds represented by general formula (5), (6), (7) or (8) are shown in Table 39 to 54 below. However, the compounds are not limited by these specific examples. In Tables 1 to 54, Ph represents a phenyl group, Tol represents a p-tolyl group, Me represents a methyl group, Et represents an ethyl group, n-$C_4H_9$ represents a butyl group, tert-Bu and $^t$Bu represent a tert-butyl group, and n-$C_6H_{13}$ represents a hexyl group.

[Table 1]

| Table 1 | | | |
|---|---|---|---|
| Number | Chemical structure | Number | Chemical structure |
| B1 | 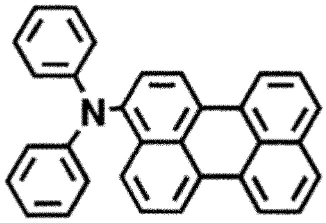 | B2 | 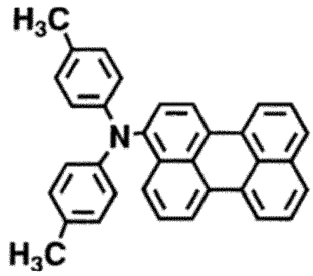 |
| B3 | 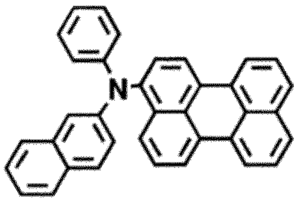 | B4 | 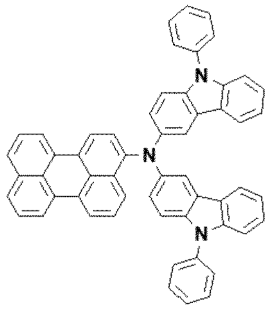 |
| B5 | 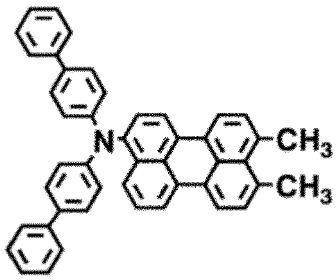 | B6 | 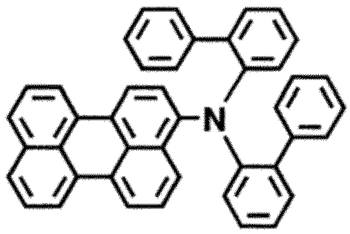 |
| B7 | 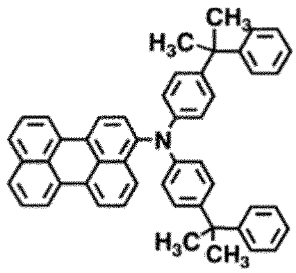 | B8 | 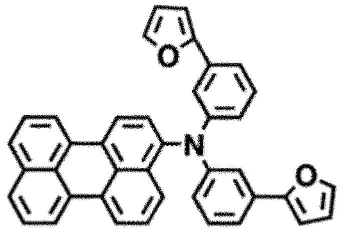 |
| B9 | 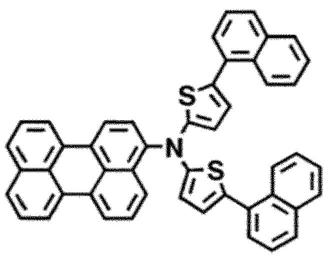 | B10 | 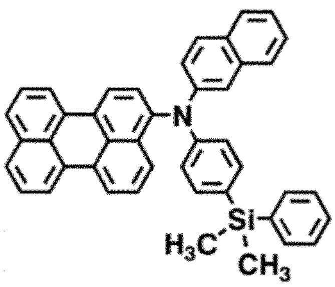 |

[Table 2]

| Table 2 | | | |
|---|---|---|---|
| Number | Chemical structure | Number | Chemical structure |
| B11 | 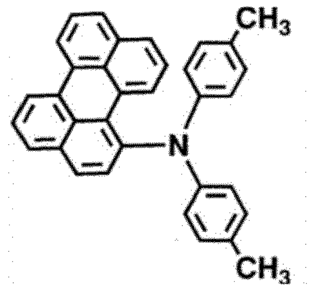 | B12 | 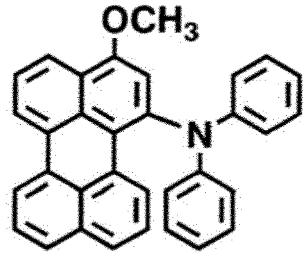 |
| B13 | 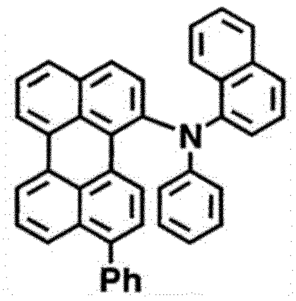 | B14 | 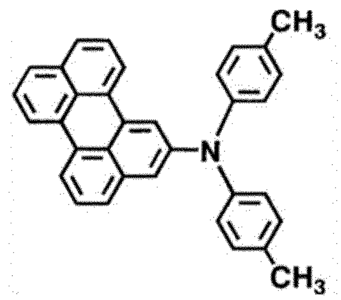 |
| B15 | 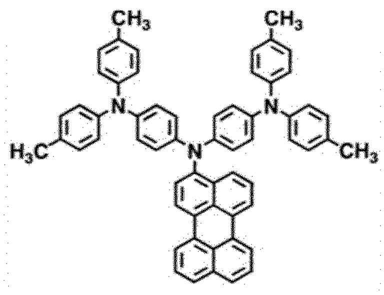 | B16 | 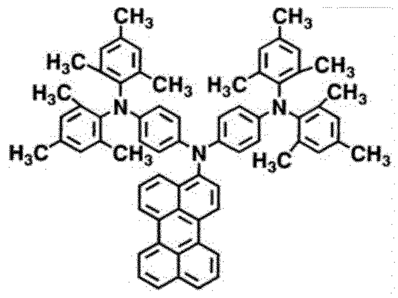 |
| B17 | 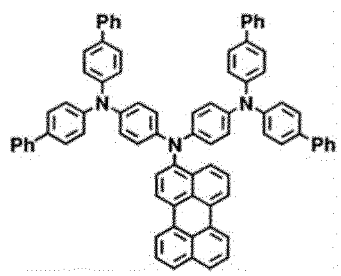 | B18 | 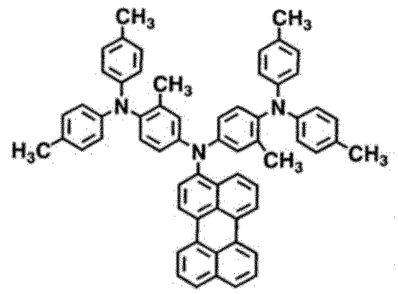 |
| B19 | 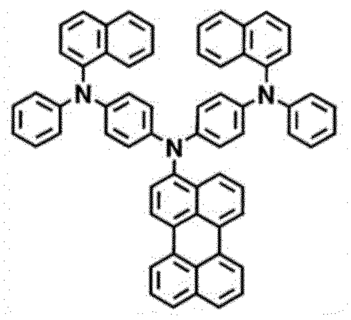 | B20 | 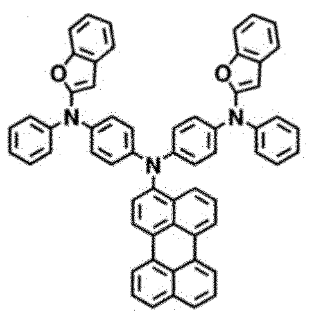 |

[Table 3]

| Table3 | | | |
|---|---|---|---|
| Number | Chemical structure | Number | Chemical structure |
| B21 | 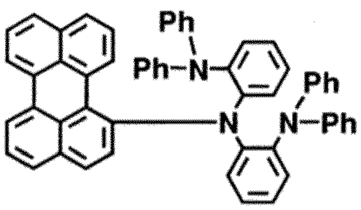 | B22 | 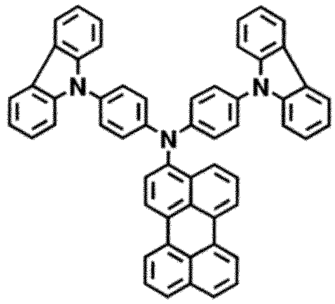 |
| B23 | 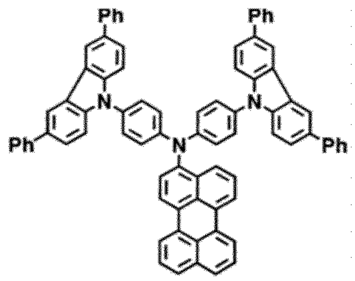 | B24 | 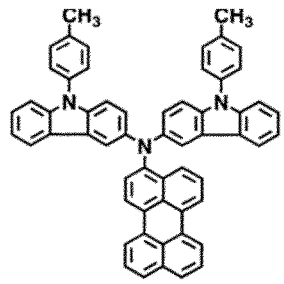 |
| B25 | 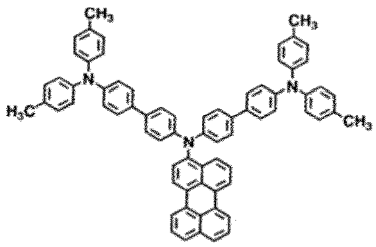 | B26 | 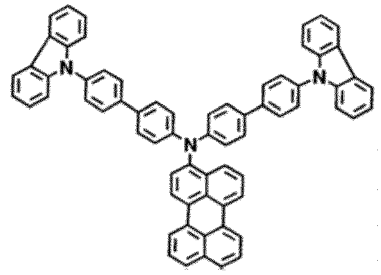 |
| B27 | 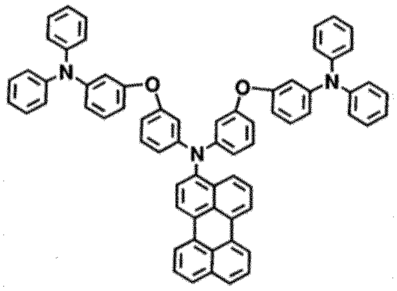 | B28 | 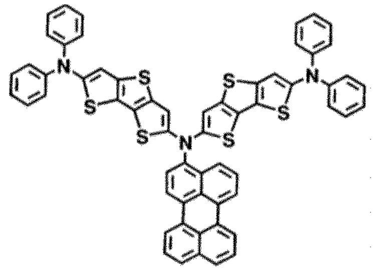 |
| B29 | 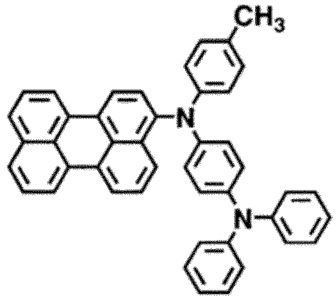 | B30 | 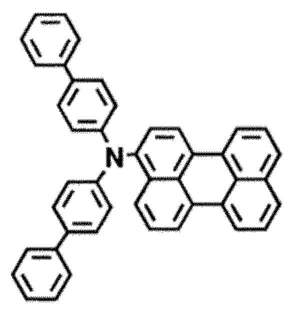 |

[Table 4]

| Table 4 | | | |
|---|---|---|---|
| Number | Chemical structure | Number | Chemical structure |
| B31 | 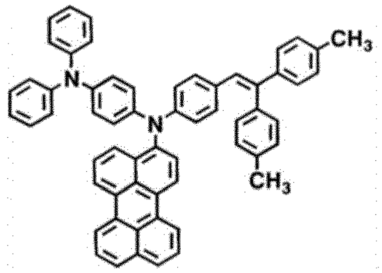 | B32 | 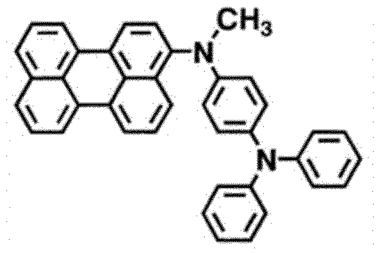 |
| B33 | 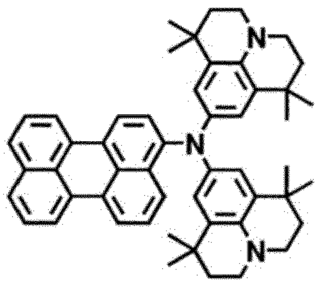 | B34 | 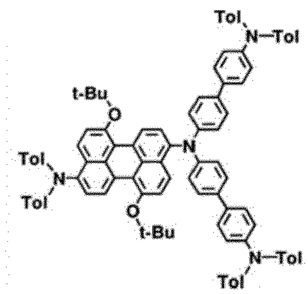 |
| B35 | 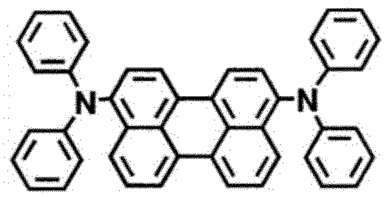 | B36 | 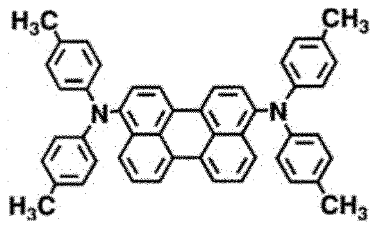 |
| B37 | 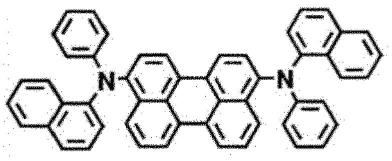 | B38 | 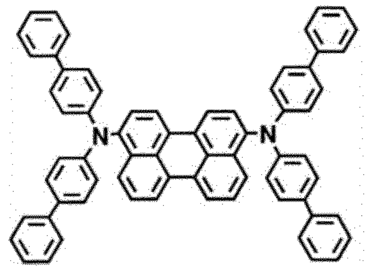 |
| B39 | 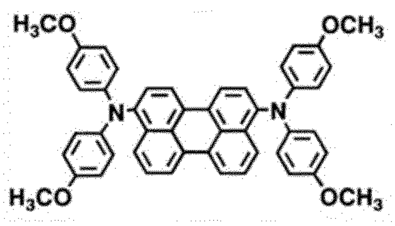 | B40 | 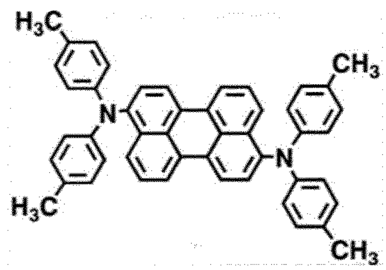 |

[Table 5]

| Table5 | | | |
|---|---|---|---|
| Number | Chemical structure | Number | Chemical structure |
| B41 | 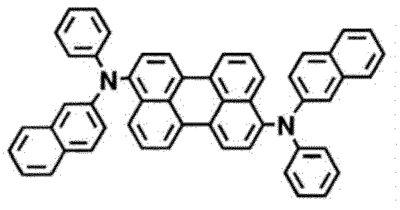 | B42 | 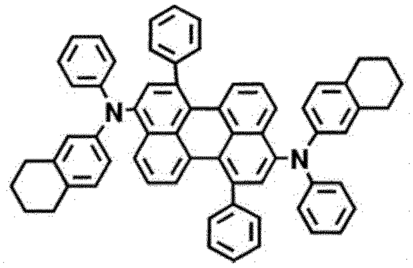 |
| B43 | 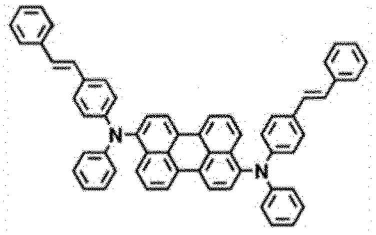 | B44 | 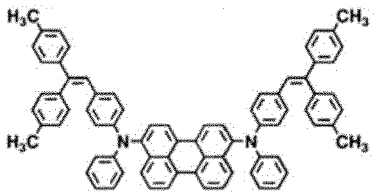 |
| B45 | 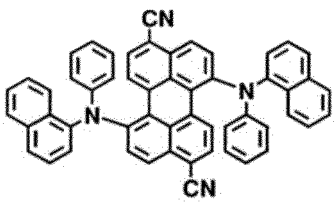 | B46 | 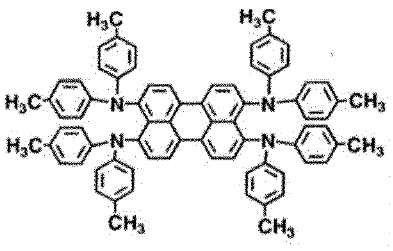 |
| B47 | 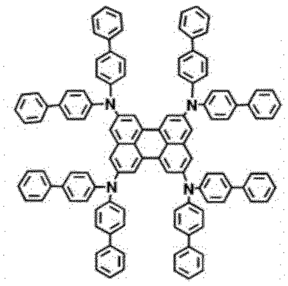 | B48 | 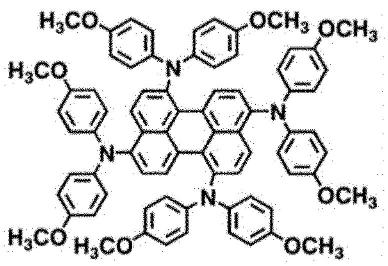 |
| B49 | 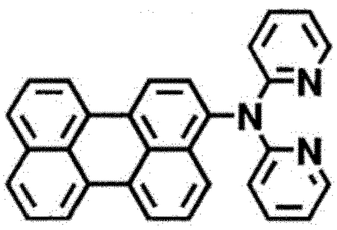 | B50 | 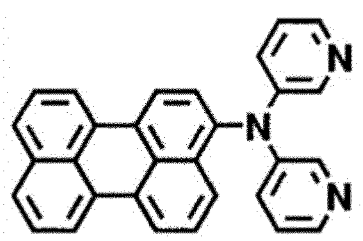 |

[Table 6]

| Table6 | | | |
|---|---|---|---|
| Number | Chemical structure | Number | Chemical structure |
| B51 | 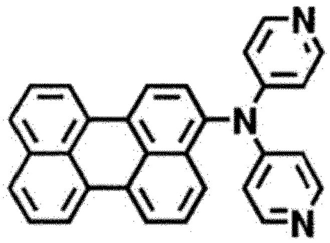 | B52 | 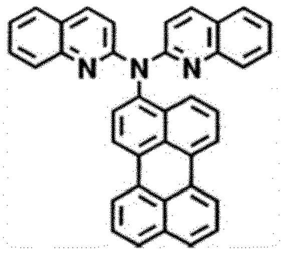 |
| B53 | 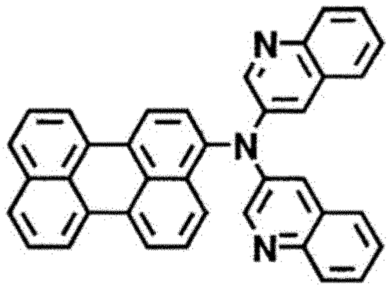 | B54 | 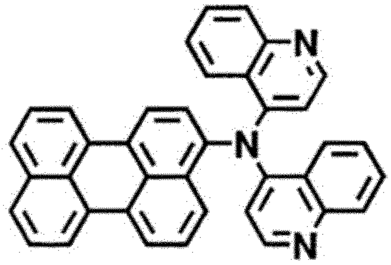 |
| B55 | 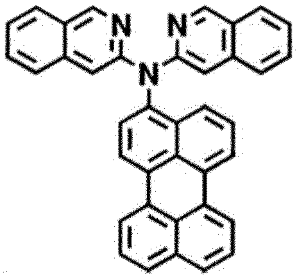 | B56 | 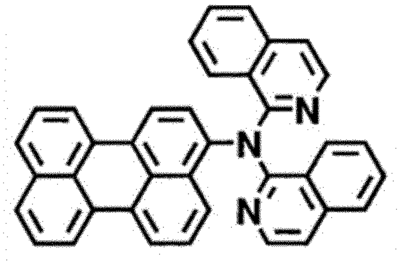 |
| B57 | 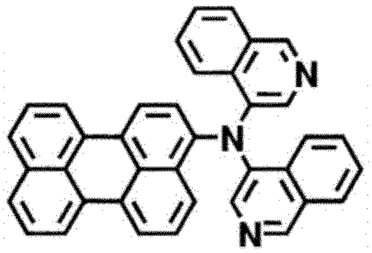 | B58 | 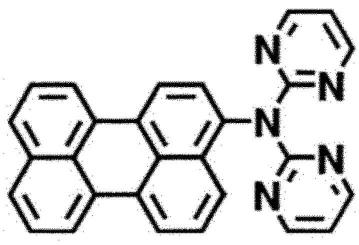 |
| B59 | 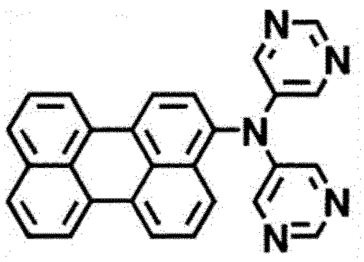 | B60 | 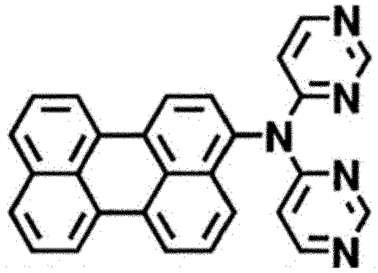 |

[Table 7]

| Table 7 | | | |
|---|---|---|---|
| Number | Chemical structure | Number | Chemical structure |
| B61 | 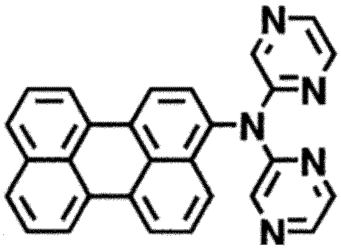 | B62 | 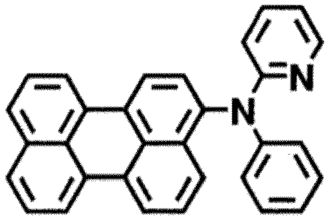 |
| B63 | 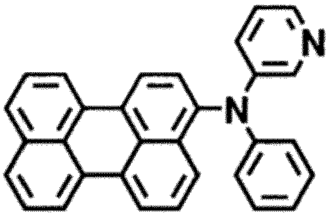 | B64 | 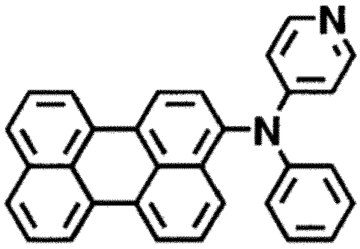 |
| B65 | 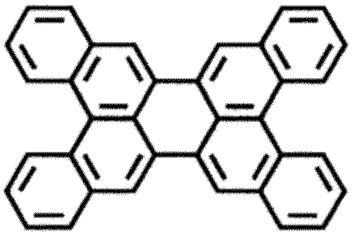 | B66 | 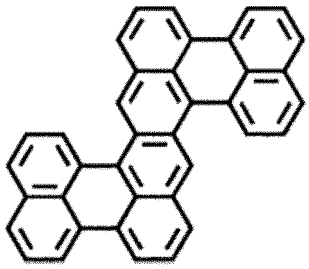 |
| B67 | 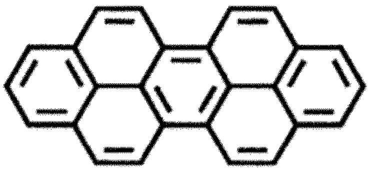 | B68 | 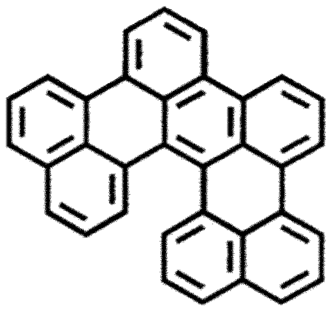 |
| B69 | 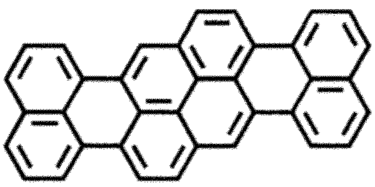 | B70 | 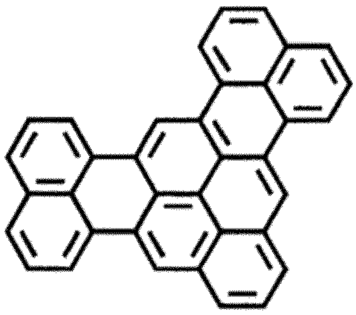 |

[Table 8]

| Table 8 | | | |
|---|---|---|---|
| Number | Chemical structure | Number | Chemical structure |
| B71 | 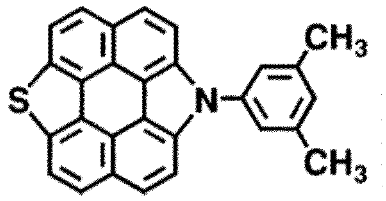 | B72 | 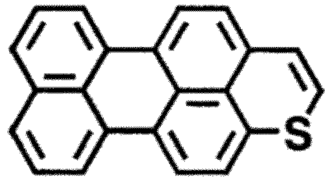 |
| B73 | 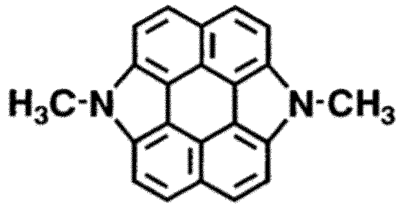 | B74 | 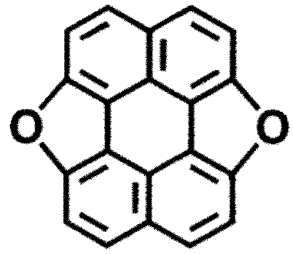 |
| B75 | 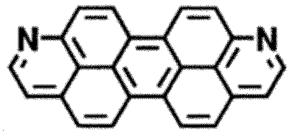 | B76 | 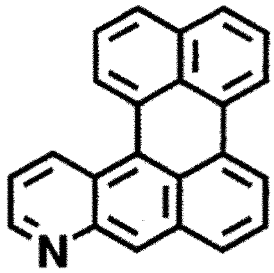 |
| B77 | 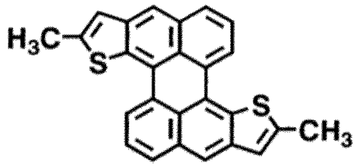 | B78 | 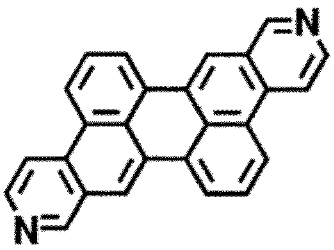 |
| B79 | 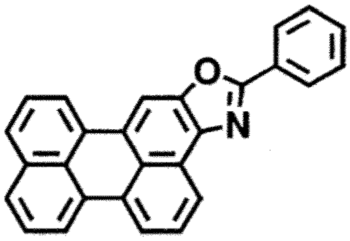 | B80 | 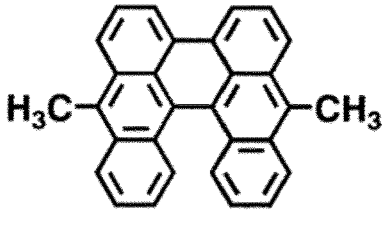 |

[Table 9]

| Table 9 | | | |
|---|---|---|---|
| Number | Chemical structure | Number | Chemical structure |
| B81 | 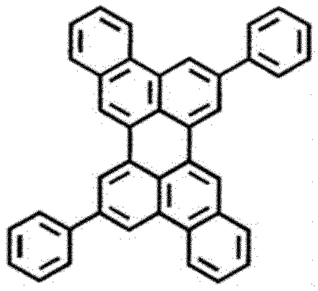 | B82 | 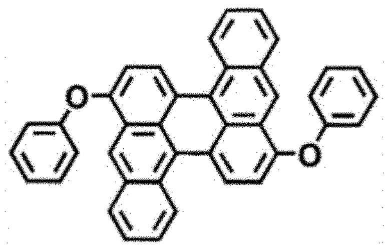 |
| B83 | 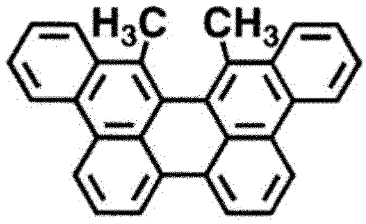 | B84 | 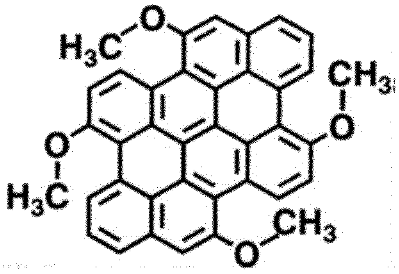 |
| B85 | 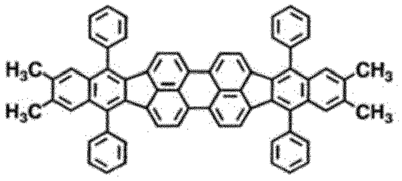 | B86 | 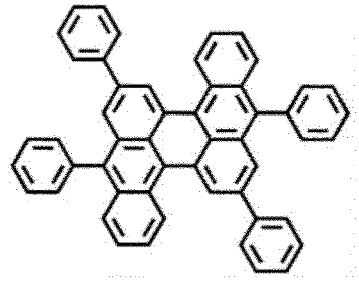 |
| B87 | 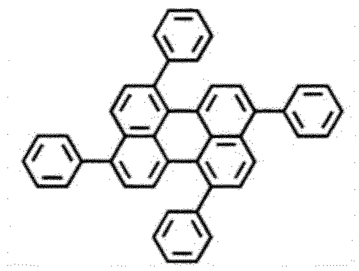 | B88 | 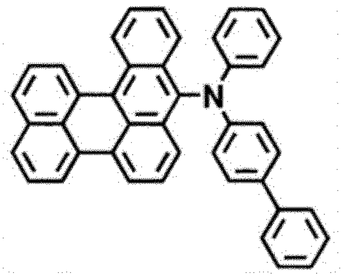 |
| B89 | 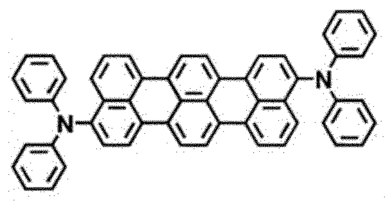 | B90 | 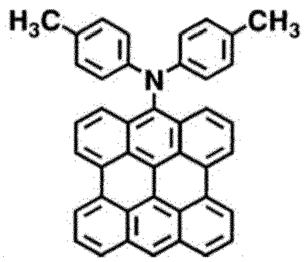 |

[Table 10]

| Table 10 | | | |
|---|---|---|---|
| Number | Chemical structure | Number | Chemical structure |
| B91 | 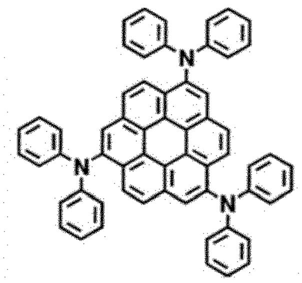 | B92 | 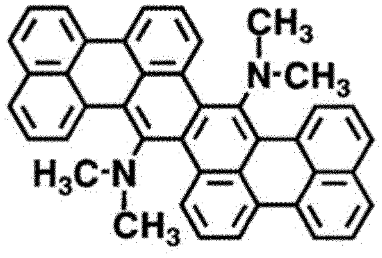 |
| B93 | 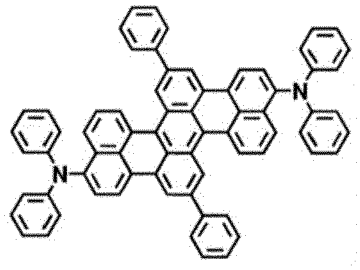 | B94 | 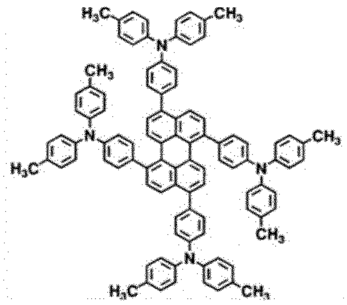 |
| B95 | 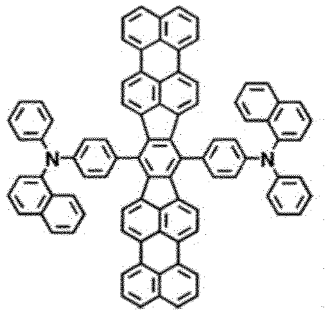 | B96 | 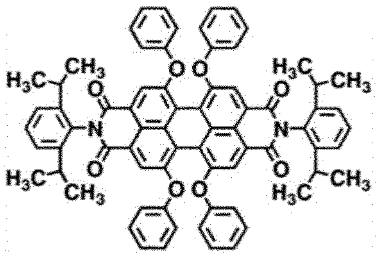 |
| B97 | 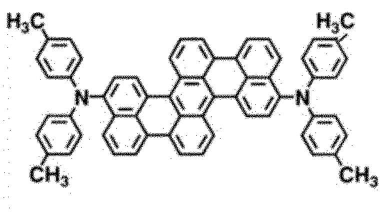 | B98 | 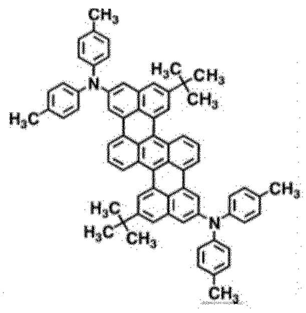 |

(continued)

| Table 10 | | | |
|---|---|---|---|
| Number | Chemical structure | Number | Chemical structure |
| B99 | 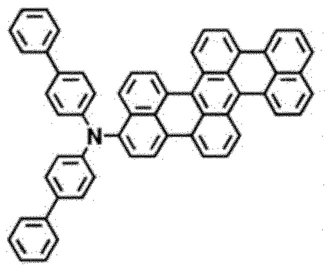 | 8100 | 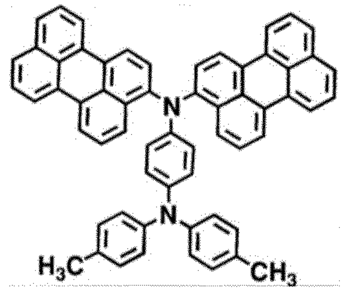 |

[Table 11]

| Table 11 | | | |
|---|---|---|---|
| Number | Chemical structure | Number | Chemical structure |
| B101 | 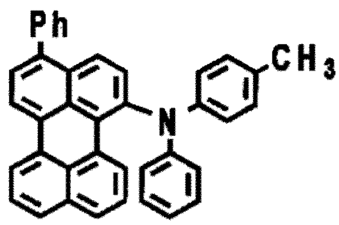 | B102 | 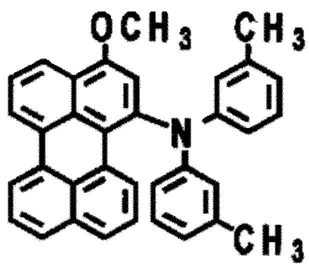 |
| B103 | 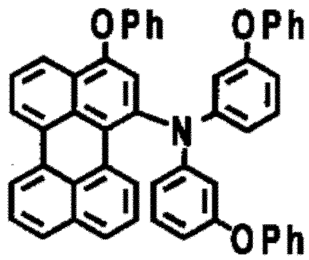 | B104 | 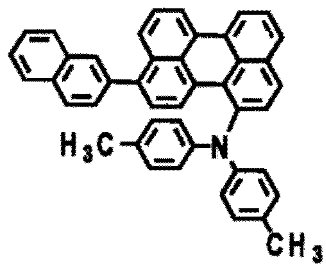 |
| B105 | 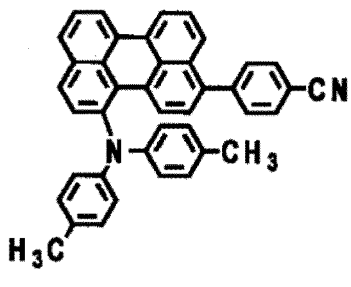 | B106 | 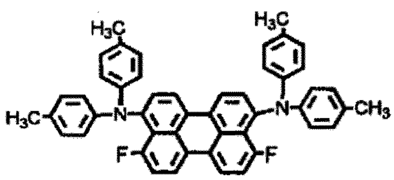 |

(continued)

| Table 11 | | | |
|---|---|---|---|
| Number | Chemical structure | Number | Chemical structure |
| B107 | 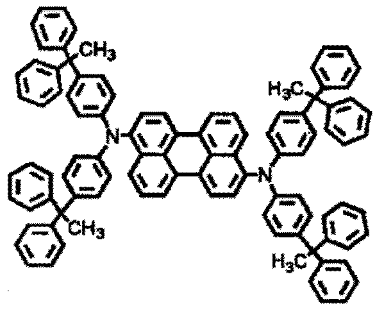 | B108 | 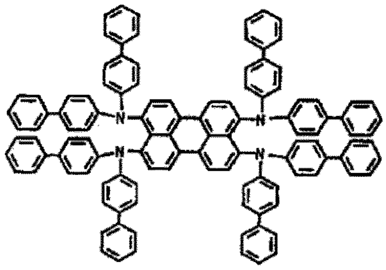 |

[Table 12]

| Table 12 | | | |
|---|---|---|---|
| Number | Chemical structure | Number | Chemical structure |
| B109 | 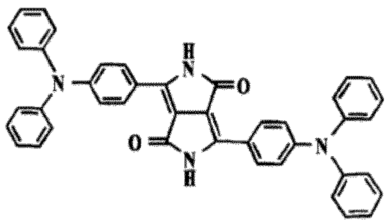 | B110 | 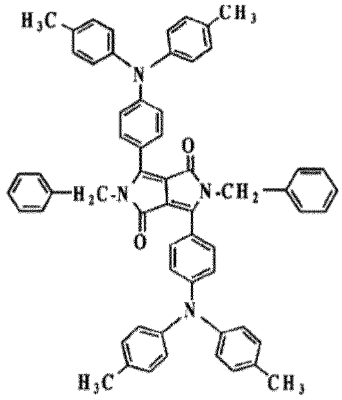 |
| 8111 | 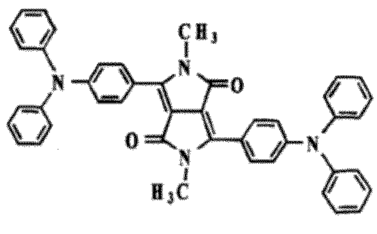 | B112 | 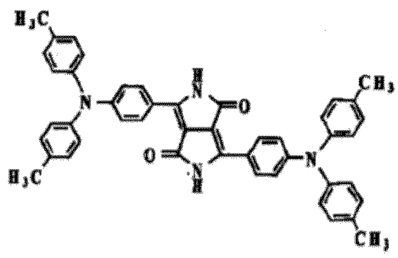 |
| B113 | 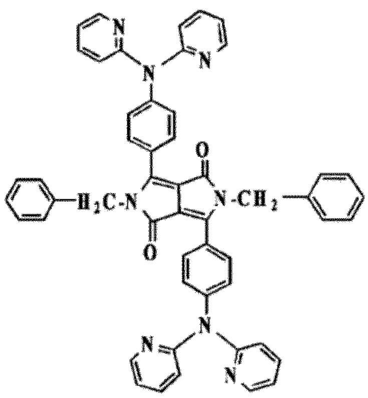 | 8114 | 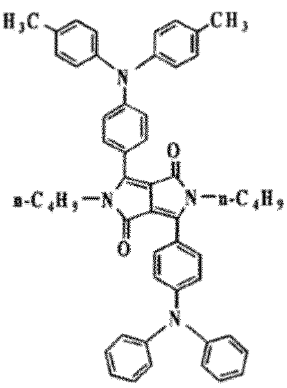 |

(continued)

| Table 12 | | | |
|---|---|---|---|
| Number | Chemical structure | Number | Chemical structure |
| B115 | 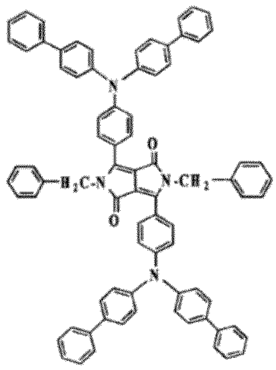 | B116 | 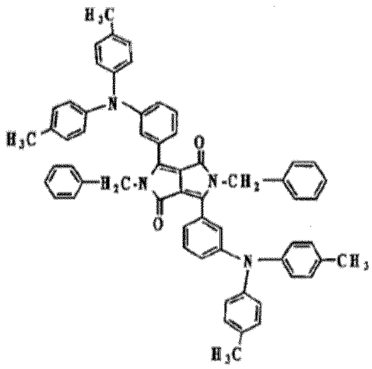 |

[Table 13]

| Table 13 | | | |
|---|---|---|---|
| Number | Chemical structure | Number | Chemical structure |
| B117 | 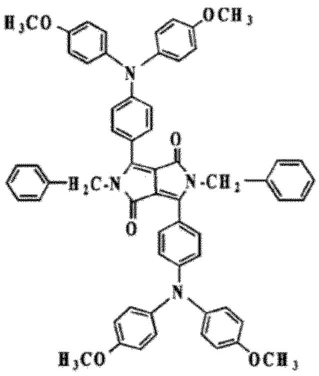 | B118 | 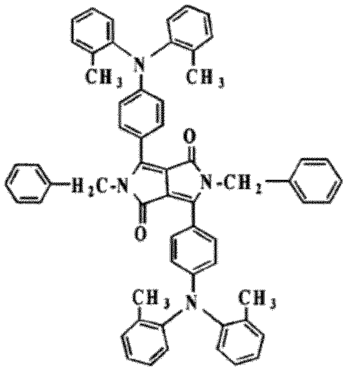 |
| B119 | 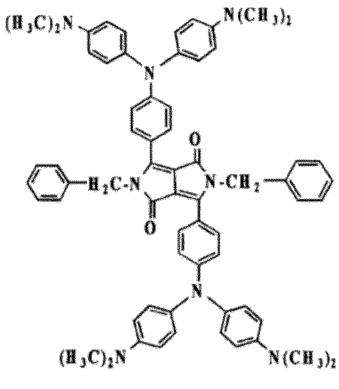 | B120 | 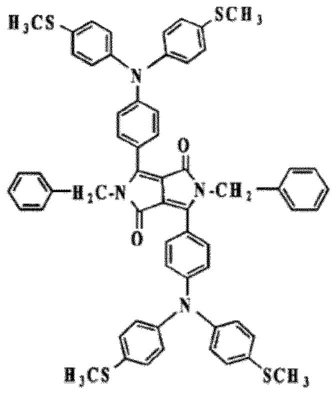 |

(continued)

| Table 13 | | | |
|---|---|---|---|
| Number | Chemical structure | Number | Chemical structure |
| B121 | 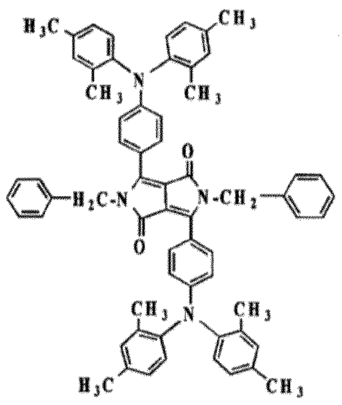 | B122 | 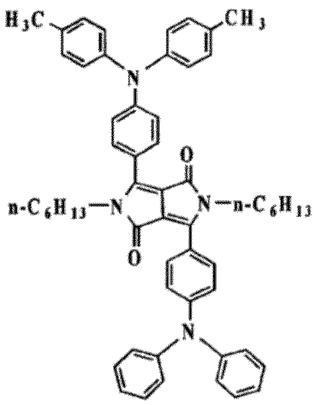 |
| B123 | 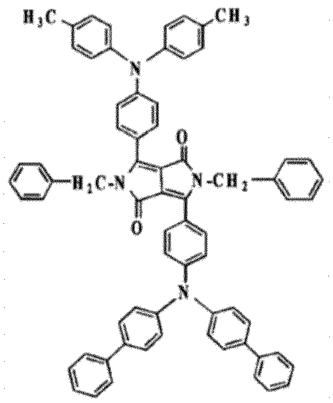 | B124 | 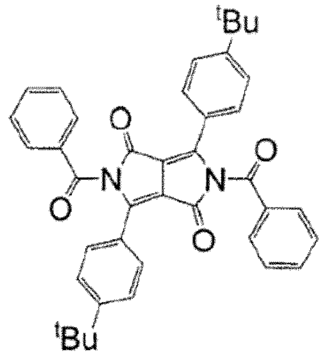 |

[Table 14]

| Table 14 | | | |
|---|---|---|---|
| Number | Chemical structure | Number | Chemical structure |
| B125 | 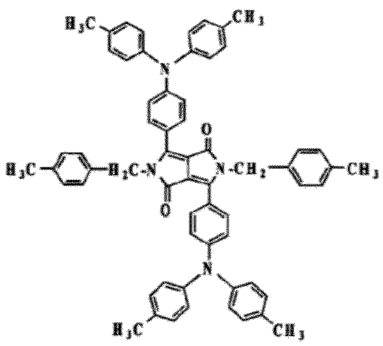 | B126 | 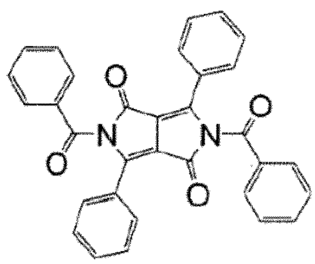 |

(continued)

| Table 14 | | | |
|---|---|---|---|
| Number | Chemical structure | Number | Chemical structure |
| B127 | 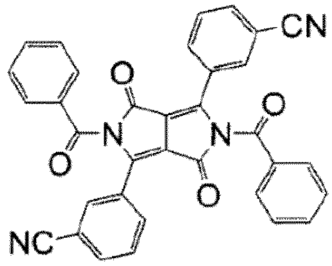 | B128 | 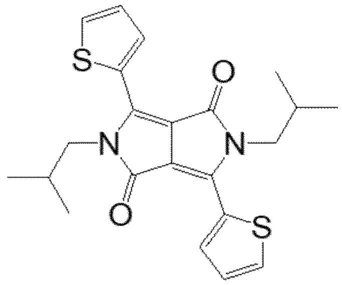 |
| B129 | 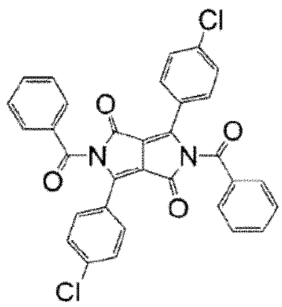 | B130 | 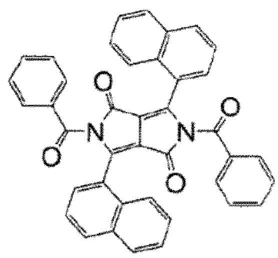 |
| B131 | 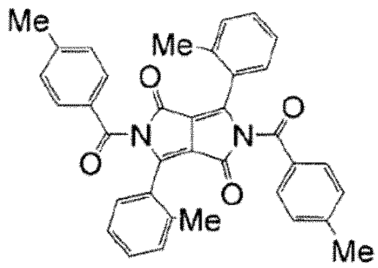 | B132 | 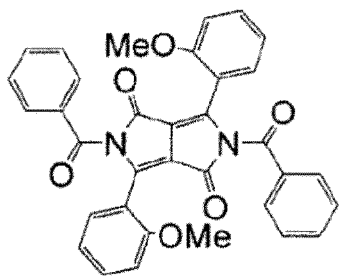 |

[Table 15]

| Table 15 | | | |
|---|---|---|---|
| Number | Chemical structure | Number | Chemical structure |
| B133 | 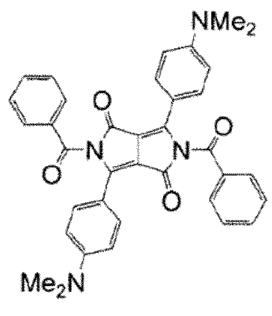 | B134 | 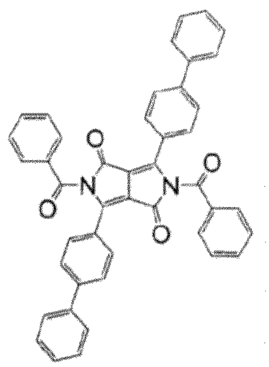 |

(continued)

| Table 15 | | | |
|---|---|---|---|
| Number | Chemical structure | Number | Chemical structure |
| B135 | 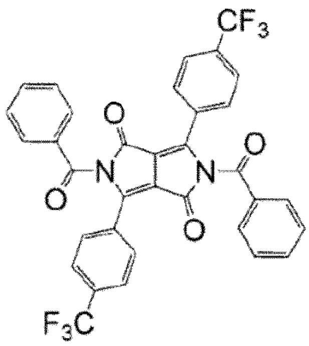 | B136 | 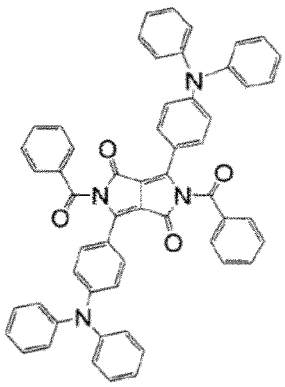 |
| B137 | 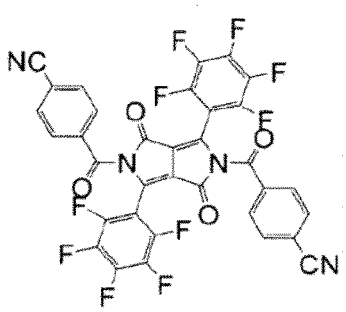 | B138 | 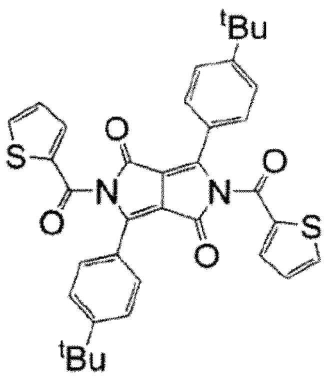 |
| B139 | 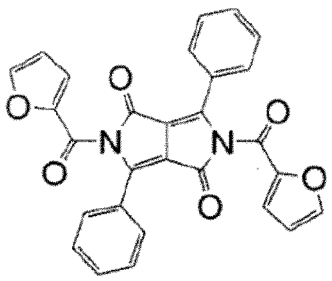 | B140 | 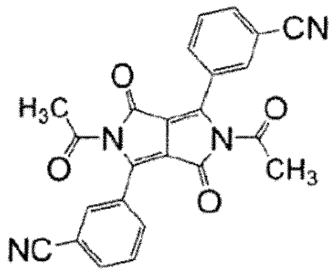 |

[Table 16]

| Table 16 | | | |
|---|---|---|---|
| Number | Chemical structure | Number | Chemical structure |
| B141 | 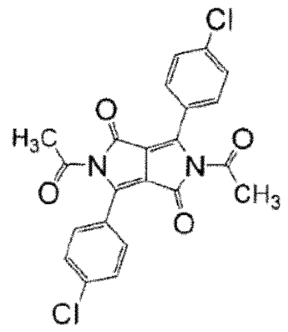 | B142 | 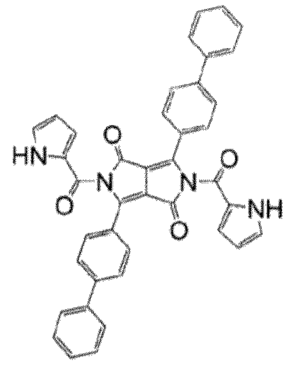 |
| B143 | 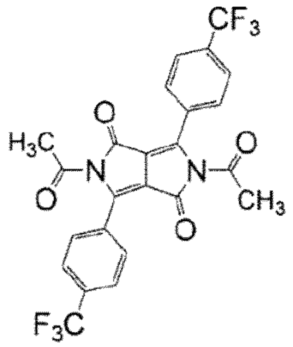 | B144 | 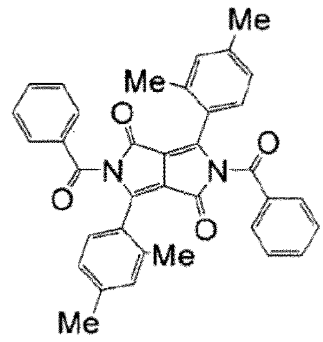 |
| B145 | 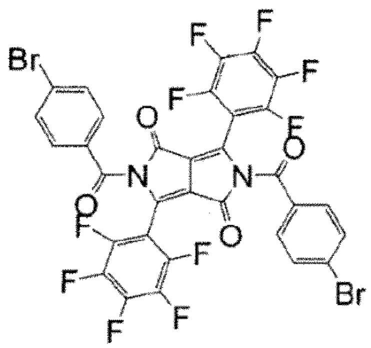 | B146 | 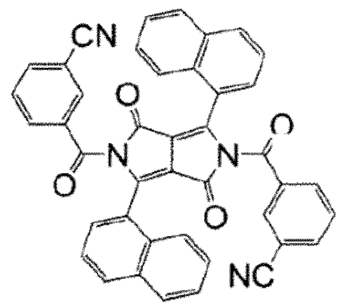 |
| B147 | 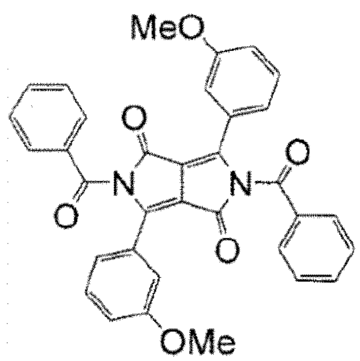 | B148 | 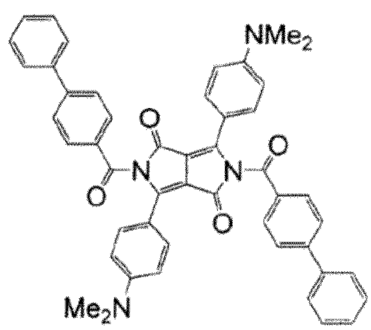 |

[Table 17]

| Table 17 | | | |
|---|---|---|---|
| Number | Chemical structure | Number | Chemical structure |
| B149 | 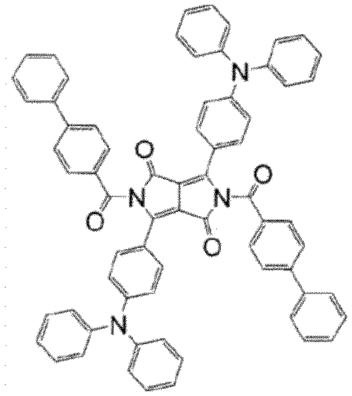 | B150 | 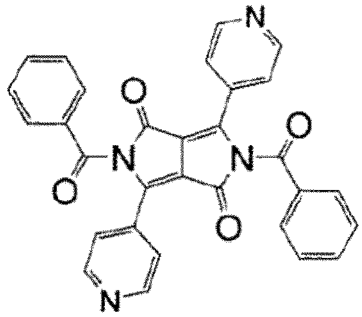 |
| B151 | 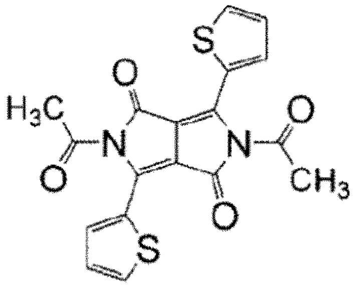 | B152 | 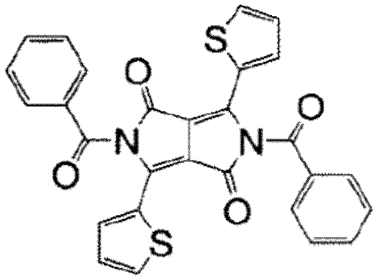 |
| B153 | 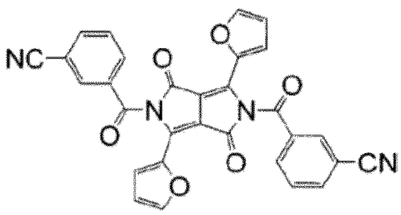 | B154 | 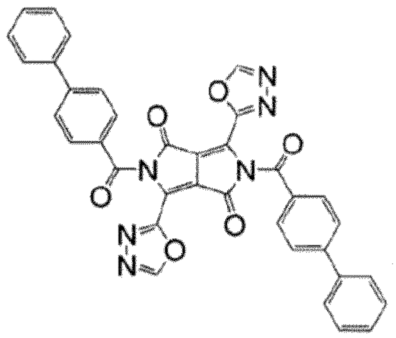 |
| B155 | 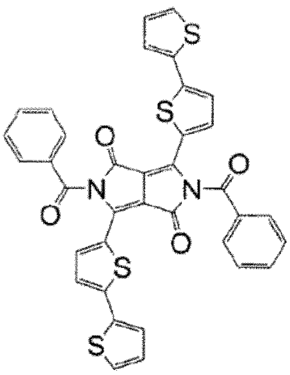 | B156 | 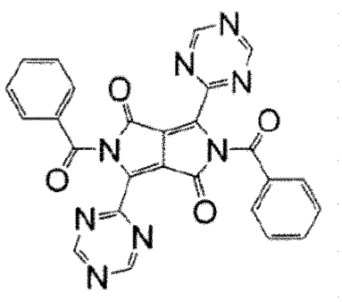 |

[Table 18]

| Table 18 | | | |
|---|---|---|---|
| Number | Chemical structure | Number | Chemical structure |
| B157 | 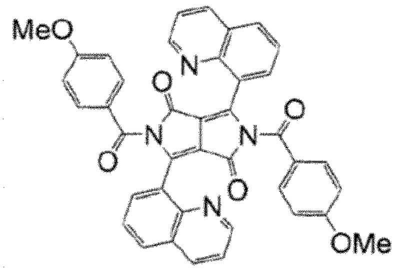 | B158 | 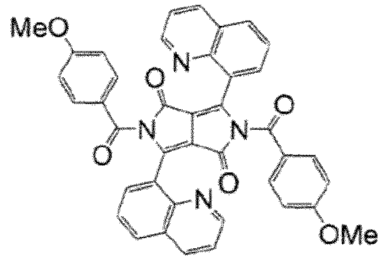 |
| B159 | 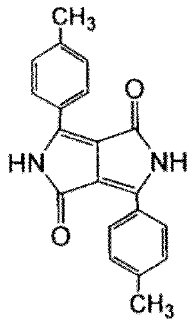 | B160 | 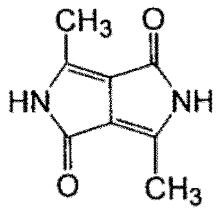 |
| B161 | 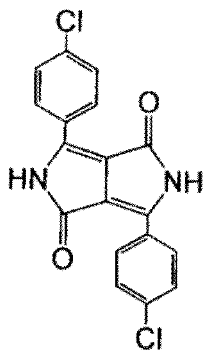 | B162 | 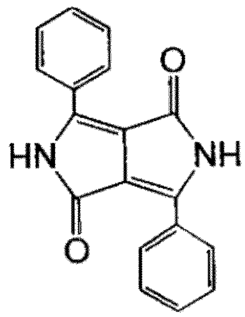 |
| B163 | 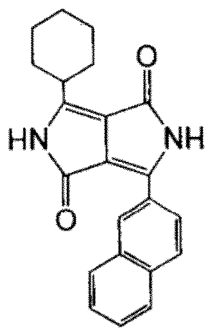 | B164 | 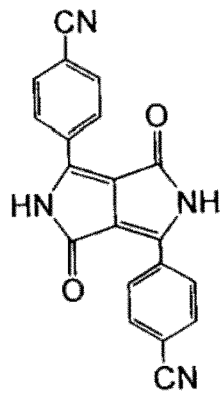 |

[Table 19]

| Table 19 | | | |
|---|---|---|---|
| Number | Chemical structure | Number | Chemical structure |
| B165 | 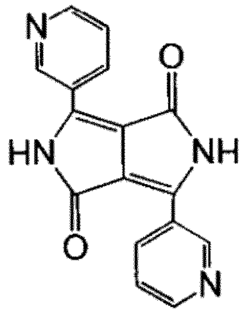 | B166 | 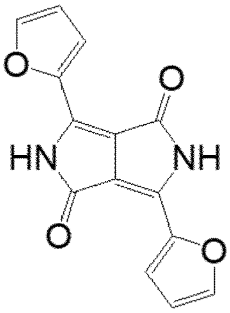 |
| B167 | 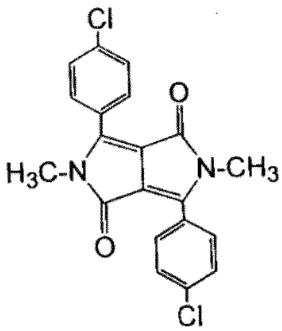 | B168 | 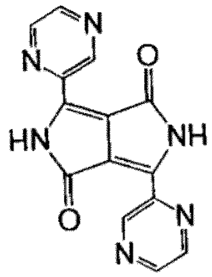 |
| B169 | 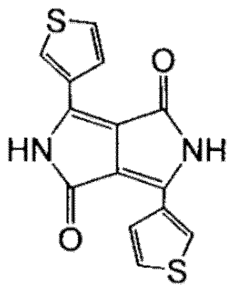 | B170 | 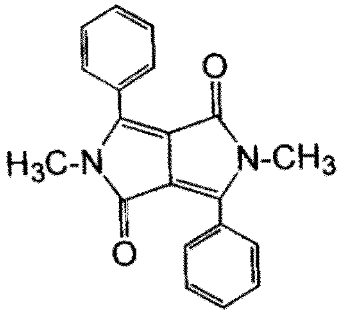 |
| B171 | 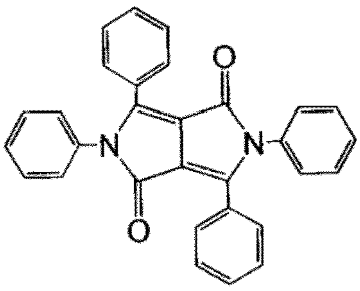 | B172 | 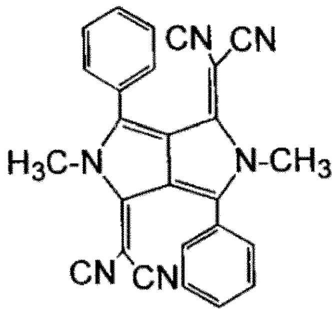 |

[Table 20]

| Table 20 | | | |
|---|---|---|---|
| Number | Chemical structure | Number | Chemical structure |
| B173 | 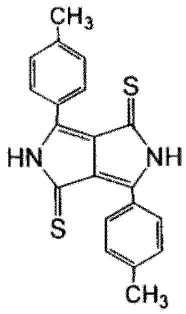 | B174 | 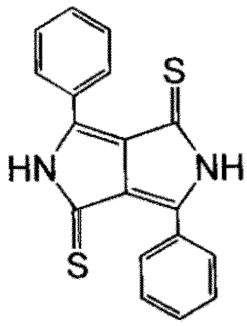 |
| B175 | 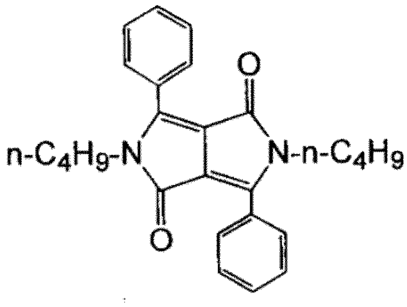 | B176 | 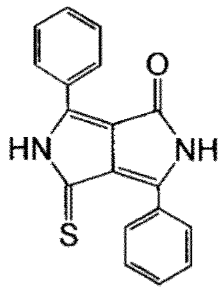 |
| B177 | 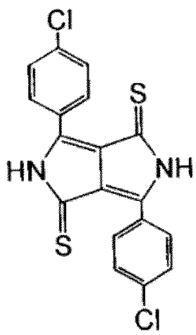 | B178 | 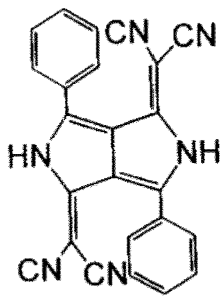 |
| B179 | 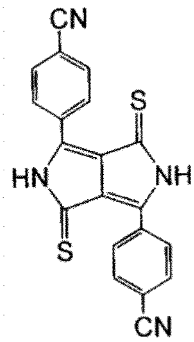 | B180 | 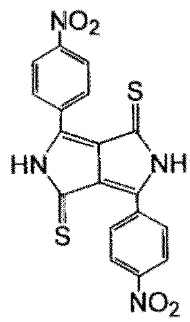 |

[Table 21]

| Table 21 | | | |
|---|---|---|---|
| Number | Chemical structure | Number | Chemical structure |
| B181 | 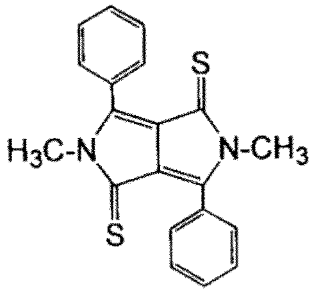 | B182 | 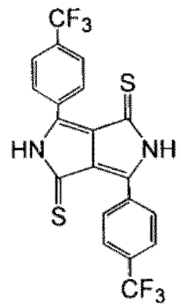 |
| B183 | 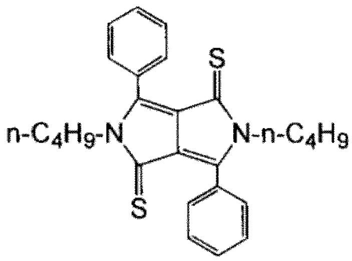 | B184 | 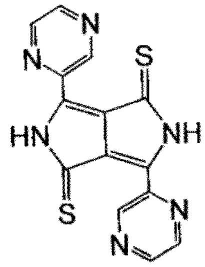 |
| B185 | 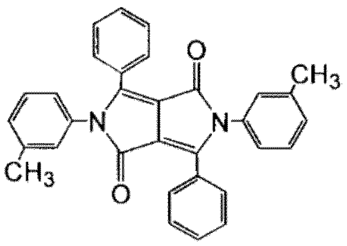 | B186 | 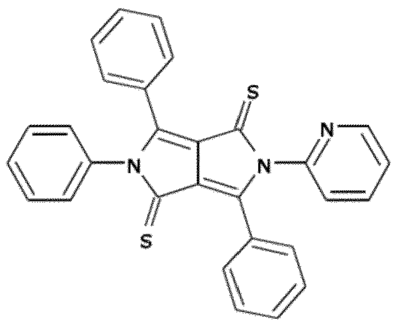 |
| B187 | 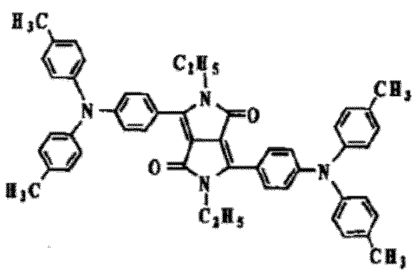 | B188 | 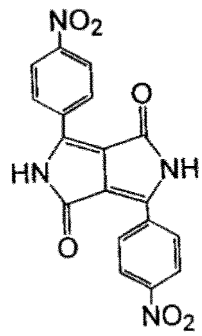 |

[Table 22]

| Table 22 | | | |
|---|---|---|---|
| Number | Chemical structure | Number | Chemical structure |
| B189 | 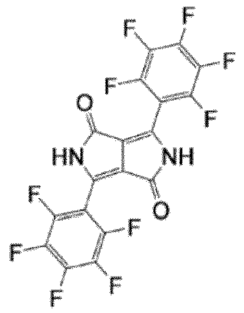 | B190 | 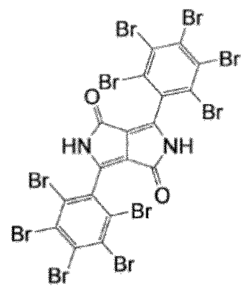 |
| B191 | 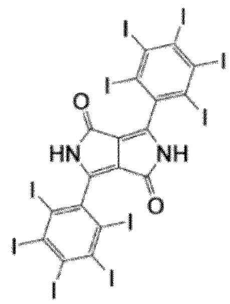 | B192 | 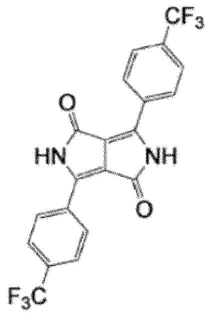 |
| B193 | 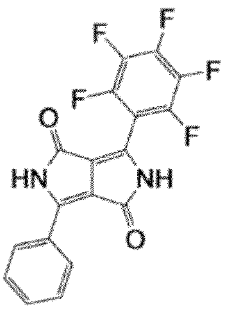 | B194 | 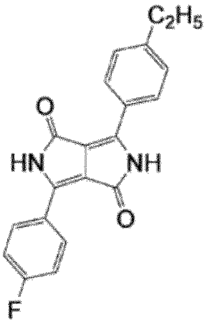 |
| B195 | 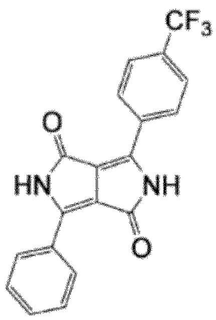 | B196 | 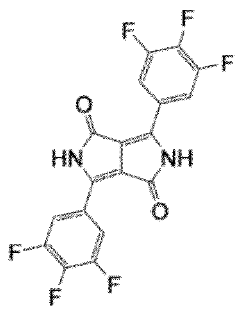 |

[Table 23]

| Table 23 | | | |
|---|---|---|---|
| Number | Chemical structure | Number | Chemical structure |
| B197 | 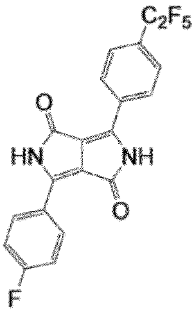 | B198 | 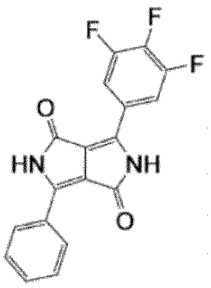 |
| B199 | 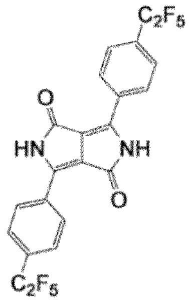 | B200 | 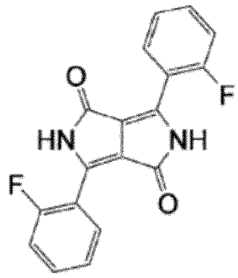 |
| B201 | 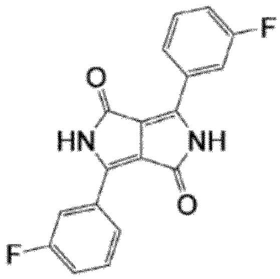 | B202 | 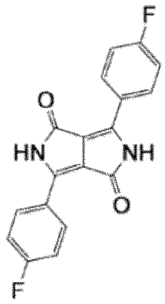 |
| B203 | 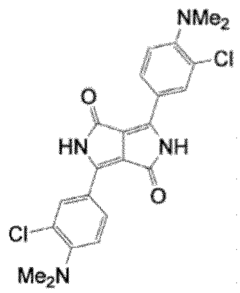 | B204 | 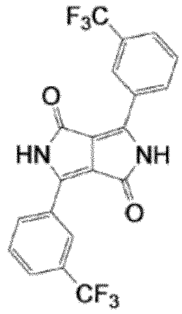 |

[Table 24]

| Table 24 | | | |
|---|---|---|---|
| Number | Chemical structure | Number | Chemical structure |
| B205 | 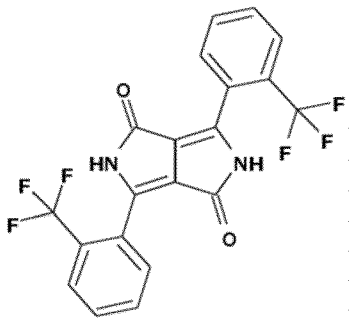 | B206 | 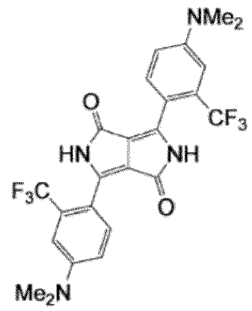 |
| B207 | 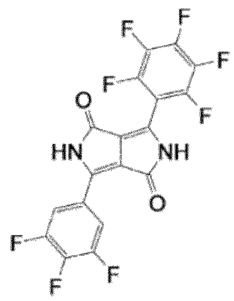 | B208 | 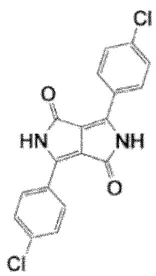 |
| B209 | 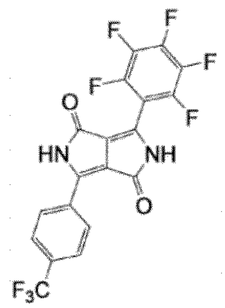 | B210 | 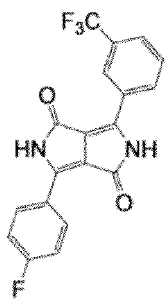 |
| B211 | 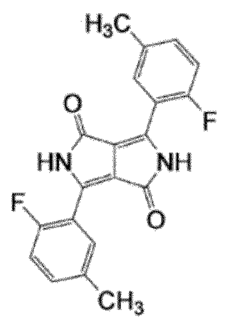 | B212 | 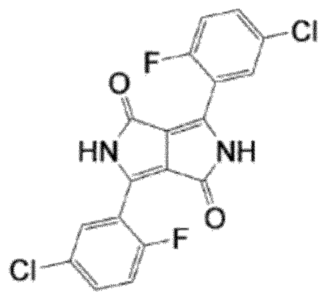 |

[Table 25]

| Table 25 | | | |
|---|---|---|---|
| Number | Chemical structure | Number | Chemical structure |
| B213 | 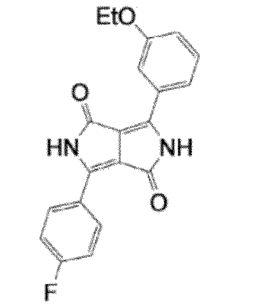 | B214 | 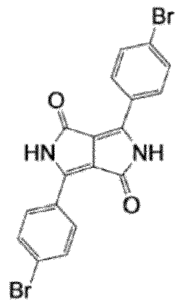 |
| B215 | 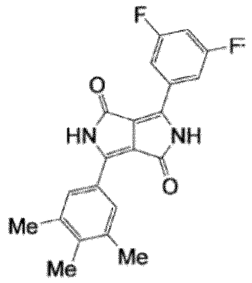 | B216 | 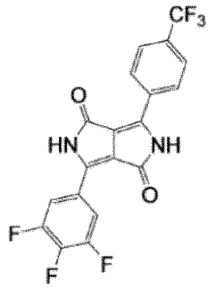 |
| B217 | 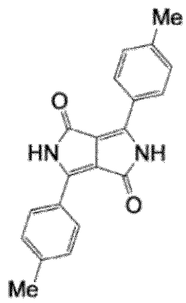 | B218 | 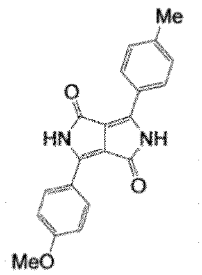 |
| B219 | 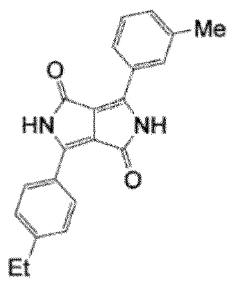 | B220 | 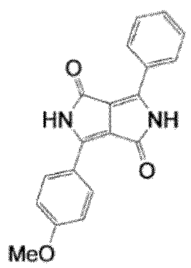 |

[Table 26]

| Table 26 | | | |
|---|---|---|---|
| Number | Chemical structure | Number | Chemical structure |
| B221 | 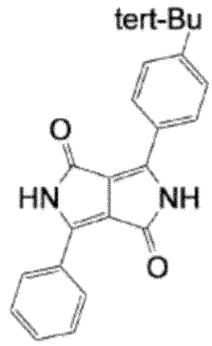 | B222 | 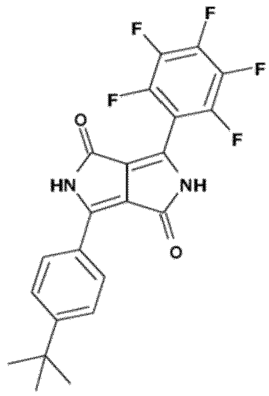 |
| B223 | 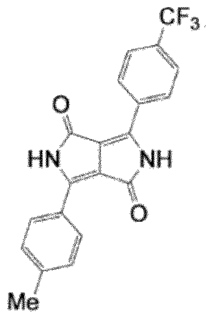 | B224 | 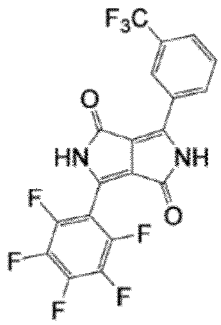 |
| B225 | 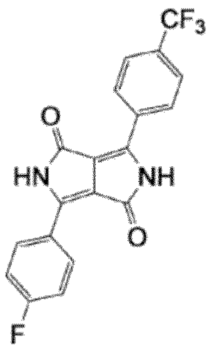 | B226 | 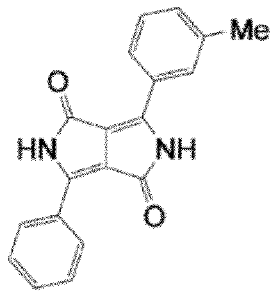 |
| B227 | 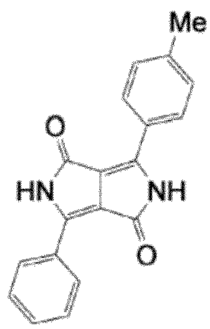 | B228 | 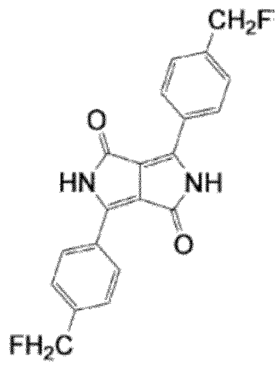 |

[Table 27]

| Table 27 | | | |
|---|---|---|---|
| Number | Chemical structure | Number | Chemical structure |
| B229 | 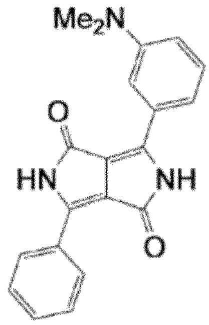 | B230 | 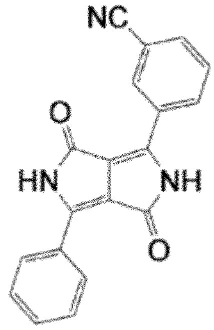 |
| B231 | 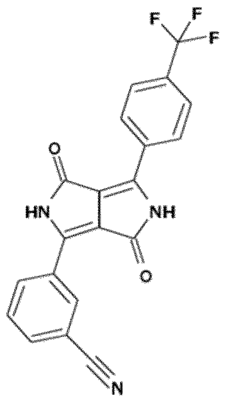 | B232 | 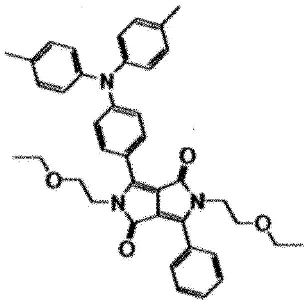 |
| B233 | 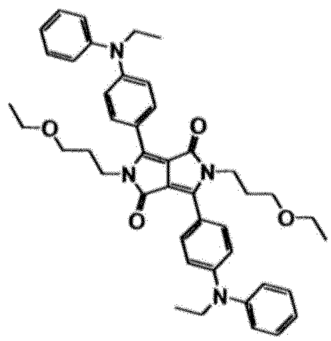 | B234 | 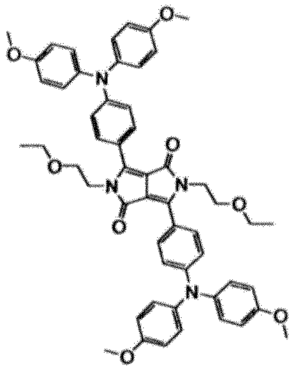 |
| B235 | 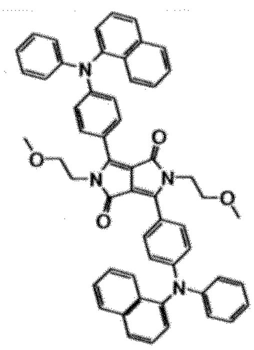 | B236 | 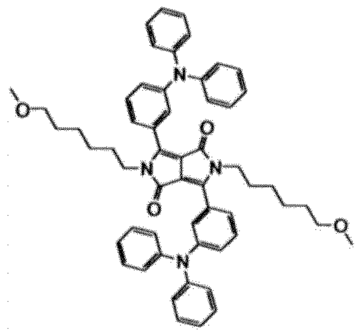 |

[Table 28]

| Table 28 | | | |
|---|---|---|---|
| Number | Chemical structure | Number | Chemical structure |
| B237 | 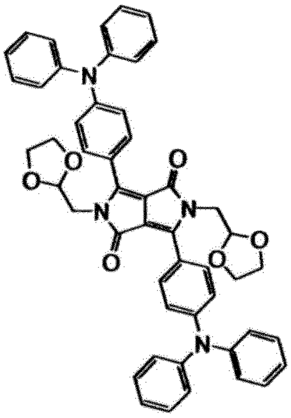 | B238 | 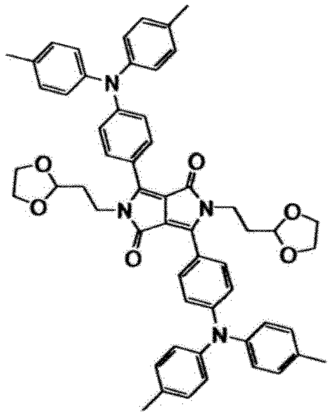 |
| B239 | 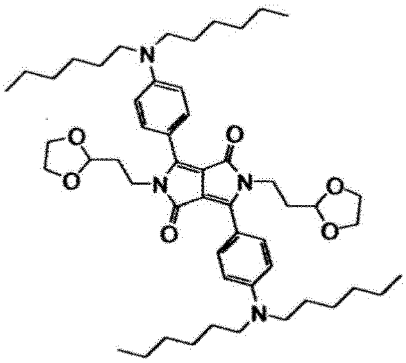 | B240 | 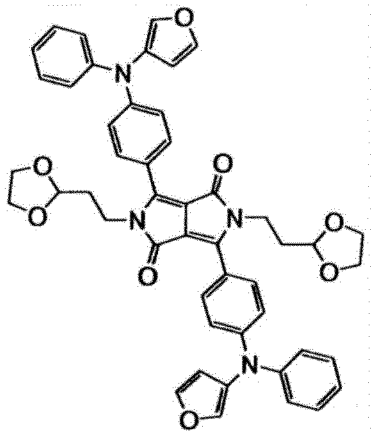 |

[Table 29]

| **Table 29** | | | |
|---|---|---|---|
| **Number** | **Chemical structure** | **Number** | **Chemical structure** |
| **B241** | 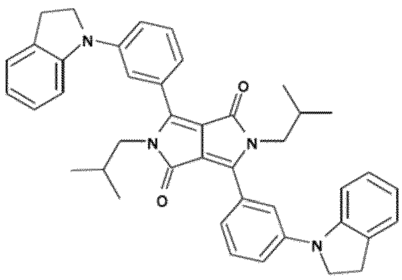 | **B242** | 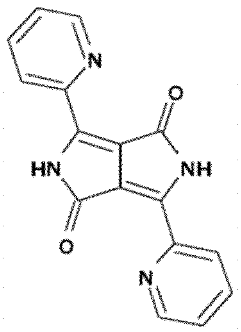 |

(continued)

| Table 29 | | | |
|---|---|---|---|
| Number | Chemical structure | Number | Chemical structure |
| B243 | 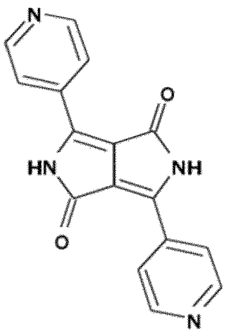 | B244 | 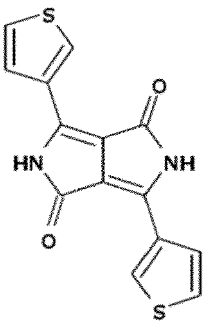 |
| B245 | 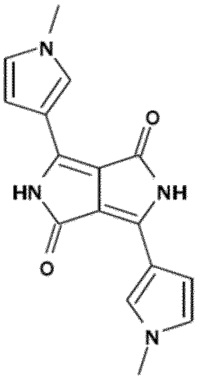 | B246 | 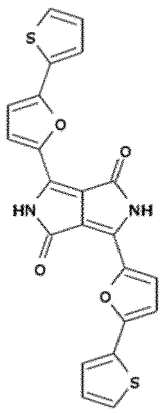 |
| B247 | 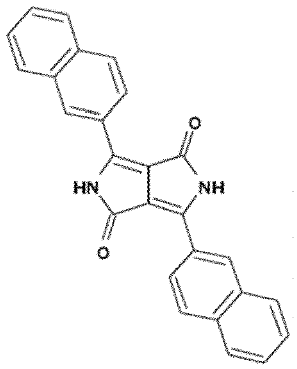 | B248 | 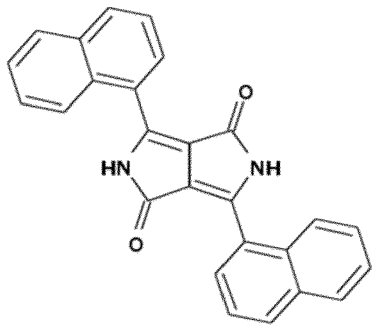 |

[Table 30]

| Table 30 | | | |
|---|---|---|---|
| **Number** | **Chemical structure** | **Number** | **Chemical structure** |
| **B249** | 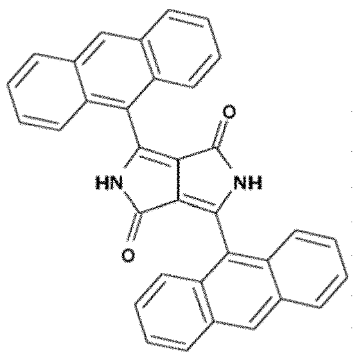 | **B250** | 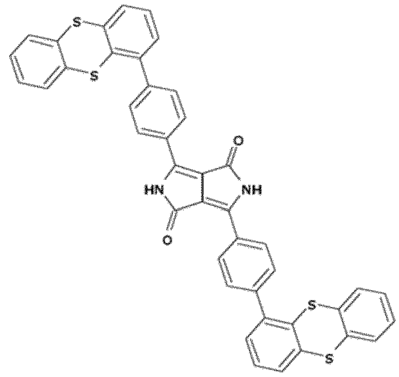 |
| **B251** | 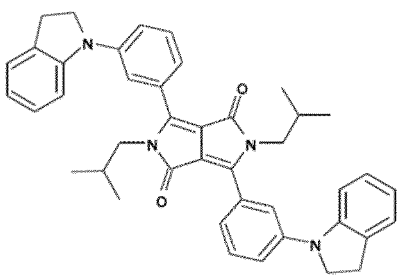 | **B252** | 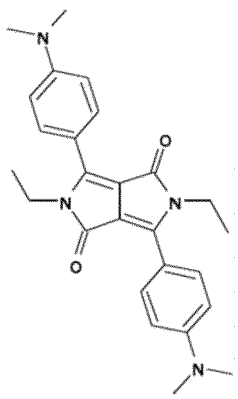 |
| **B253** | 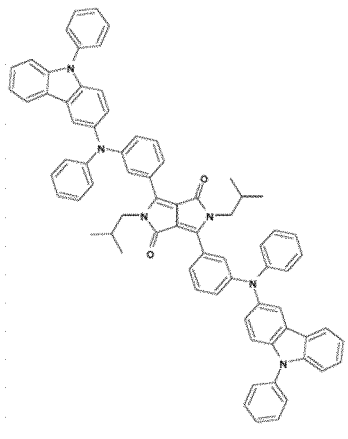 | **B254** | 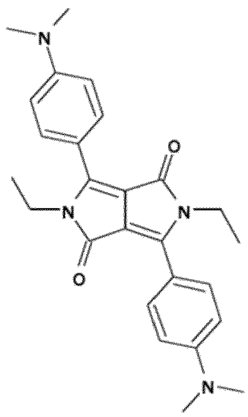 |

(continued)

| Table 30 | | | |
|---|---|---|---|
| Number | Chemical structure | Number | Chemical structure |
| B255 | 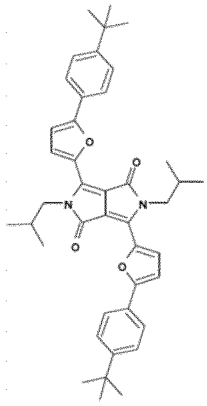 | B256 | 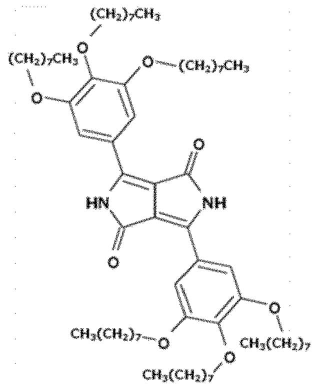 |

[Table 31]

| Table 31 | | | |
|---|---|---|---|
| Number | Chemical structure | Number | Chemical structure |
| B257 | 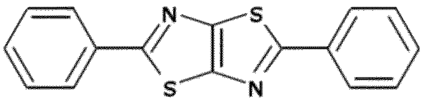 | B258 | 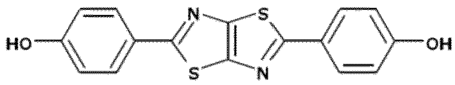 |
| B259 | 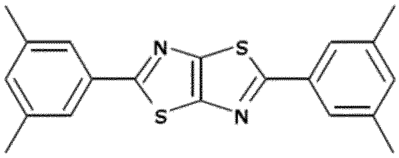 | B260 | 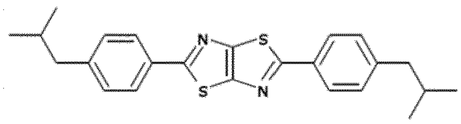 |
| B261 | 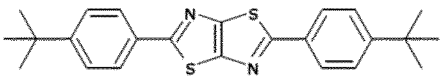 | B262 | 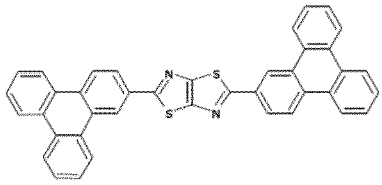 |
| B263 | 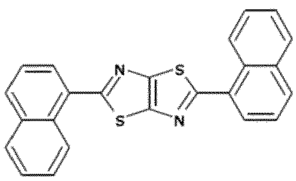 | B264 | 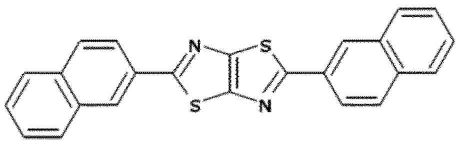 |
| B265 | 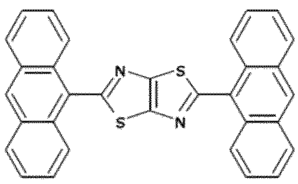 | B266 | 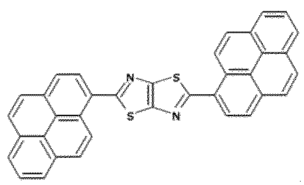 |

(continued)

| Table 31 | | | |
|---|---|---|---|
| **Number** | **Chemical structure** | **Number** | **Chemical structure** |
| **B267** | 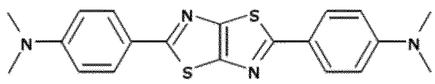 | **B268** | 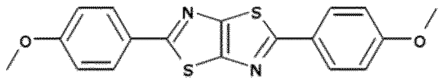 |
| **B269** | 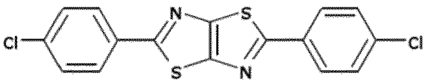 | **B270** | 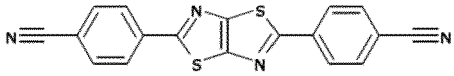 |
| **B271** | 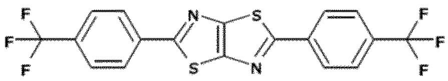 | **B272** | 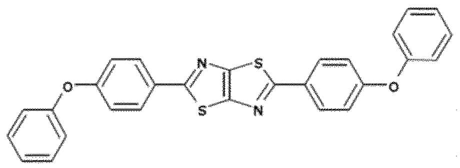 |

[Table 32]

| Table32 | | | |
|---|---|---|---|
| **Number** | **Chemical structure** | **Number** | **Chemical structure** |
| **B273** | 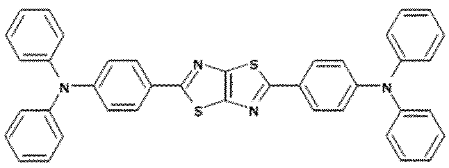 | **B274** | 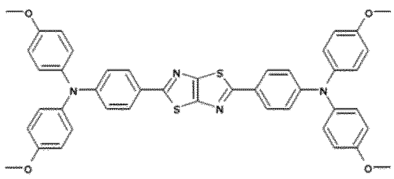 |
| **B275** | 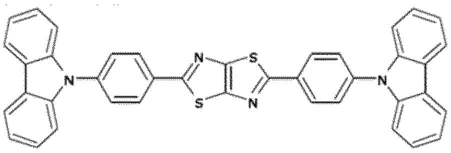 | **B276** | 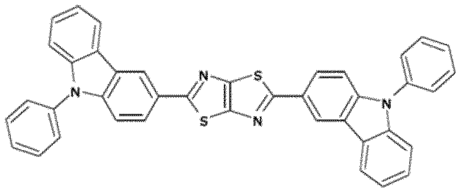 |
| **B277** | 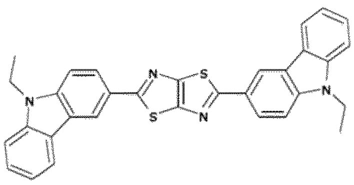 | **B278** | 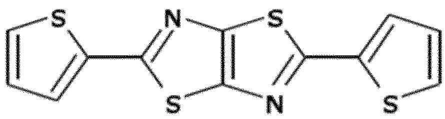 |
| **B279** | 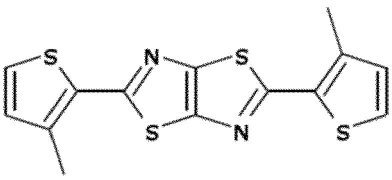 | **B280** | 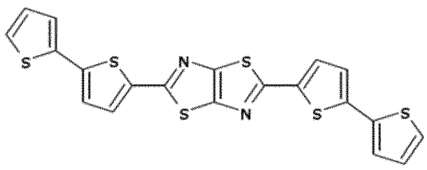 |

(continued)

| Table32 | | | |
|---|---|---|---|
| Number | Chemical structure | Number | Chemical structure |
| B281 | 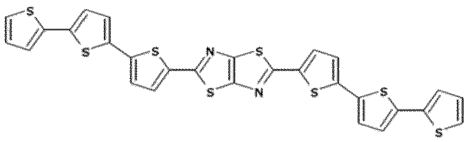 | B282 | 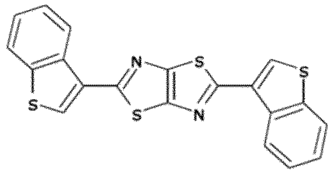 |
| B283 | 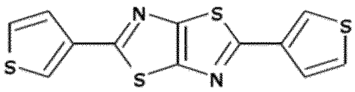 | B284 | 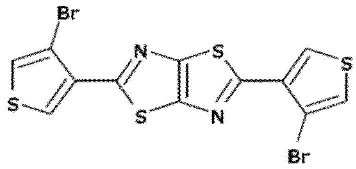 |
| B285 | 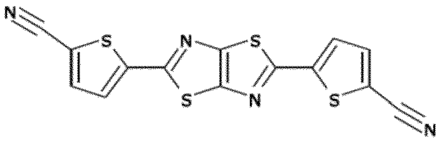 | B286 | 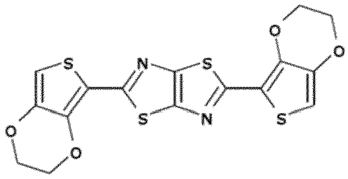 |
| B287 | 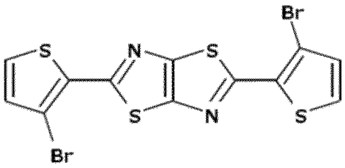 | B288 | 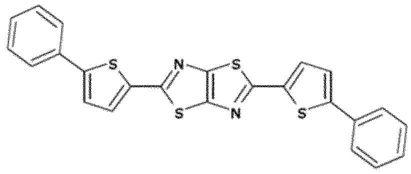 |

[Table 33]

| Table33 | | | |
|---|---|---|---|
| Number | Chemical structure | Number | Chemical structure |
| B289 | 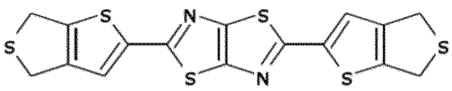 | B290 | 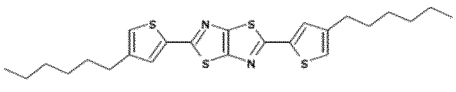 |
| B291 | 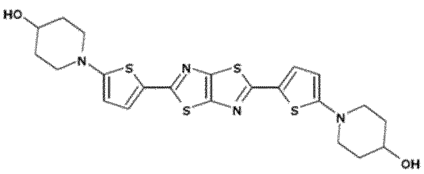 | B292 | 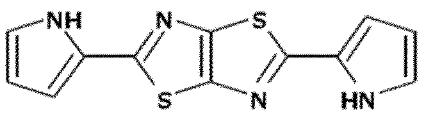 |
| B293 | 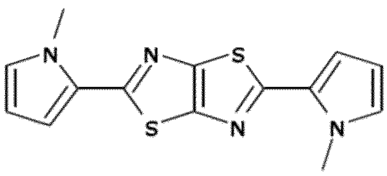 | B294 | 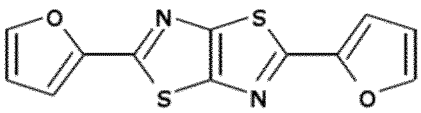 |

(continued)

Table33

| Number | Chemical structure | Number | Chemical structure |
|---|---|---|---|
| B295 | 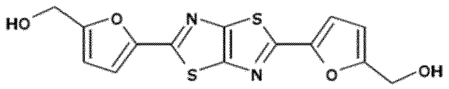 | B296 | 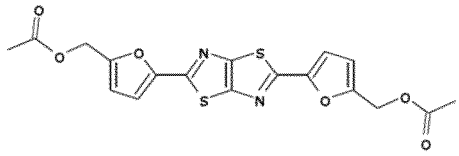 |
| B297 | 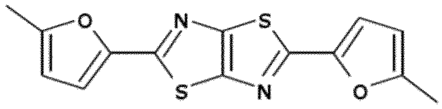 | B298 | 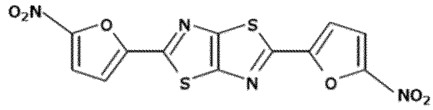 |
| B299 | 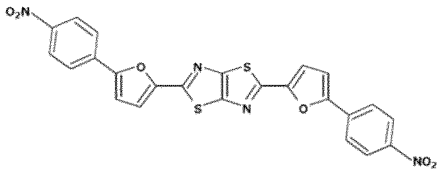 | B300 | 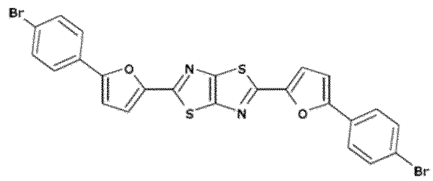 |
| B301 | 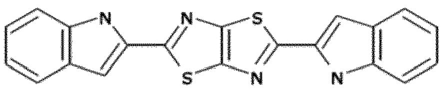 | B302 | 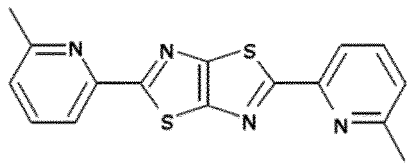 |
| B303 | 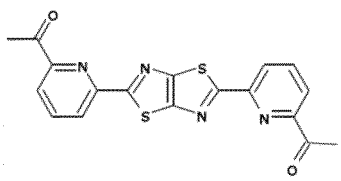 | B304 | 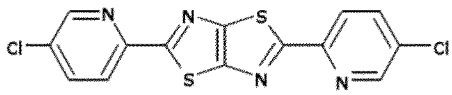 |

[Table 34]

Table34

| Number | Chemical structure | Number | Chemical structure |
|---|---|---|---|
| B305 | 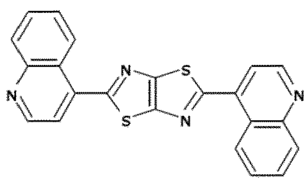 | B306 | 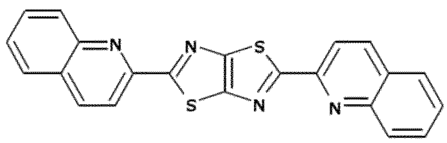 |
| B307 | 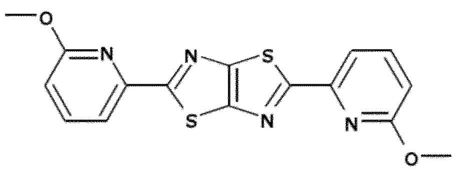 | B308 | 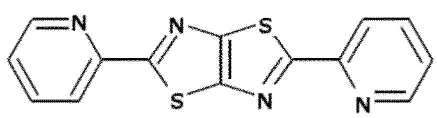 |

(continued)

| Table34 | | | |
|---|---|---|---|
| **Number** | **Chemical structure** | **Number** | **Chemical structure** |
| **B309** | 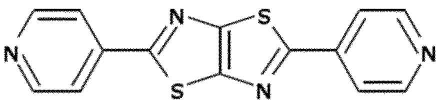 | **B310** | 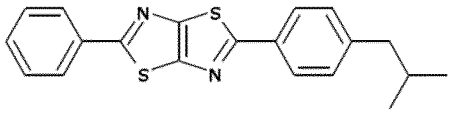 |
| **B311** | 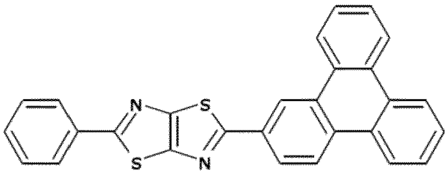 | **B312** | 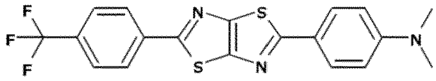 |
| **B313** | 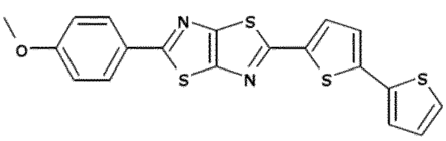 | **B314** | 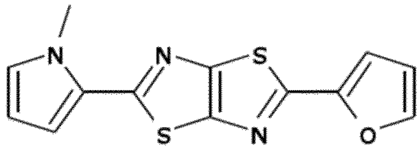 |
| **B315** | 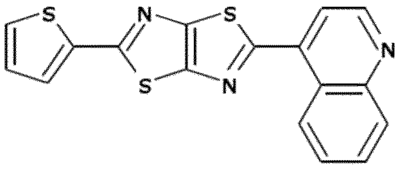 | | |

[Table 35]

| Table35 | | | |
|---|---|---|---|
| **Number** | **Chemical structure** | **Number** | **Chemical structure** |
| **B316** | 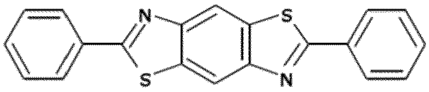 | **B317** | 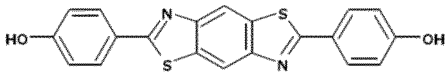 |
| **B318** | 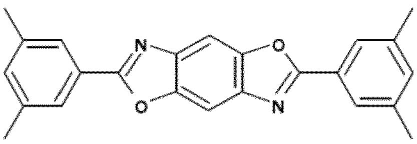 | **B319** | 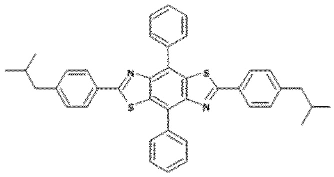 |
| **B320** | 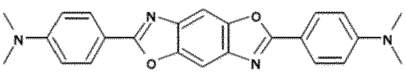 | **B321** | 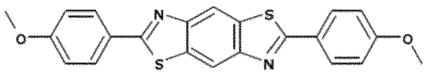 |
| **B322** | 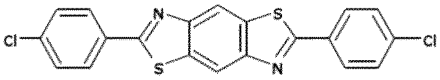 | **B323** | 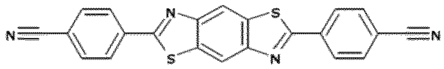 |
| **B324** | 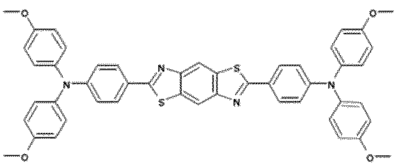 | **B325** | 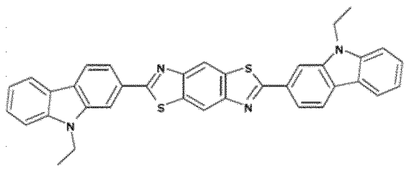 |

(continued)

| Table35 | | | |
|---|---|---|---|
| Number | Chemical structure | Number | Chemical structure |
| B326 | 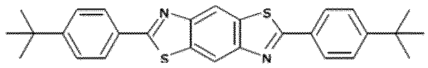 | B327 | 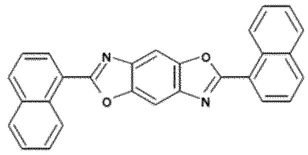 |
| B328 | 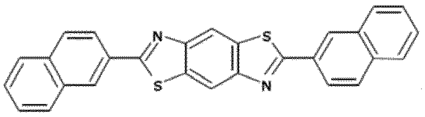 | B329 | 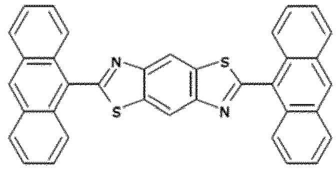 |
| B330 | 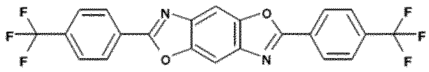 | B331 | 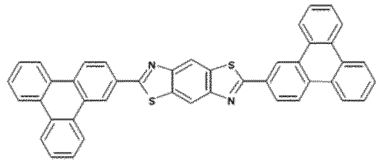 |

[Table 36]

| Table36 | | | |
|---|---|---|---|
| Number | Chemical structure | Number | Chemical structure |
| B332 | 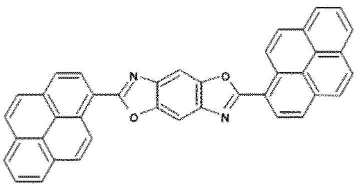 | B333 | 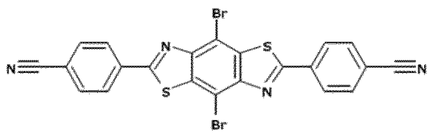 |
| B334 | 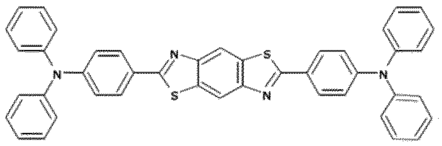 | B335 | 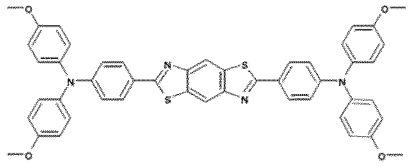 |
| B336 | 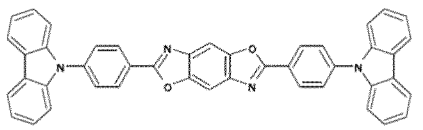 | B337 | 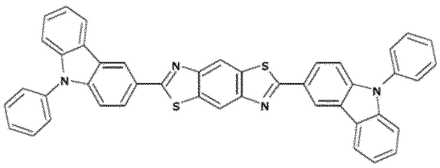 |
| B338 | 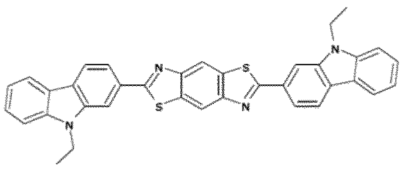 | B339 | 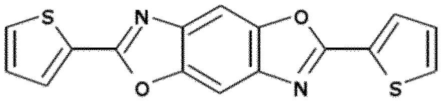 |

(continued)

| Table36 | | | |
|---|---|---|---|
| **Number** | **Chemical structure** | **Number** | **Chemical structure** |
| **B340** | 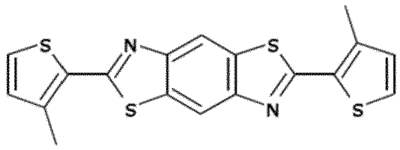 | **B341** | 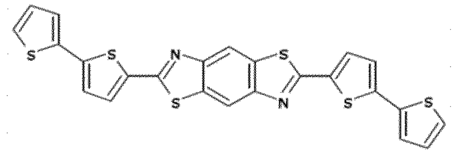 |
| **B342** | 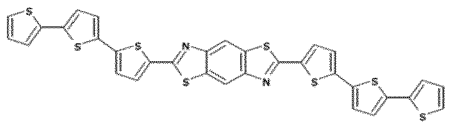 | **B343** | 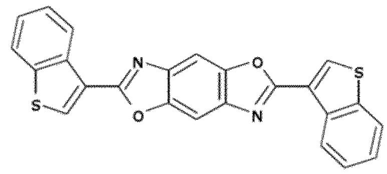 |
| **B344** | 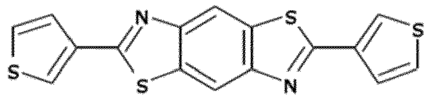 | **B345** | 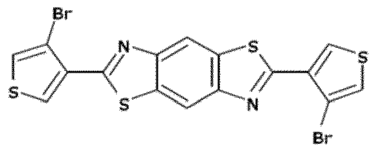 |
| **B346** | 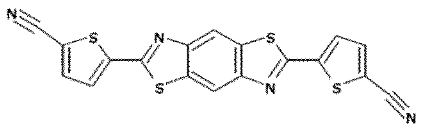 | **B347** | 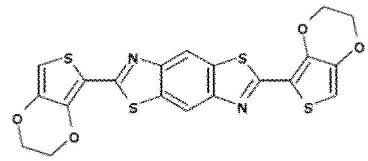 |

[Table 37]

| Table37 | | | |
|---|---|---|---|
| **Number** | **Chemical structure** | **Number** | **Chemical structure** |
| **B348** | 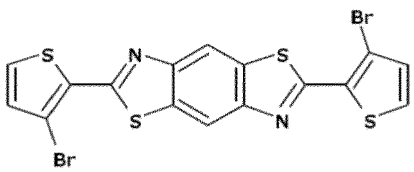 | **B349** | 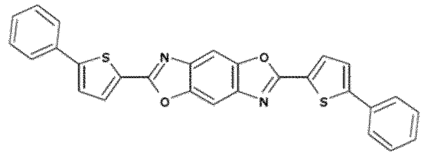 |
| **B350** | 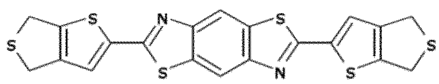 | **B351** | 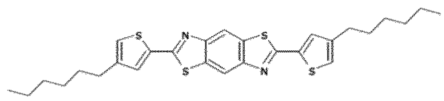 |
| **B352** | 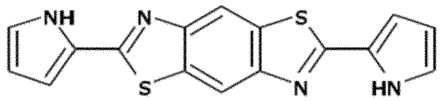 | **B353** | 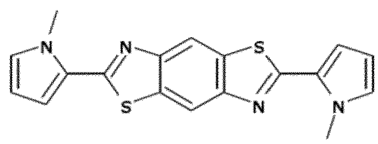 |
| **B354** | 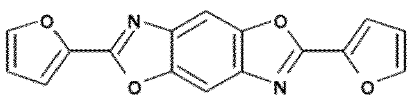 | **B355** | 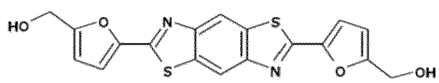 |

(continued)

| Table37 | | | |
|---|---|---|---|
| **Number** | **Chemical structure** | **Number** | **Chemical structure** |
| **B356** | 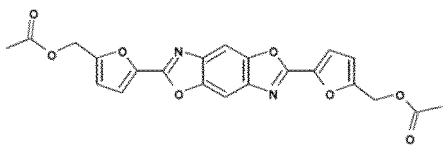 | **B357** | 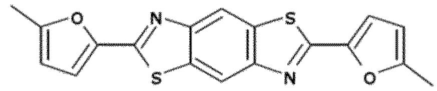 |
| **B358** | 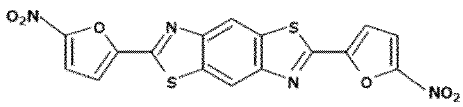 | **B359** | 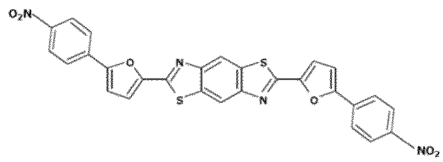 |
| **B360** | 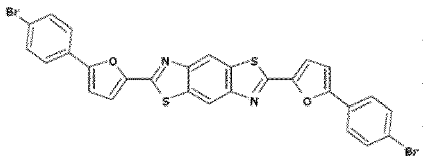 | **B361** | 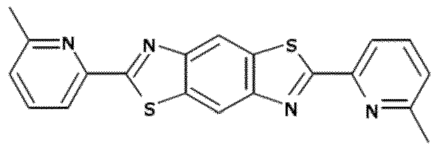 |
| **B362** | 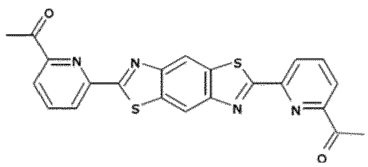 | **B363** | 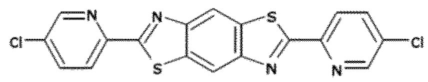 |

[Table 38]

| Table 38 | | | |
|---|---|---|---|
| **Number** | **Chemical structure** | **Number** | **Chemical structure** |
| **B364** | 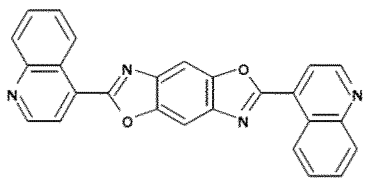 | **B365** | 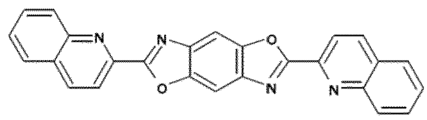 |
| **B366** | 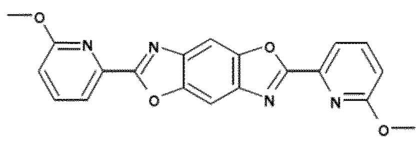 | **B367** | 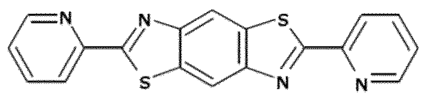 |
| **B368** | 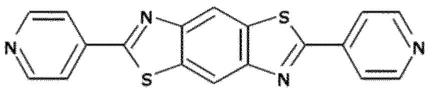 | **B369** | 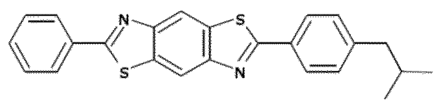 |
| **B370** | 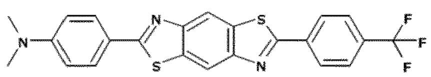 | **B371** | 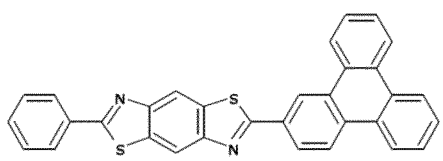 |

(continued)

| Table 38 | | | |
|---|---|---|---|
| **Number** | **Chemical structure** | **Number** | **Chemical structure** |
| **B372** | 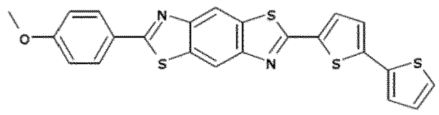 | **B373** | 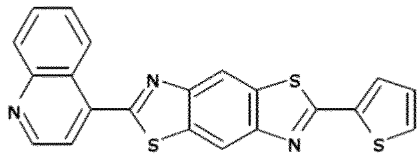 |
| **B374** | 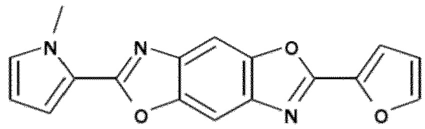 | **B375** | 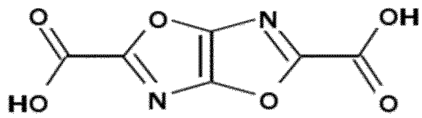 |
| **B376** | 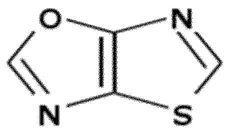 | **B377** | 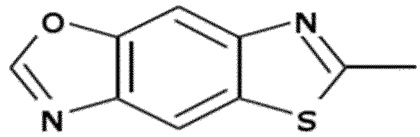 |

[Table 39]

**Table39**

| Number | Chemical structure | Number | Chemical structure |
|---|---|---|---|
| **B378** | 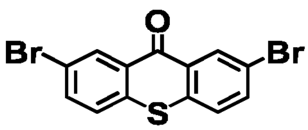 | **B379** | 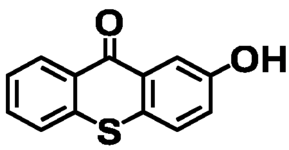 |
| **B380** | 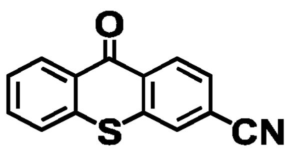 | **B381** | 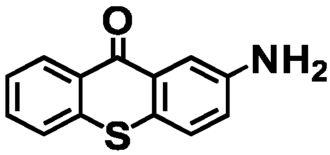 |
| **B382** | 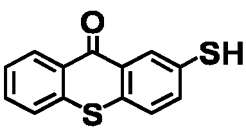 | **B383** | 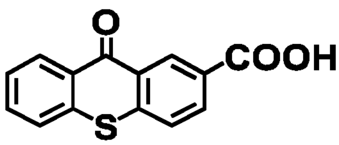 |
| **B384** | 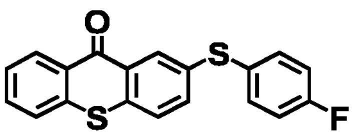 | **B385** | 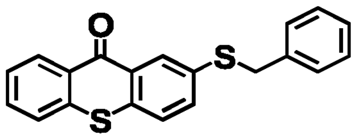 |
| **B386** | 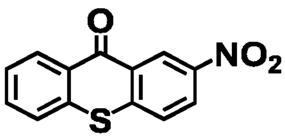 | **B387** | 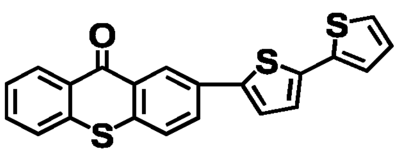 |

(continued)

| Number | Chemical structure | Number | Chemical structure |
|---|---|---|---|
| B388 | 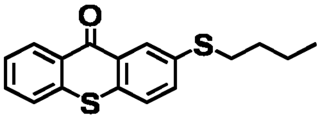 | B389 | 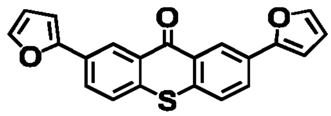 |
| B390 | 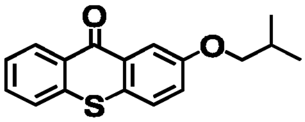 | B391 | 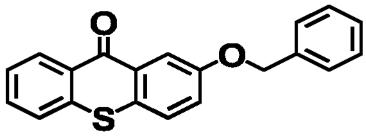 |
| B392 | 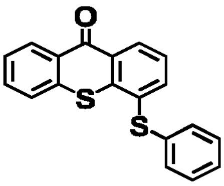 | B393 | 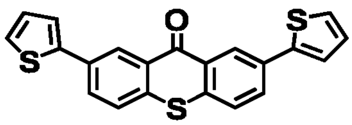 |

[Table 40]

Table40

| Number | Chemical structure | Number | Chemical structure |
|---|---|---|---|
| B394 | 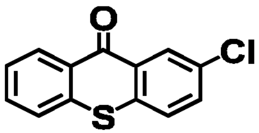 | B395 | 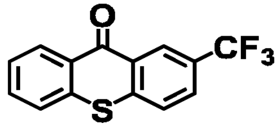 |
| B396 | 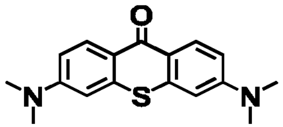 | B397 | 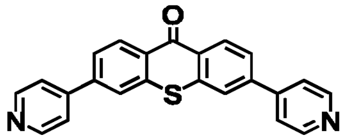 |
| B398 | 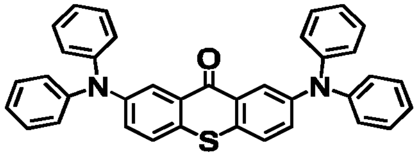 | B399 | 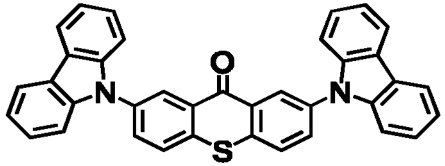 |
| B400 | 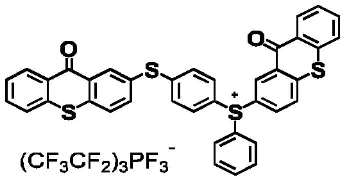 $(CF_3CF_2)_3PF_3^-$ | B401 | 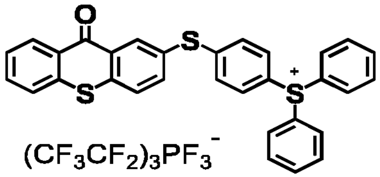 $(CF_3CF_2)_3PF_3^-$ |
| B402 | 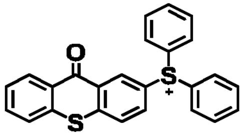 $(CF_3CF_2)_3PF_3^-$ | B403 | 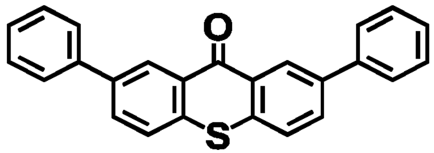 |

(continued)

| Number | Chemical structure | Number | Chemical structure |
|---|---|---|---|
| B404 | 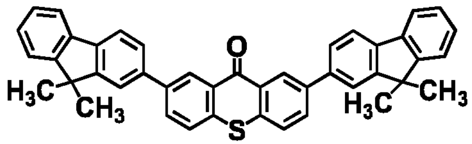 | B405 | 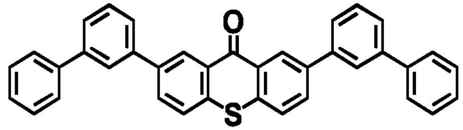 |
| B406 | 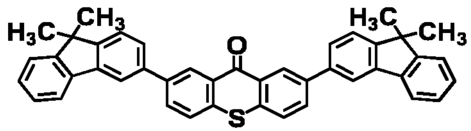 | B407 | 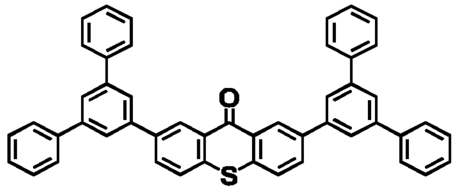 |
| B408 | 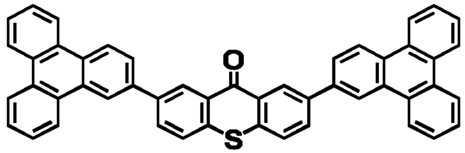 | B409 | 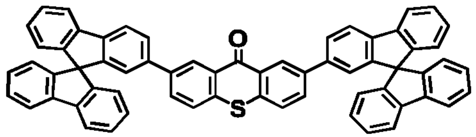 |

[Table 41]

Table41

| Number | Chemical structure | Number | Chemical structure |
|---|---|---|---|
| B410 | 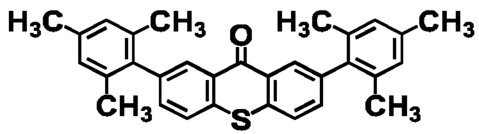 | B411 | 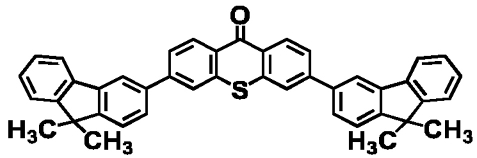 |
| B412 | 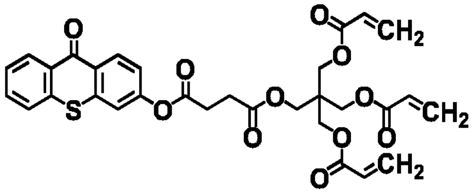 | B413 | 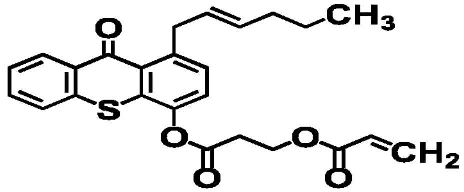 |
| B414 | 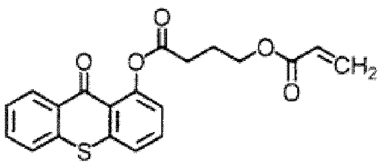 | B415 | 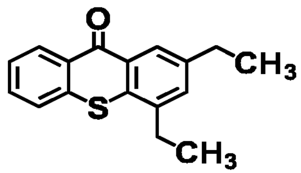 |
| B416 | 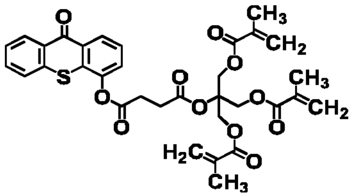 | B417 | 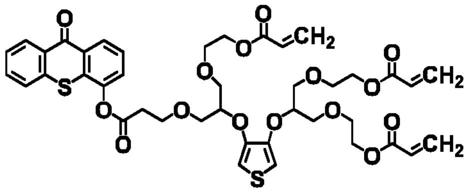 |
| B418 | 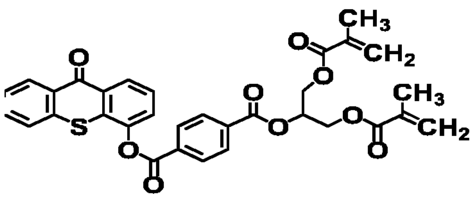 | B419 | 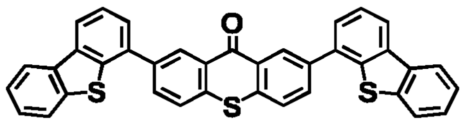 |

(continued)

| Number | Chemical structure | Number | Chemical structure |
|---|---|---|---|
| B420 | 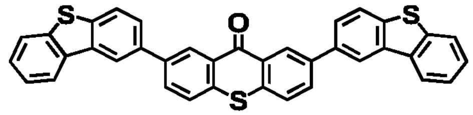 | B421 | 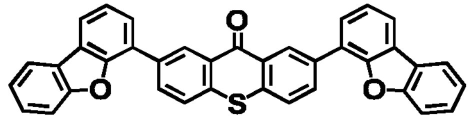 |
| B422 | 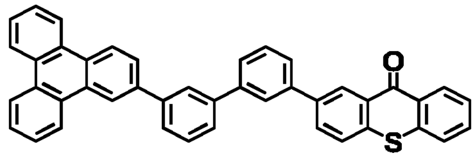 | B423 | 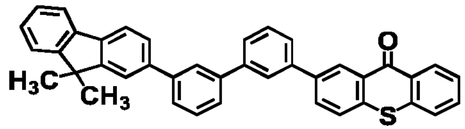 |
| B424 | 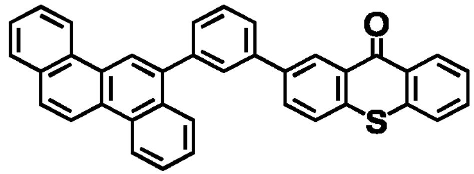 | B425 | 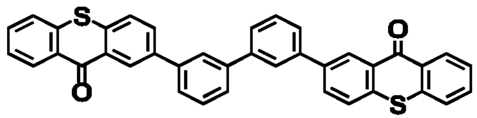 |

[Table 42]

Table42

| Number | Chemical structure | Number | Chemical structure |
|---|---|---|---|
| B426 | 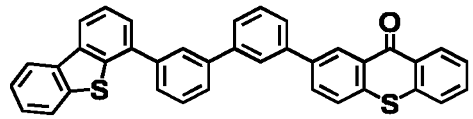 | B427 | 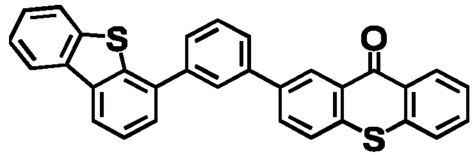 |
| B428 | 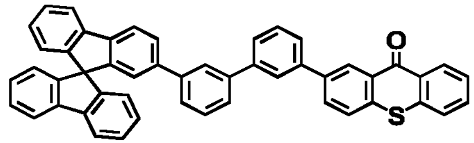 | B429 | 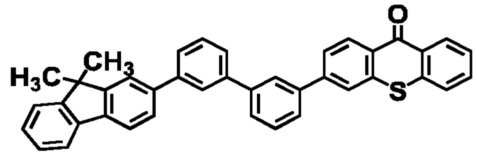 |
| B430 | 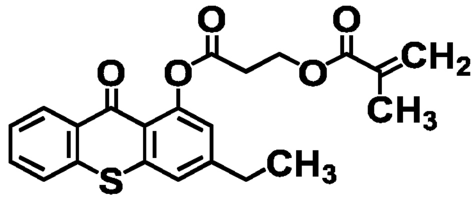 | B431 | 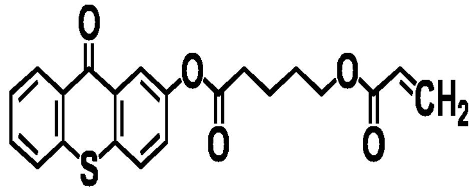 |
| B432 | 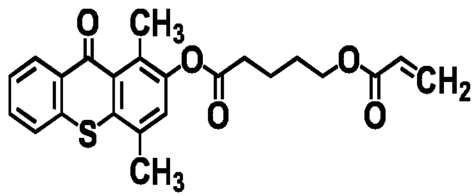 | B433 | 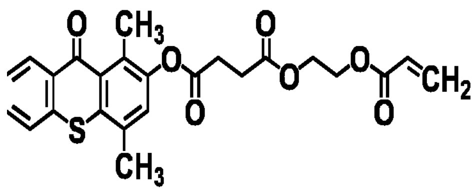 |
| B434 | 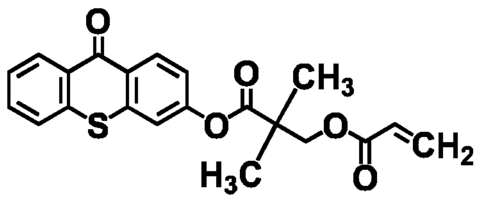 | B435 | 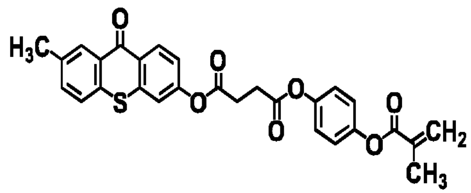 |

(continued)

| Number | Chemical structure | Number | Chemical structure |
|---|---|---|---|
| B436 | 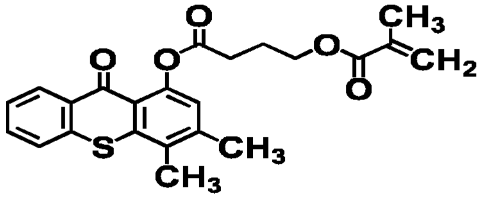 | B437 | 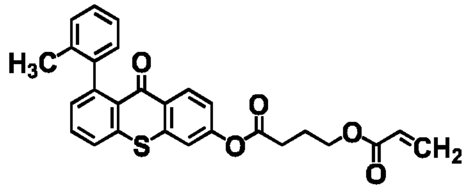 |
| B438 | 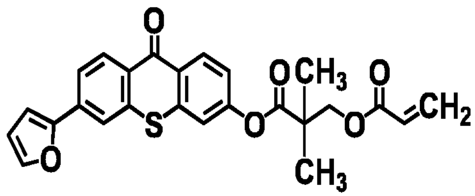 | B439 | 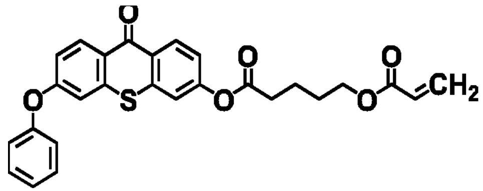 |
| B440 | 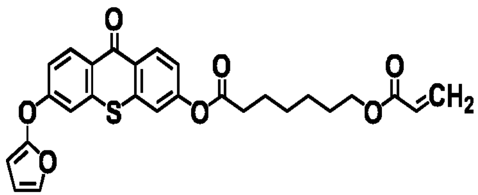 | B441 | 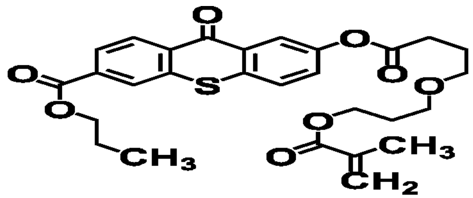 |

[Table 43]

Table43

| Number | Chemical structure | Number | Chemical structure |
|---|---|---|---|
| B442 | 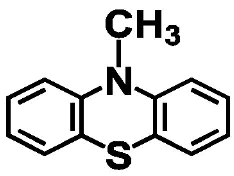 | B443 | 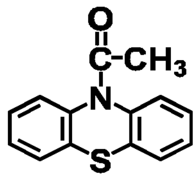 |
| B444 | 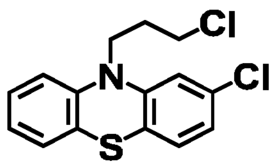 | B445 | 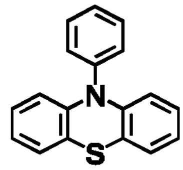 |
| B446 | 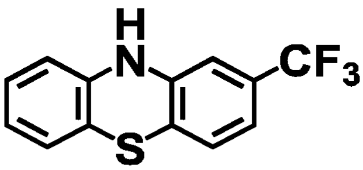 | B447 | 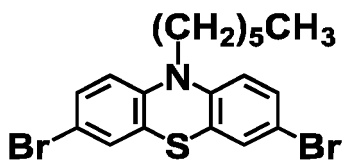 |
| B448 | 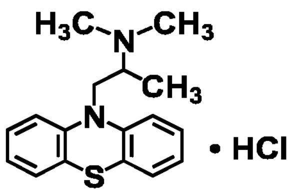 | B449 | 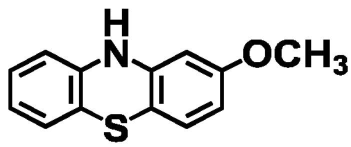 |
| B450 | 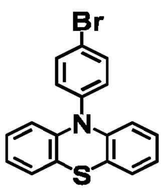 | B451 | 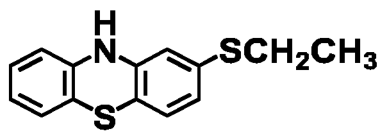 |
| B452 | 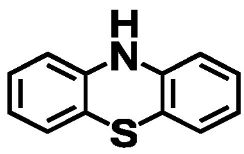 | B453 | 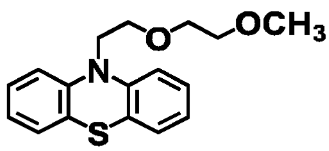 |
| B454 | 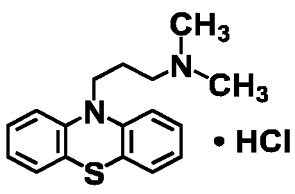 | B455 | 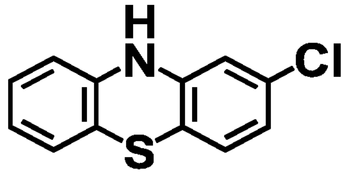 |

(continued)

| Number | Chemical structure | Number | Chemical structure |
|---|---|---|---|
| B456 | 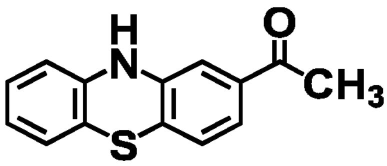 | B457 | 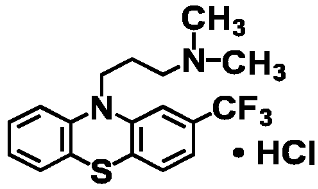 |

[Table 44]

Table44

| Number | Chemical structure | Number | Chemical structure |
|---|---|---|---|
| B458 | 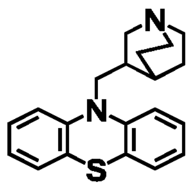 | B459 | 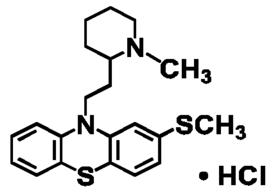 |
| B460 | 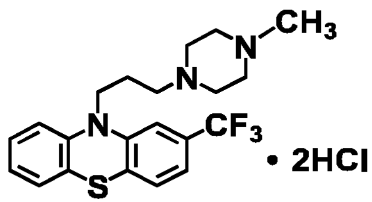 | B461 | 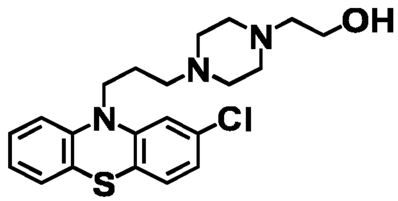 |
| B462 | 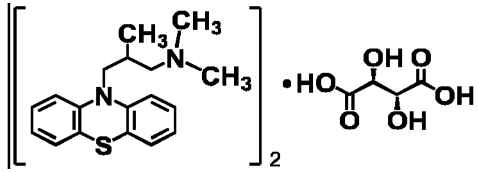 | B463 | 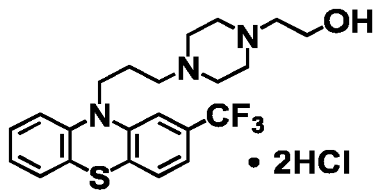 |
| B464 | 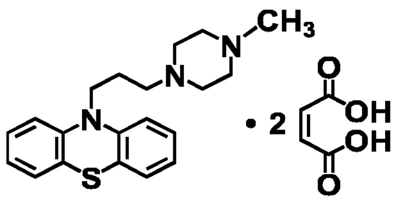 | B465 | 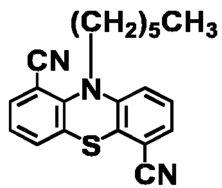 |
| B466 | 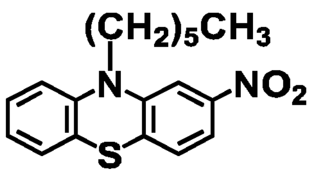 | B467 | 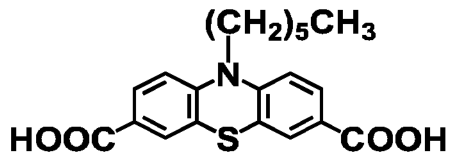 |

(continued)

| Number | Chemical structure | Number | Chemical structure |
|---|---|---|---|
| B468 | 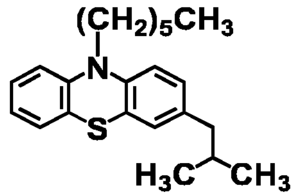 | B469 | 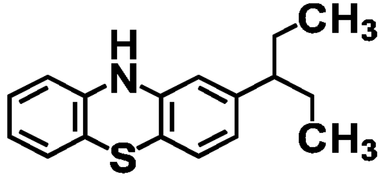 |
| B470 | 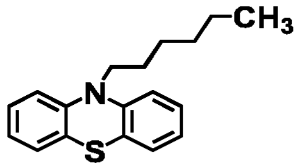 | B471 | 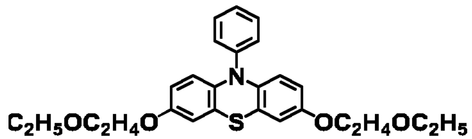 |
| B472 | 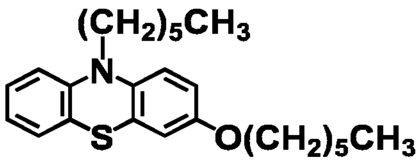 | B473 | 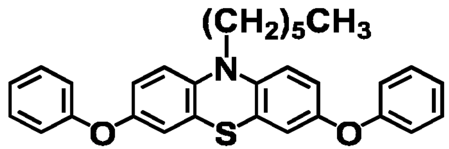 |

[Table 45]

Table45

| Number | Chemical structure | Number | Chemical structure |
|---|---|---|---|
| B474 | 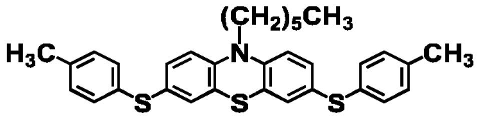 | B475 | 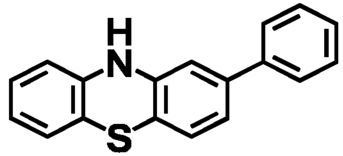 |
| B476 | 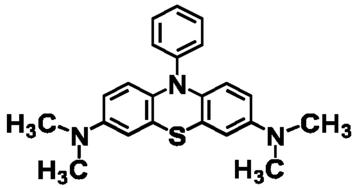 | B477 | 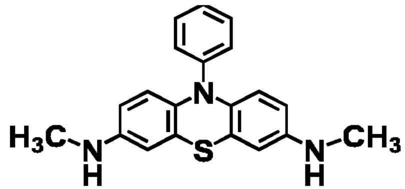 |
| B478 | 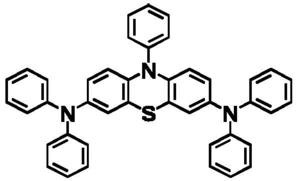 | B479 | 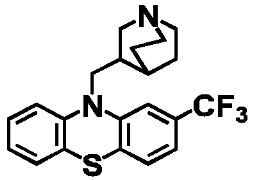 |
| B480 | 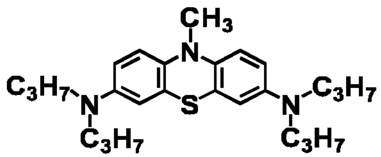 | B481 | 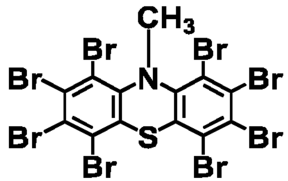 |
| B482 | 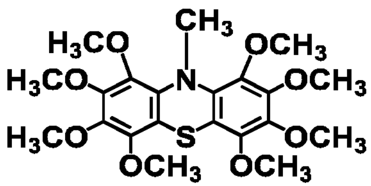 | B483 | 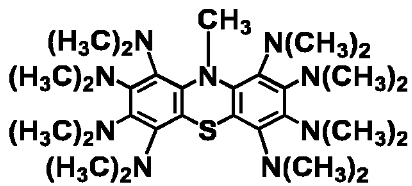 |

(continued)

| Number | Chemical structure | Number | Chemical structure |
|---|---|---|---|
| B484 | 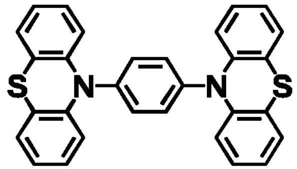 | B485 | 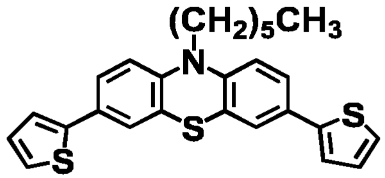 |
| B486 | 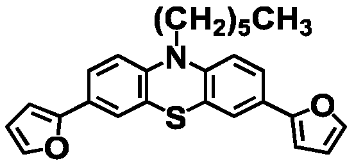 | B487 | 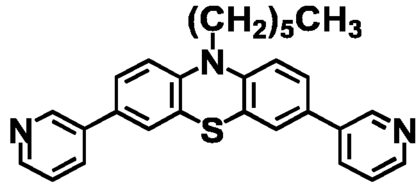 |
| B488 | 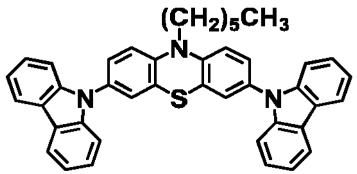 | B489 | 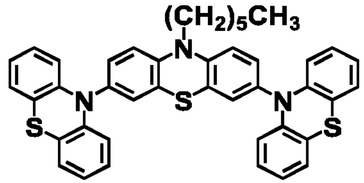 |

[Table 46]

**Table46**

| Number | Chemical structure | Number | Chemical structure |
|---|---|---|---|
| B490 | 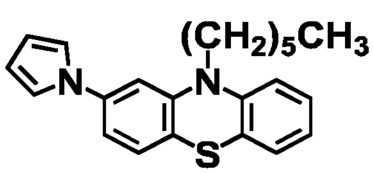 | B491 | 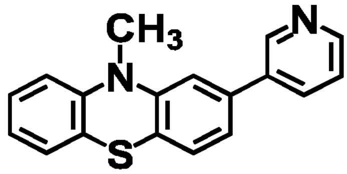 |
| B492 | 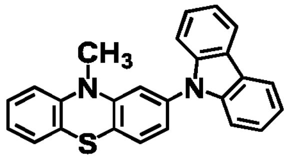 | B493 | 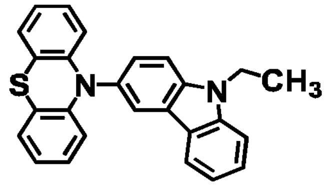 |
| B494 | 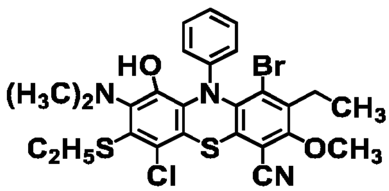 | B495 | 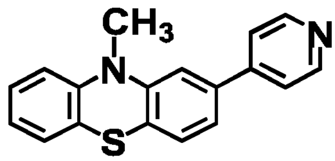 |
| B496 | 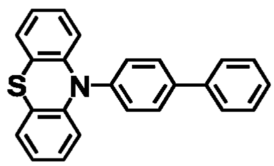 | B497 | 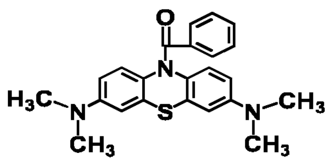 |
| B498 | 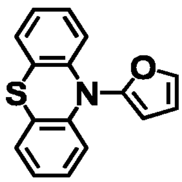 | B499 | 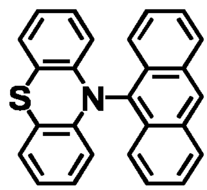 |
| B500 | 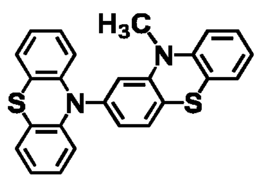 | B501 | 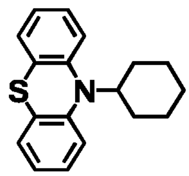 |
| B502 | 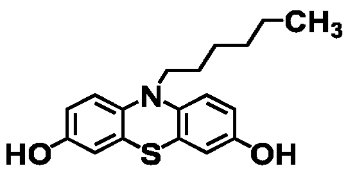 | B503 | 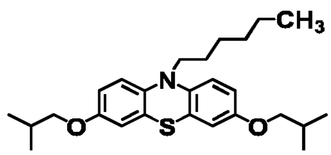 |

(continued)

| Number | Chemical structure | Number | Chemical structure |
|---|---|---|---|
| B504 | 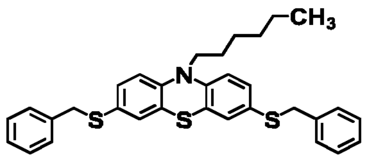 | B505 | 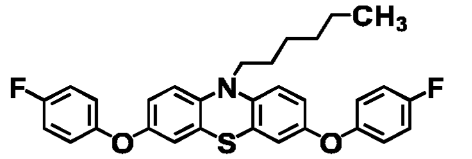 |

[Table 47]

**Table47**

| Number | Chemical structure | Number | Chemical structure |
|---|---|---|---|
| B506 | 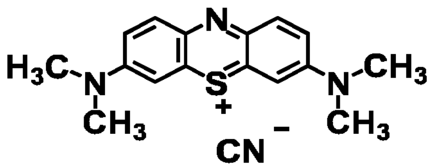 | B507 | 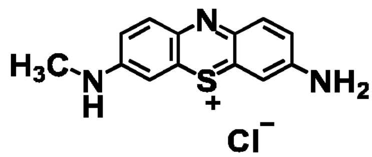 |
| B508 | 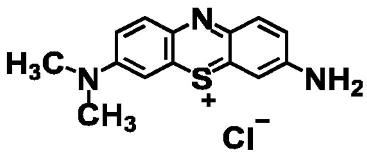 | B509 | 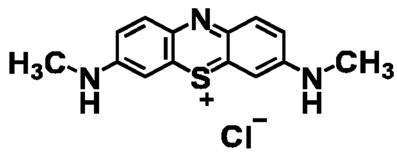 |
| B510 | 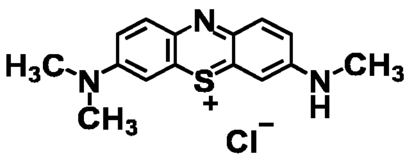 | B511 | 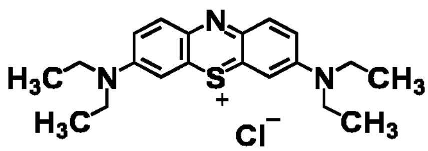 |
| B512 | 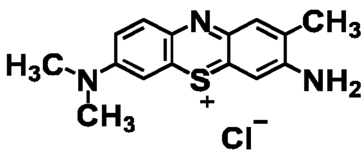 | B513 | 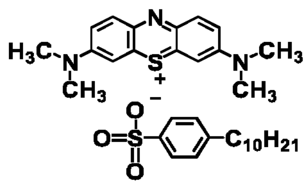 |
| B514 | 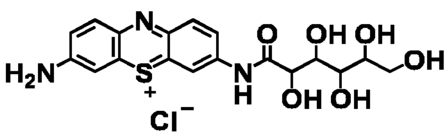 | B515 | 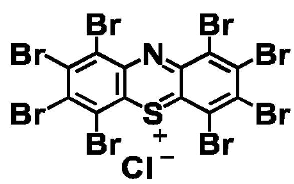 |
| B516 | 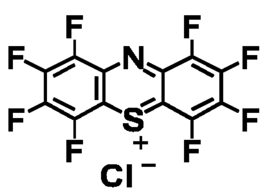 | B517 | 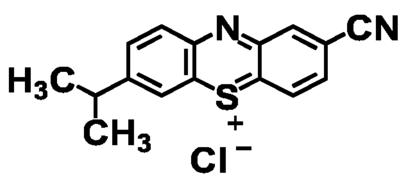 |

(continued)

| Number | Chemical structure | Number | Chemical structure |
|---|---|---|---|
| B518 | 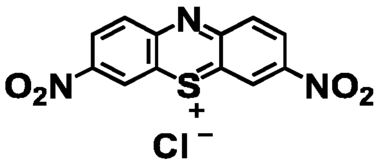 | B519 | 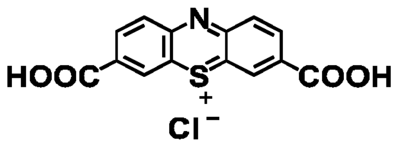 |
| B520 | 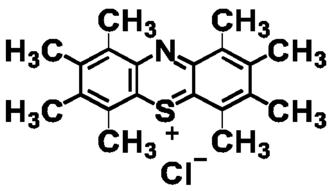 | B521 | 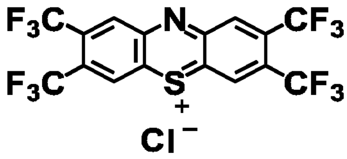 |

[Table 48]

**Table48**

| Number | Chemical structure | Number | Chemical structure |
|---|---|---|---|
| B522 | 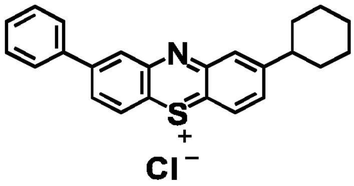 | B523 | 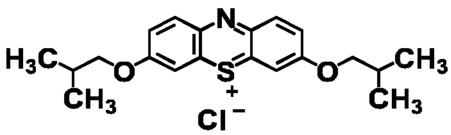 |
| B524 | 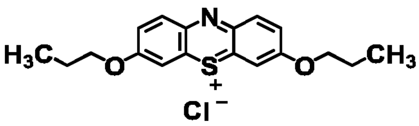 | B525 | 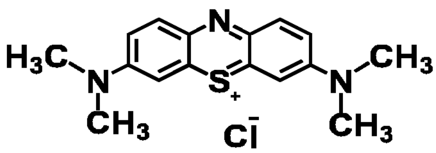 |
| B526 | 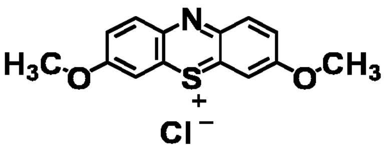 | B527 | 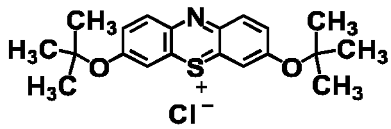 |
| B528 | 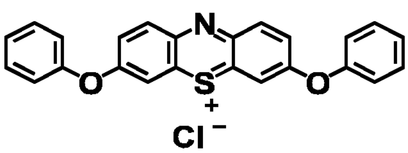 | B529 | 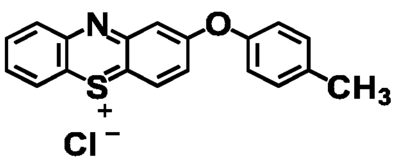 |
| B530 | 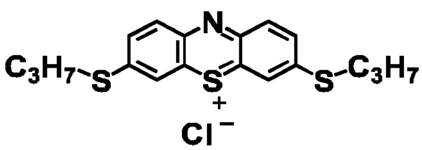 | B531 | 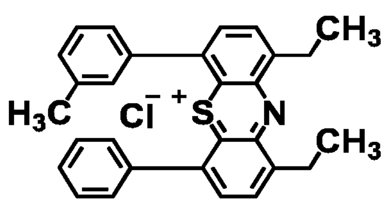 |

(continued)

| Number | Chemical structure | Number | Chemical structure |
|---|---|---|---|
| B532 | 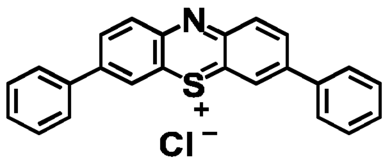 | B533 | 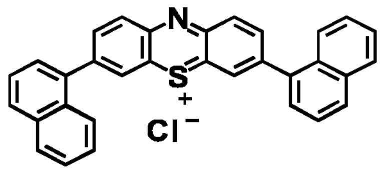 |
| B534 | 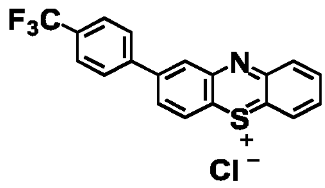 | B535 | 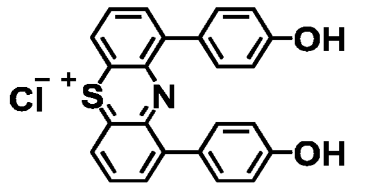 |
| B536 | 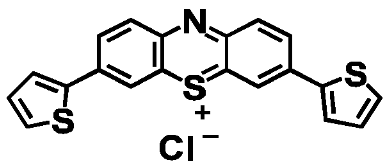 | B537 | 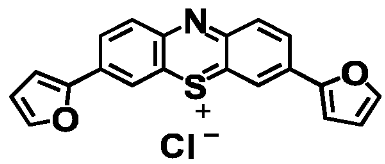 |

[Table 49]

**Table49**

| Number | Chemical structure | Number | Chemical structure |
|---|---|---|---|
| B538 | 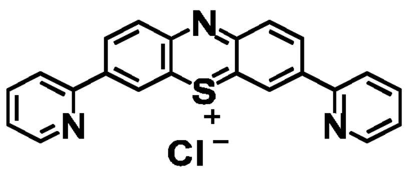 | B539 | 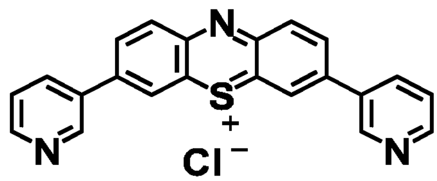 |
| B540 | 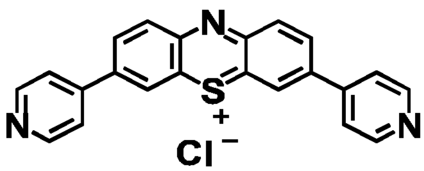 | B541 | 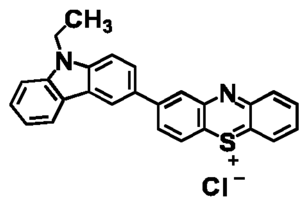 |
| B542 | 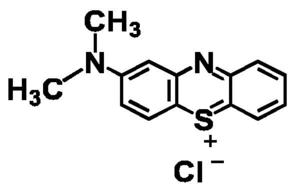 | B543 | 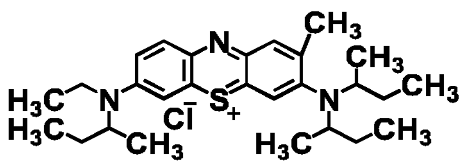 |
| B544 | 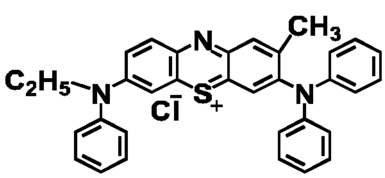 | B545 | 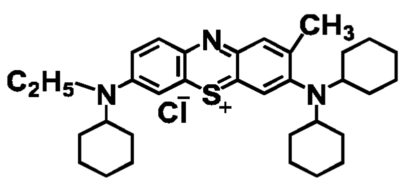 |

(continued)

| Number | Chemical structure | Number | Chemical structure |
|---|---|---|---|
| B546 | 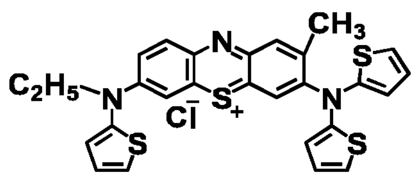 | B547 | 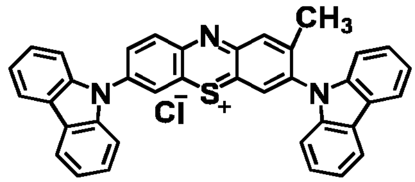 |
| B548 | 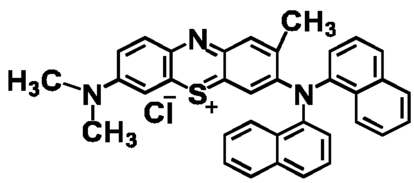 | B549 | 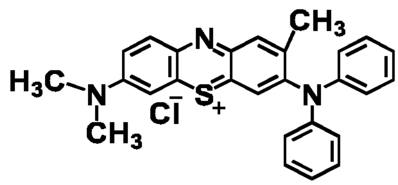 |
| B550 | 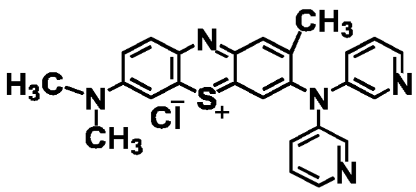 | B551 | 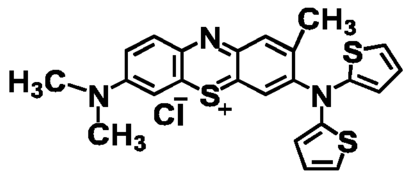 |
| B552 | 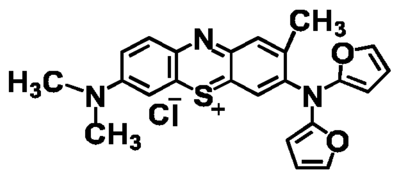 | B553 | 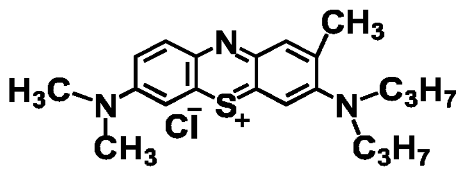 |

[Table 50]

Table50

| Number | Chemical structure | Number | Chemical structure |
|---|---|---|---|
| B554 | 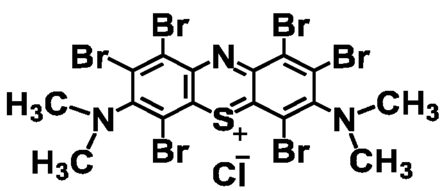 | B555 | 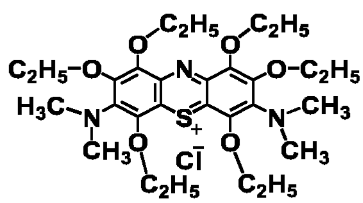 |
| B556 | 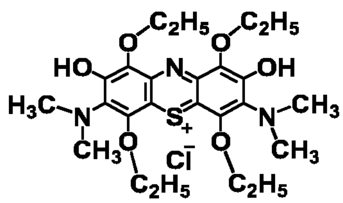 | B557 | 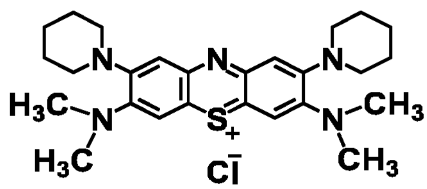 |
| B558 | 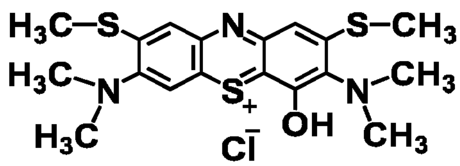 | B559 | 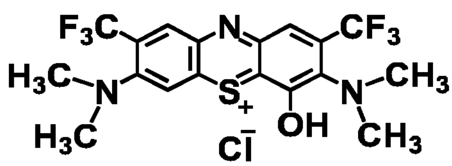 |

(continued)

| Number | Chemical structure | Number | Chemical structure |
|---|---|---|---|
| B560 | 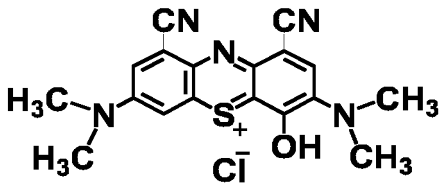 | B561 | 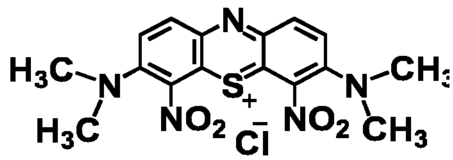 |
| B562 | 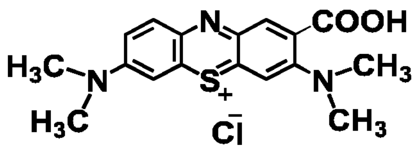 | B563 | 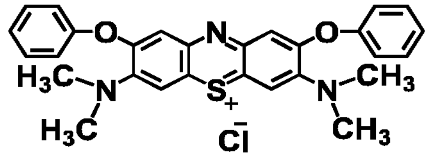 |
| B564 | 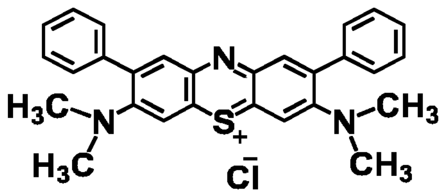 | B565 | 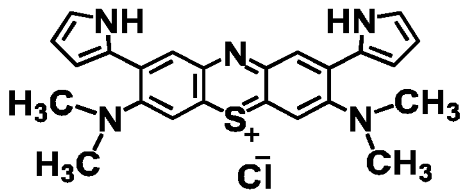 |
| B566 | 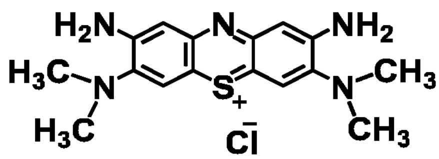 | B567 | 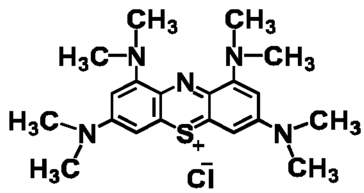 |
| B568 | 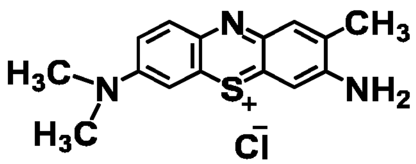 | B569 | 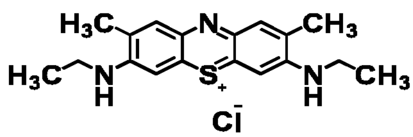 |

[Table 51]

Table51

| Number | Chemical structure | Number | Chemical structure |
|---|---|---|---|
| B570 | 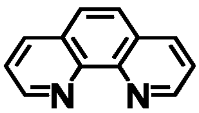 | B571 | 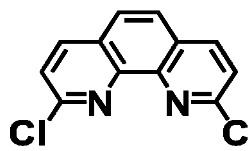 |
| B572 | 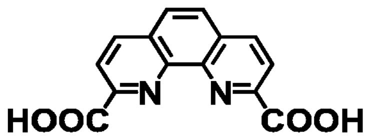 | B573 | 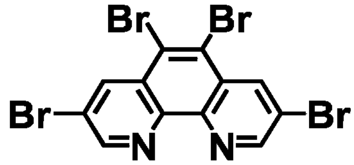 |

(continued)

| Number | Chemical structure | Number | Chemical structure |
|---|---|---|---|
| B574 | 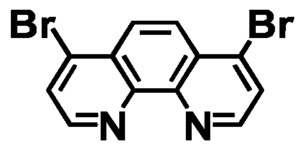 | B575 | 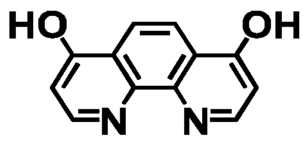 |
| B576 | 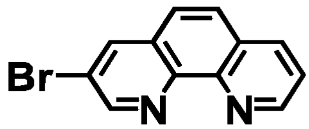 | B577 | 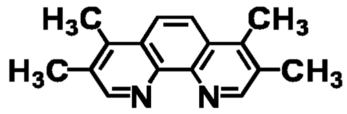 |
| B578 | 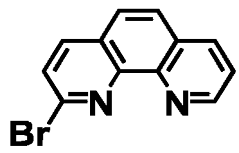 | B579 | 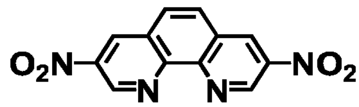 |
| B580 | 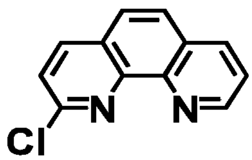 | B581 | 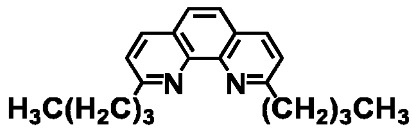 |
| B582 | 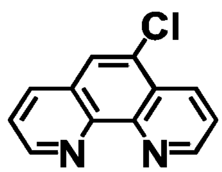 | B583 | 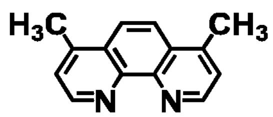 |
| B584 | 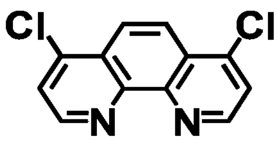 | B585 | 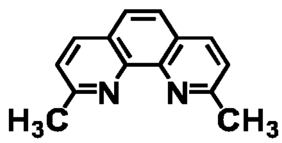 |

[Table 52]

**Table52**

| Number | Chemical structure | Number | Chemical structure |
|---|---|---|---|
| B586 | 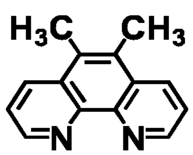 | B587 | 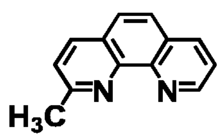 |
| B588 | 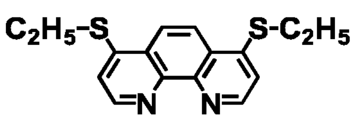 | B589 | 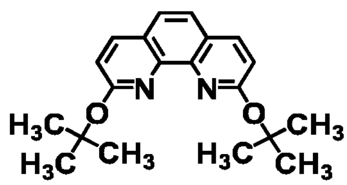 |

(continued)

| Number | Chemical structure | Number | Chemical structure |
|---|---|---|---|
| B590 | 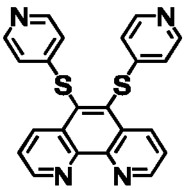 | B591 | 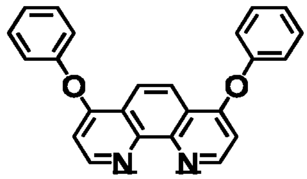 |
| B592 | 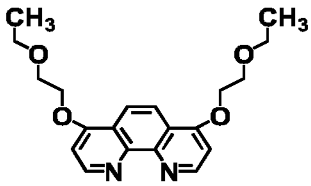 | B593 | 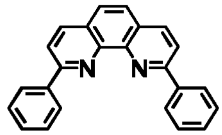 |
| B594 | 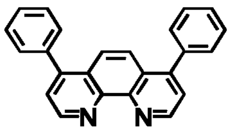 | B595 | 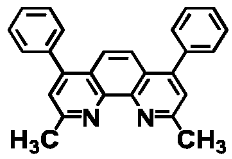 |
| B596 | 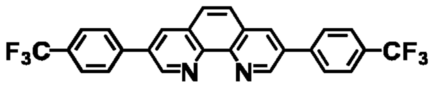 | B597 | 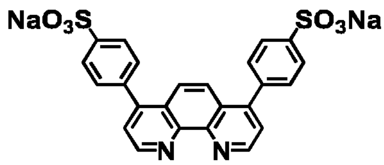 |
| B598 | 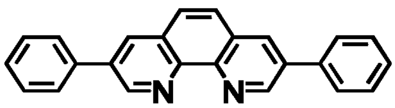 | B599 | 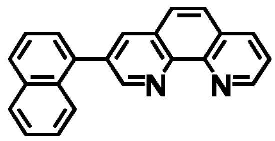 |
| B600 | 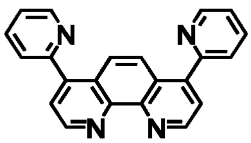 | B601 | 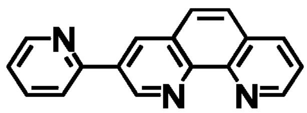 |

[Table 53]

**Table53**

| Number | Chemical structure | Number | Chemical structure |
|---|---|---|---|
| B602 | 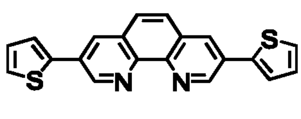 | B603 | 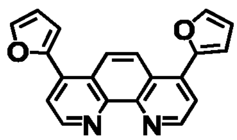 |

(continued)

| Number | Chemical structure | Number | Chemical structure |
|---|---|---|---|
| B604 | 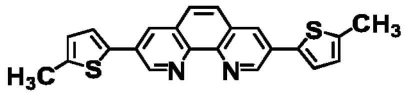 | B605 | 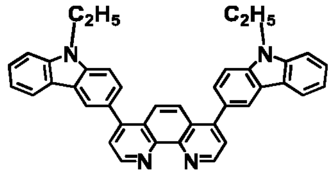 |
| B606 | 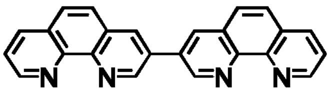 | B607 | 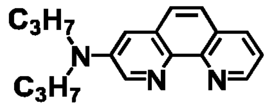 |
| B608 | 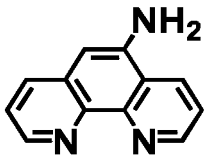 | B609 | 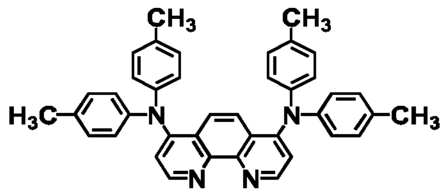 |
| B610 | 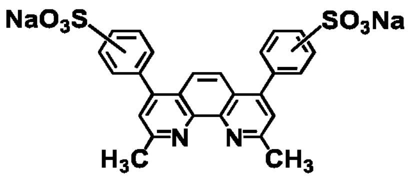 | B611 | 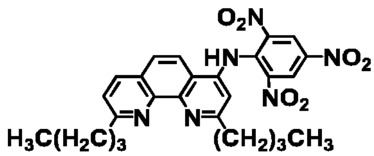 |
| B612 | 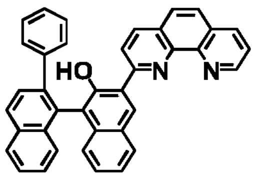 | B613 | 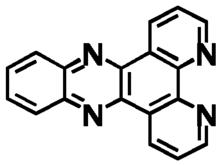 |
| B614 | 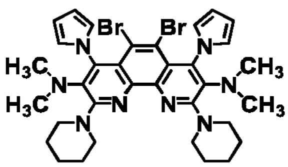 | B615 | 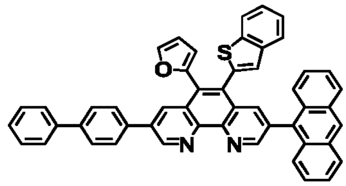 |
| B616 | 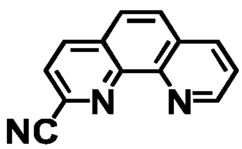 | B617 | 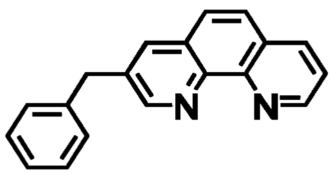 |

[Table 54]

**Table54**

| Number | Chemical structure | Number | Chemical structure |
|---|---|---|---|
| B618 | 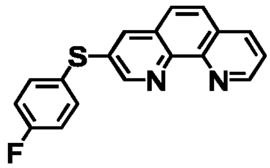 | B619 | 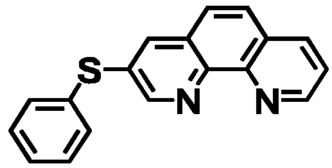 |
| B620 | 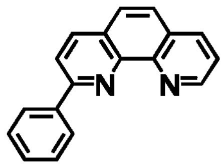 | B621 | 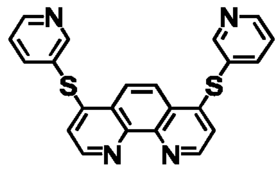 |
| B622 | 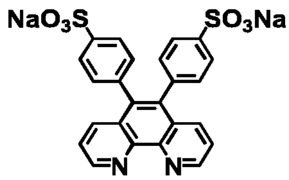 | B623 | 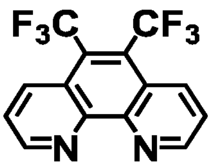 |
| B624 | 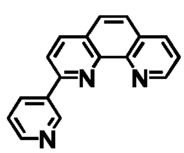 | B625 | 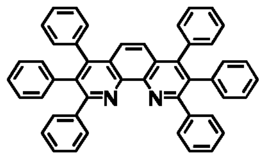 |
| B626 | 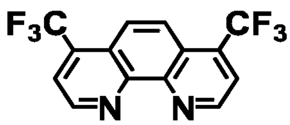 | B627 | 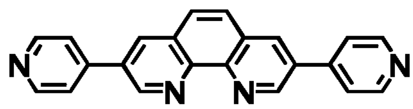 |
| B628 | 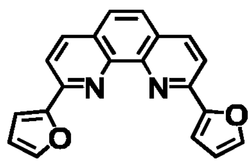 | B629 | 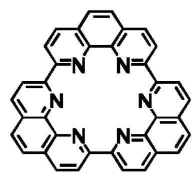 |
| B630 | 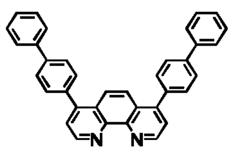 | B631 | 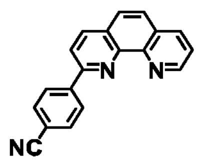 |
| B632 | 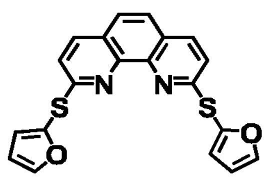 | B633 | 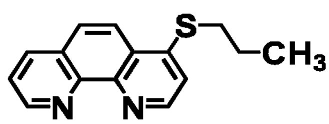 |

<Other Components>

The thermoelectric conversion material of this embodiment may contain an additional component as needed from the viewpoint of improving its properties. For example, it is possible to further improve coating properties, electroconductive properties, and thermoelectric properties by adding an auxiliary agent exemplified below.

(Solvent)

The solvent used in this embodiment is used as a medium for mixing the electrically conductive material (A) and the organic compound (B), and the coating properties can be improved by forming an ink. The solvent to be used is not particularly limited as long as the electrically conductive material (A) and the organic compound (B) can be dissolved or dispersed well. Examples of the solvents include organic solvents and water. Two or more solvents may be used in combination.

The organic solvent can be appropriately selected from the group consisting of alcohols, such as methanol, ethanol, propanol, butanol, ethylene glycol methyl ether, diethylene glycol methyl ether, terpineol, dihydroterpineol, 2,4-diethyl-1,5-pentanediol, 1,3-butylene glycol, isobornylcyclohexanol, ethylene glycol, 1,3-propanediol, 1,4-butanediol, diethylene glycol, triethylene glycol, glycerol, polyethylene glycol, polypropylene glycol, trifluoroethanol, m-cresol, and thiodiglycol; ketones such as acetone, methyl ethyl ketone, methyl isobutyl ketone, and cyclohexanone; ethers such as tetrahydrofuran, dioxane, ethylene glycol dimethyl ether, diethylene glycol dimethyl ether; hydrocarbons such as hexane, heptane, and octane; aromatic compounds such as benzene, toluene, xylene, and cumene; esters such as ethyl acetate, and butyl acetate; N-methylpyrrolidone, and the like, as necessary. As the solvent, N-methylpyrrolidone is particularly preferable.

(Auxiliary Agent)

Examples of the auxiliary agents that can be used include, but are not limited to, lactams, alcohols, amino alcohols, carboxylic acids, acid anhydrides, and ionic liquids. Specific examples are as follows.

Lactams: N-methylpyrrolidone, pyrrolidone, caprolactam, N-methylcaprolactam, N-octylpyrrolidone and the like.

Alcohols: sucrose, glucose, fructose, lactose, sorbitol, mannitol, xylitol, ethylene glycol, 1,3-propanediol, 1,4-butanediol, diethylene glycol, triethylene glycol, glycerol, polyethylene glycol, polypropylene glycol, trifluoroethanol, m-cresol, thiodiglycol and the like.

Amino alcohols: diethanolamine, triethanolamine, and the like.

Carboxylic acids: 2-furancarboxylic acid, 3-furancarboxylic acid, dichloroacetic acid, trifluoroacetic acid, and the like.

Acid anhydrides: acetic anhydride, propionic anhydride, acrylic anhydride, methacrylic anhydride, benzoic anhydride, succinic anhydride, maleic anhydride, itaconic anhydride, glutaric anhydride, phthalic anhydride, tetrahydrophthalic anhydride, hexahydrophthalic anhydride (also known as cyclohexane-1,2-dicarboxylic anhydride), trimellitic anhydride, hexahydrotrimellitic anhydride, pyromellitic anhydride, nadic anhydride, biphenyltetracarboxylic anhydride, 1,2,3,4-butanetetracarboxylic anhydride, naphthalenetetracarboxylic anhydride, 9,9-fluorenylidene bisphthalic anhydride, and the like; and copolymers obtained by copolymerizing maleic anhydride with another vinyl monomer, such as a styrene-maleic anhydride copolymer, an ethylene-maleic anhydride copolymer, an isobutylene-maleic anhydride copolymer, an alkyl vinyl ether-maleic anhydride copolymer; and the like.

From the viewpoint of the electroconductive properties and the thermoelectric properties, at least one of a lactam or an alcohol is preferably used as an auxiliary agent. The amount of the auxiliary agent, relative to the total mass of the thermoelectric conversion material, is preferably within a range from 0.1 to 50% by mass, more preferably within a range from 1 to 10% by mass, and still more preferably within a range from 1 to 5% by mass. When the amount of the auxiliary agent is at least 0.1% by mass, the effect of improving the electroconductive properties and the thermoelectric properties can be easily obtained. When the amount of the auxiliary agent is not more than 50% by mass, the deterioration of film properties can be suppressed.

(Resin)

The thermoelectric conversion material of this embodiment may contain a resin to the extent that the effects of the electroconductive properties and the thermoelectric properties are not impaired, for the purpose of adjusting film forming properties and film strength.

The resin may any resin that is compatible with or is mixed with and dispersed in the components of the thermoelectric conversion material. Either a thermosetting resin or a thermoplastic resin may be used. Specific examples of usable resins include polyester resins, polyimide resins, polyamide resins, fluororesins, vinyl resins, epoxy resins, xylene resins, aramid resins, polyurethane resins, polyurea resins, melamine resins, phenol resins, polyether resins, acrylic resins, acrylamide resins, and copolymer resins of these resins. Although the resin is not particularly limited, in this embodiment, at least one selected from the group consisting of a polyurethane resin, a polyether resin, an acrylic resin, and an acrylamide resin is preferably used.

(Inorganic Thermoelectric Conversion Material)

The thermoelectric conversion material of this embodiment may optionally contain an inorganic thermoelectric conversion material (or microparticles formed with inorganic thermoelectric conversion material) in order to enhance thermoelectric conversion performance.

Examples of the inorganic thermoelectric conversion materials include Bi-(Te, Se) based materials, Si-Ge based materials, Mg-Si based materials, Pb-Te based materials, GeTe-AgSbTe based materials, (Co, Ir, Ru)-Sb based materials, and (Ca, Sr, Bi) $Co_2O_5$ based materials. More specifically, for example, at least one selected from the group consisting of $Bi_2Te_3$, PbTe, $AgSbTe_2$, GeTe, $Sb_2Te_3$, $NaCo_2O_4$, $CaCoO_3$, $SrTiO_3$, ZnO, SiGe, $Mg_2Si$, $FeSi_2$, $Ba_8Si_{46}$, $MnSi_{1.73}$, ZnSb, $Zn_4Sb_3$, $GeFe_3CoSb_{12}$, and $LaFe_3CoSb_{12}$ can be used. Here, an impurity may be added to the inorganic thermoelectric conversion material as described above to control the polarity (p-type, n-type) and the electrical conductivity, and such a material may be used. When an inorganic thermoelectric conversion material is used, the amount of the inorganic thermoelectric conversion material used is adjusted within a range in which the effects of the film forming properties and the film strength are not impaired.

In the case of manufacturing a dispersion containing a thermoelectric conversion material, for example, the dispersion can be manufactured by mixing the thermoelectric conversion material, a solvent, and one or more other optional components if necessary, and then dispersing the mixture using a disperser or ultrasonic waves.

The disperser is not particularly limited. Examples of the dispersers which can be used include a kneader, an attritor, a ball mill, and a sandmill using, for example, glass beads or zirconia beads, a scandex, an Eiger mill, a paint conditioner, a media disperser such as a paint shaker, and a colloid mill.

<Thermoelectric Conversion Element>

The thermoelectric conversion element of this embodiment is configured using the thermoelectric conversion material described above. In this embodiment, the thermoelectric conversion element has a thermoelectric conversion film formed using the thermoelectric conversion material and an electrode, and the thermoelectric conversion film and the electrode are electrically connected to each other. The thermoelectric conversion film has excellent heat resistance and flexibility in addition to excellent electroconductive properties and thermoelectric properties. Therefore, according to this embodiment, a high-quality thermoelectric conversion element can be easily realized.

The thermoelectric conversion film may be a film obtained by applying a thermoelectric conversion material onto a substrate. Since the thermoelectric conversion material has excellent moldability, a good film can be easily obtained by a coating method. A wet film forming method is mainly used for forming the thermoelectric conversion film. Specific examples of the method include a method using any of various measures, such as a spin coating method, a spray method, a roller coating method, a gravure coating method, a die coating method, a comma coating method, a roll coating method, a curtain coating method, a bar coating method, an ink jet method, a dispenser method, a silk screen printing method, and a flexographic printing method. Depending on the thickness for the coating, the viscosity of the material, and the like, the method may be appropriately selected from the above methods.

The thickness of the thermoelectric conversion film is not particularly limited. However, as described below, it is preferable to form the thermoelectric conversion film so as to have a thickness of a certain value or greater so as to generate and transmit a temperature difference in the thickness direction or the plane direction of the thermoelectric conversion film. In this embodiment, from the viewpoint of thermoelectric properties, the film thickness of the thermoelectric conversion film is preferably from 0.1 to 200 $\mu$m, more preferably from 1 to 100 $\mu$m, and still more preferably from 1 to 50 $\mu$m.

As the substrate to which the thermoelectric conversion material is applied, a nonwoven fabric, paper, a plastic film made of a material such as polyethylene, polyethylene terephthalate (PET), polyethylene naphthalate, polyether sulfone, polypropylene, polyimide, polycarbonate, or cellulose triacetate, glass, or the like may be used.

In order to improve the adhesion between the substrate and the thermoelectric conversion film, any of various treatments can be applied to the substrate surface. Specifically, prior to the application of the thermoelectric conversion material, a UV ozone treatment, a corona treatment, a plasma treatment, or a treatment for easy adhesion may be performed.

The thermoelectric conversion element of this embodiment can be configured applying a technique well-known in the art, with the exception of using the thermoelectric conversion material as described above. Representatively, a more specific configuration of the thermoelectric conversion element and a method of manufacturing the thermoelectric conversion element are described.

In this embodiment, the thermoelectric conversion element has a thermoelectric conversion film obtained using a thermoelectric conversion material and a pair of electrodes electrodically connected to the thermoelectric conversion film. As used herein, the term "to be electrically connected" means that they are joined to each other or are in a state in which they can be energized through another component such as a wire.

The material of the electrode can be selected from metals, alloys, and semiconductors. In this embodiment, the material of the electrode is preferably a metal or an alloy, since the electrical conductivity is high and the contact resistance with the thermoelectric conversion material of this embodiment that is a component of the thermoelectric conversion film is low. As a specific example, the electrode preferably contains at least one selected from the group consisting of gold, silver, copper, and aluminum. The electrode more preferably contains silver.

The electrode can be formed using a method such as a vacuum deposition method, thermocompression bonding of a film having an electrode material foil or an electrode material film, or applying of a paste in which microparticles of the electrode material are dispersed. In view of the simplicity of the process, a method using thermocompression bonding of an electrode material foil or a film having an electrode material film, or using applying of a paste in which an electrode material is dispersed is preferable.

From the positional relationship between the thermoelectric conversion film and a pair of electrodes, specific examples of the structure of the thermoelectric conversion element is roughly divided into (1) a structure in which the electrodes are formed at both ends of the thermoelectric conversion film and (2) a structure in which the thermoelectric conversion film is disposed between the two electrodes.

For example, the thermoelectric conversion element having the structure described in (1) above can be obtained by forming a thermoelectric conversion film on a substrate and then applying a silver paste to both ends of the thermoelectric conversion film to form a first electrode and a second electrode. In the thermoelectric conversion element in which the electrodes are formed at both ends of the thermoelectric conversion film, the distance between the two electrodes can be easily widened. Therefore, it is easy to generate a large temperature difference between the two electrodes to efficiently perform thermoelectric conversion.

The thermoelectric conversion element having the structure described in (2) above can be obtained, for example, by applying a silver paste on a substrate to form a first electrode, forming a thermoelectric conversion film thereon, and applying a silver paste thereon to form a second electrode. In the thermoelectric conversion element in which the thermoelectric conversion film is disposed between the two electrodes, it is difficult to increase the distance between the two electrodes. Therefore, it is difficult to generate a large temperature difference between the two electrodes. However, it is possible to increase the temperature difference by increasing the film thickness of the thermoelectric conversion film. Further, the thermoelectric conversion element having such a structure can utilize the temperature difference in the direction perpendicular to the substrate, and thus can be used in a form in which the thermoelectric conversion element is adhered to the heating element. Accordingly, this thermoelectric conversion element is preferable in that a large area of the heat source can be easily utilized.

The thermoelectric conversion element can generate a high voltage in a case of being connected in series, and can generate a large current in a case of being connected in parallel. The thermoelectric conversion element may be the one in which two or more thermoelectric conversion elements are connected. According to this embodiment, since the thermoelectric conversion element has excellent flexibility, the thermoelectric conversion element can follow and can be satisfactorily installed in a heat source having a shape which is not a plane.

In this embodiment, it is also effective to combine the thermoelectric conversion element with a thermoelectric conversion element configured with another thermoelectric material. Examples of inorganic thermoelectric conversion materials include Bi-(Te, Se) based materials, Si-Ge based materials, Mg-Si based materials, Pb-Te based materials, GeTe-AgSbTe based materials, (Co, Ir, Ru)-Sb based materials, and (Ca, Sr, Bi)$Co_2O_5$ based materials. Specifically, at least one selected from the group consisting of $Bi_2Te_3$, PbTe, $AgSbTe_2$, GeTe, $Sb_2Te_3$, $NaCo_2O_4$, $CaCoO_3$, $SrTiO_3$, ZnO, SiGe, $Mg_2Si$, $FeSi_2$, $Ba_8Si_{46}$, $MnSi_{1.73}$, ZnSb, $Zn_4Sb_3$, $GeFe_3CoSb_{12}$, $LaFe_3CoSb_{12}$ and the like may be used. Here, an impurity may be added to the inorganic thermoelectric conversion material as described above to control the polarity (p-type, n-type) and the electrical conductivity, and such a material may be used. As another material, as an organic thermoelectric material, at least one selected from the group consisting of polythiophene, polyaniline, polyacetylene, fullerene and their derivatives can be used. When combining the thermoelectric conversion element of this embodiment with another thermoelectric conversion element configured with any of these materials, it is preferable to manufacture this another thermoelectric conversion element within a range in which the flexibility of the element is not impaired.

When a plurality of thermoelectric conversion elements are connected, they may be connected in a state where they are integrated on a single substrate, and such a product may be used. In such an embodiment, it is preferable to combine a thermoelectric conversion element of the present embodiment and a thermoelectric conversion element formed using a thermoelectric material exhibiting the polarity as an n-type is preferable, and it is more preferable to connect these elements in series. According to the present embodiment, the thermoelectric conversion elements may be densely integrated more easily.

(Second Embodiment)

The thermoelectric conversion material of the present embodiment satisfies all of the following (1) to (3).

(1) 0 < ((HOMO of the organic compound (B)) - (HOMO of the electrically conductive material (A))) × ((HOMO of the organic compound (C)) - (HOMO of the electrically conductive material (A)))
(2) | (HOMO of the organic compound (B)) - (HOMO of the electrically conductive material (A)) | < | (HOMO of the organic compound (C)) - (HOMO of the electrically conductive material (A)) |
(3) The adsorptivity of the organic compound (B) to the electrically conductive material (A) is greater than the adsorptivity of the organic compound (C) to the electrically conductive material (A).

Using such a specific combination, compatibility between a high Seebeck coefficient and high electroconductive properties may be achieved, and excellent thermoelectric performance may be exhibited. This is because holes (careers) efficiently move from the organic compound having a small thermal excitation energy to the electrically conductive material, and these holes move within the electrically conductive material, thereby achieving a high Seebeck coefficient and a high electrical conductivity.

<Electrically Conductive Material (A)>

With respect to the electrically conductive material (A), the description for the first embodiment can be employed.

<Organic Compound>

Each of the organic compound (B) and the organic compound (C) is determined by a difference in physical properties relative to each other and a difference in physical property relative to the electrically conductive material (A), not by the intrinsic physical properties. Specifically, the requirements (1) to (3) as described above are satisfied. Those satisfying the requirements of (1) and (2) can be further classified into (4) and (5) below.

(4) The HOMO of the electrically conductive material (A) is greater than the HOMO of the organic compound (B), and the HOMO of the organic compound (B) is greater than the HOMO of the organic compound (C).
(5) The HOMO of the electrically conductive material (A) is smaller than the HOMO of the organic compound (B), and the HOMO of the organic compound (B) is smaller than the HOMO of the organic compound (C).

The mechanism of thermoelectric conversion is suggested as follows.

When the adsorptivity to the conductive material (A) is greater in the organic compound (B) than in the organic compound (C), the organic compound (B) is preferentially adsorbed on the surface of the electrically conductive material (A) than the organic compound (C). For the generation of thermoelectric conversion, the carrier movement among the electrically conductive material (A), the organic compound (B), and the organic compound (C) is required to occur. Here, if the HOMO of the organic compound (B) is closer to the HOMO of the electrically conductive material (A) than the HOMO of the organic compound (C), then the carrier movement between the electrically conductive material (A) and the organic compound (B) existing in the vicinity of the surface becomes smooth, and the carrier movement between the organic compound (B) and the organic compound (C) also becomes smooth, resulting in efficient carrier movement among the electrically conductive material (A), the organic compound (B), and the organic compound (C), thus enhancing the thermoelectric conversion efficiency.

The HOMO of the organic compound (B) and the HOMO of the electrically conductive material (A) are preferably from 0.1 to 2.0 eV apart, and more preferably from 0.1 to 1.5 eV apart. The HOMO of the organic compound (C) and the HOMO of the organic compound (B) are preferably from 0.1 to 2.0 eV apart, and more preferably from 0.1 to 1.5 eV apart. For example, when the HOMO of the electrically conductive material (A) is -5.1 eV, the HOMO of the organic compound (B) is preferably from -7.1 to -5.2 eV or from -5.0 to -3.0 eV. When the HOMO of the electrically conductive material (A) is -5.1 eV and the HOMO of the organic compound (B) is -5.4 eV, the HOMO of the organic compound (C) is preferably from -7.5 to -5.5 eV. When the HOMO of the electrically conductive material (A) is -5.1 eV and the HOMO of the organic compound (B) is -5.0 eV, the HOMO of the organic compound (C) is preferably from -4.9 to -2.9 eV.

In order to promote surface adsorption to the electrically conductive material (A) and uniformity and to further increase the molecular ratio, the molecular weight of the organic compound (B) is preferably small, and the molecular weight or the mass-average molecular weight (Mw) is preferably not more than 5,000 or less, and more preferably 3,000 or less. Each of the organic compound (B) and the organic compound (C) is preferably an organic semiconductor.

The organic compound (B) and the organic compound (C) satisfying the condition as described above are preferably a compound having any one of a perylene skeleton, a pyrrolopyrrole skeleton, a phenothiazine skeleton, a thiazolothiazole skeleton, an oxazolothiazole skeleton, an oxazolooxazole skeleton, a benzobisthiazole skeleton, a benzobisoxazole skeleton, a thiazolobenzoxazole skeleton, a thioxanthone skeleton, a fluorene skeleton, an oxazole skeleton, and a phthalocyanine skeleton. With respect to examples of preferable embodiments, when the electrically conductive material (A) is a carbon nanotube, the organic compound (B) is preferably a compound having a pyrrolopyrrole

skeleton or a phenothiazine skeleton, and the organic compound (C) is preferably a compound having a thioxanthone skeleton, a fluorene skeleton or an oxazole skeleton.

The organic compound (B) and the organic compound (C) contribute to the improvement of the Seebeck coefficient in the thermoelectric conversion material. In a case in which the total amount of the organic compound (B) and the organic compound (C) increase, although the Seebeck coefficient can be improved, the electroconductive properties decrease. Accordingly, from the viewpoint of the compatibility between the Seebeck coefficient and the electrical conductivity, the total amount of the organic compound (B) and the organic compound (C), relative to the total mass of the electrically conductive material (A), is preferably not more than 300% by mass, and more preferably 200 mass% or less. The lower limit value is preferably at least 10% by mass, and more preferably 20% by mass or greater.

(Solvent)

The solvent used in this embodiment is used as a medium for mixing the electrically conductive material (A), the organic compound (B), and the organic compound (C), and the coating property can be improved by forming an ink. The solvent which can be used is not particularly limited as long as the electrically conductive material (A), the organic compound (B) and the organic compound (C) can be dissolved or dispersed well, and example thereof include organic solvents and water. Two or more solvents may be used in combination. With respect to the solvent, the description for the first embodiment can be employed.

(Inorganic Thermoelectric Conversion Material)

With respect to the inorganic thermoelectric conversion material in this embodiment, the description for the first embodiment can be employed.

<Thermoelectric Conversion Element>

The thermoelectric conversion element of this embodiment has a thermoelectric conversion film formed using the thermoelectric conversion material as described above, and an electrode, and the thermoelectric conversion film and the electrode are electrically connected to each other. With respect to the thermoelectric conversion element, the description for the first embodiment can be employed.

The thickness of the thermoelectric conversion film is not particularly limited. However, as described below, it is preferable to form the thermoelectric conversion film so as to have a thickness of a predetermined value or greater so as to generate and transmit a temperature difference in the thickness direction or the surface direction of the thermoelectric conversion film. From the viewpoint of thermoelectric properties, the film thickness of the thermoelectric conversion film is preferably from 0.1 to 500 μm, more preferably from 1 to 300 μm, and still more preferably from 1 to 200 μm.

EXAMPLES

The present invention is described below in further detail using experimental examples. In the following description, "part(s)" represents "part(s) by mass", and "%" represents "% by mass". NMP represents N-methylpyrrolidone.

(First Experimental Example)

<Method for Measuring Mass-Average Molecular Weight (Mw)>

For the measurement of mass-average molecular weight (Mw), a GPC (gel permeation chromatography) "HPC-8020" manufactured by Tosoh Co., Ltd. was used. The GPC is a liquid chromatography to separate and quantify a substance dissolved in a solvent (THF; tetrahydrofuran) by the difference of its molecular size. For the measurement in this experimental example, as the column, two "LF-604" (GPC column for rapid analysis: 6 mm ID × 150 mm size, manufactured by Showa Denko K.K.) were connected in series, and the measurement was performed under the conditions of a flow rate of 0.6 ml/min and a column temperature of 40°C. The mass-average molecular weight (Mw) was determined in terms of polystyrene.

<Method for Measuring HOMO Value and Fermi Level>

The HOMO levels of the electrically conductive material (A) and the organic compound (B) (or, if the electrically conductive material is metal, the Fermi level) were measured by photoelectron spectroscopy (AC-2, manufactured by

Riken Keiki Co., Ltd.) after fixing each single component on an electrically conductive tape adhered onto an ITO glass substrate to obtain a measurement sample. The measured values and the values ΔHOMO of | (HOMO of the organic compound (B)) - (HOMO of the electrically conductive material (A)) | calculated from the measured values are shown in Table 55, Table 56, and Table 57.

<Synthesis of Organic Compound (B)>

(Synthesis Example 1: Organic Compound (B4))

In 20 ml of nitrobenzene, 5.0 g of 3-aminoperylene, 15.2 g of 3-bromo-9-phenylcarbazole, 1.5 g of sodium hydroxide and 1.0 g of copper oxide were added, and the resulting mixture was heated and stirred at 200°C for 50 hours under a nitrogen atmosphere. After cooling, the mixture was diluted with 500 ml of water and the resulting mixture was subjected to extraction with toluene. After concentrating the extract liquid, purification using column chromatography with silica gel was performed. Thus, 7.3 g of an organic compound (B4) was obtained.

Organic Compound (B4)

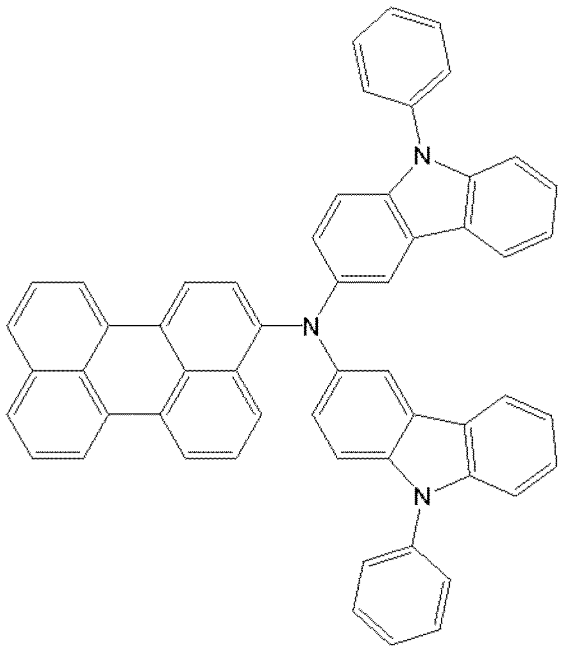

(Synthesis Example 2: Organic Compound (B30))

In 20 ml of nitrobenzene, 5.0 g of 3-aminoperylene, 12.3 g of 4-bromobiphenyl, 1.5 g of sodium hydroxide and 1.0 g of copper oxide were added, and the resulting mixture was heated and stirred at 200°C for 50 hours under a nitrogen atmosphere. After cooling, the mixture was diluted with 500 ml of water and the resulting mixture was subjected to extraction with toluene. After concentrating the extract liquid, purification was performed using column chromatography with silica gel. Thus, 5.6 g of an organic compound (B30) was obtained.

Organic Compound (B30)

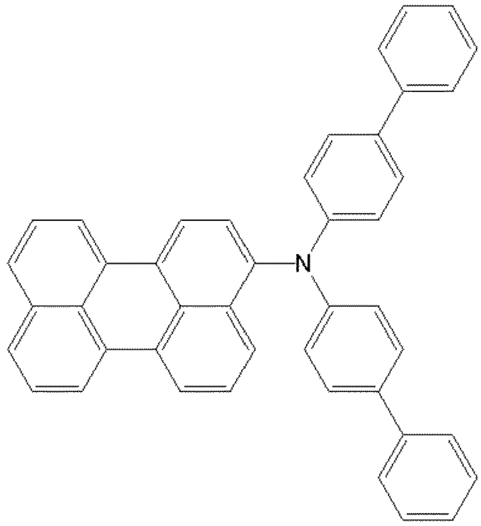

(Synthesis Example 3: Organic Compound (B128))

In 300 g of amyl alcohol, 18.5 g of dimethyl succinate, 30 g of 2-cyanothiophene, and 13.5 g of sodium hydride were dissolved, and the resulting mixture was refluxed for 8 hours. After cooling, the precipitate was filtered and washed with acetic acid and methanol to give 17.67 g of a red-brown solid. Thereafter, 10 g of the thus obtained solid, 26.1 g of 1-iodo-2-methylpropane and 10.3 g of sodium tert-butoxide were dissolved in 300 g of dimethylacetamide, and the obtained mixture was refluxed for 8 hours. After cooling, the mixture was added to 1000 ml of methanol, a solid was precipitated, collected by filtration, and purified by column chromatography with silica gel. Thus, 9.58 g of an organic compound (B128) was obtained.

Organic Compound (B128)

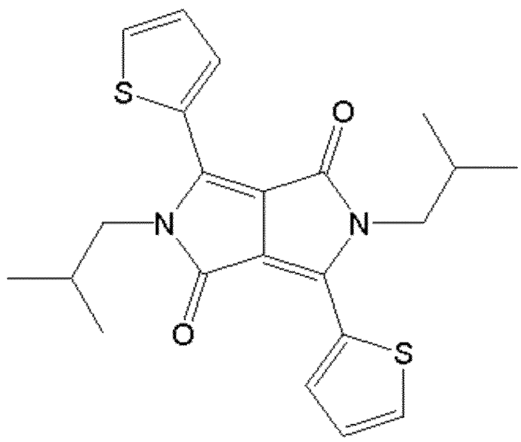

(Synthesis Example 4: Organic Compound (B166))

In 300 g of amyl alcohol, 18.5 g of dimethyl succinate, 28.5 g of 2-cyanofuran, and 13.5 g of sodium hydride were dissolved, and the resulting mixture was refluxed for 8 hours. After cooling, the precipitate was filtered and washed with acetic acid and methanol. Thus, 15.67 g of an organic compound (B166) was obtained.

Organic Compound (B166)

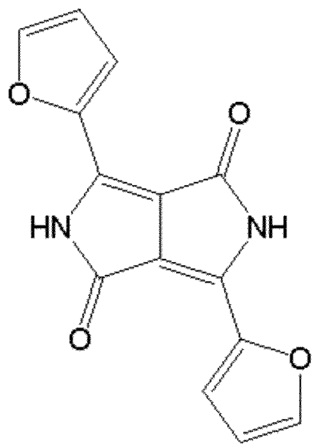

(Synthesis Example 5: Organic Compound (B187))

In 150 g of tert-pentyl alcohol, 20.2g of diisopropyl succinate, 59.6 g of di(p-tolyl)aminobenzonitrile, and 22.4 g of potassium tert-butoxide were dissolved, and the resulting mixture was refluxed for 8 hours. After cooling, the precipitate was filtered and washed with acetic acid and methanol, and 36.8 g of a red solid was obtained. After suspending 34 g of the thus obtained red solid in 340 g of nitrobenzene, 75 g of ethyl p-toluenesulfonate and 41.4 g of potassium carbonate were added. The temperature was raised to 200°C by heating, and stirring was performed at this temperature under a nitrogen atmosphere for 3 hours. The mixture was then cooled to room temperature, and the precipitate was filtered and washed with methanol. The obtained product was then suspended in 1700 g of water, and the resulting mixture was stirred at a temperature of 80 to 90°C for 30 minutes, followed by filtering, washing with water and drying. Thus, 27.6 g of an organic compound (B187) was obtained.

Organic Compound (B187)

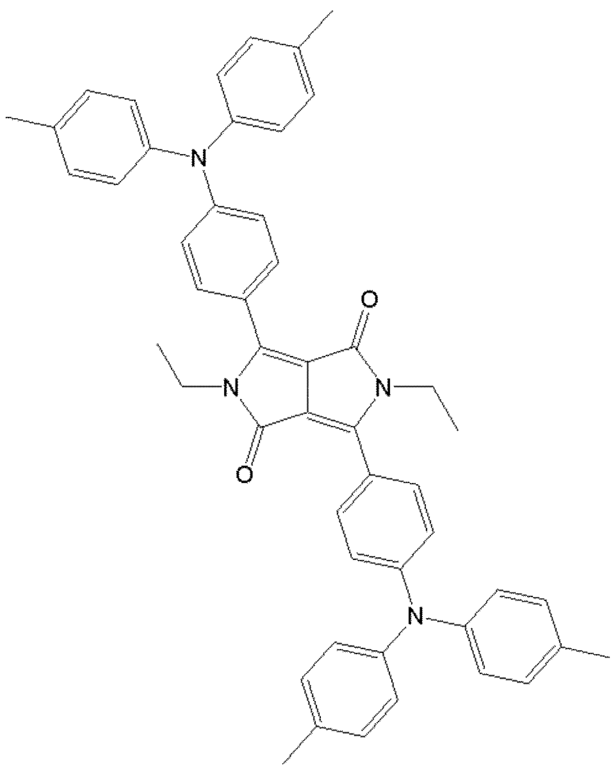

(Synthesis Example 6: Organic Compound (B192))

In 200 g of amyl alcohol, 18.4 g of dimethyl succinate, 53.1 g of 4-trifluoromethylbenzonitrile and 25.3 g of sodium butoxide were dissolved, and the resulting mixture was refluxed for 8 hours. After cooling, the precipitate was filtered and washed with acetic acid and methanol. Thus, 21.2 g of an organic compound (B192) was obtained.

Organic Compound (B192)

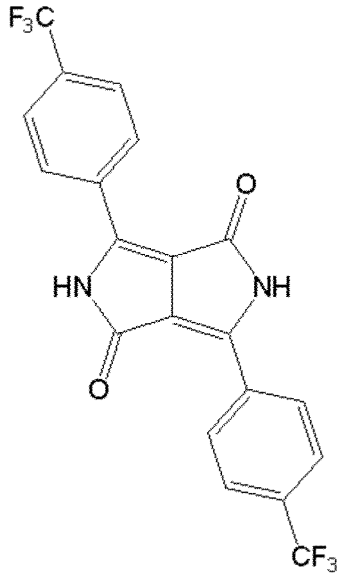

(Synthesis Example 7: Organic Compound (B0))

In 190 g of dehydrated pyridine, 21.00 g of 2-(2-aminophenyl)benzimidazole (a reagent manufactured by Aldrich) was dissolved at room temperature. To this solution, 19.24 g of 2,4,6-trimethylbenzoyl chloride was added in several batches, and the resulting mixture was stirred for 2 hours while the temperature is kept at room temperature. The obtained solution was poured into 1000 g of ice water, and after adjusting the pH to 3 by adding hydrochloric acid, the precipitate was filtered out, washed with water, and then dried under reduced pressure at 100°C. To the thus obtained solid, 400 ml of acetonitrile was added, and the resulting mixture was subjected to reslurrying at a boiling point for one hour. The solid in the resulting slurry was filtered out, washed with cold acetonitrile, and dried at 100°C under reduced pressure. Thus, 21.59 g of an organic compound (B0) was obtained.

Organic Compound (B0)

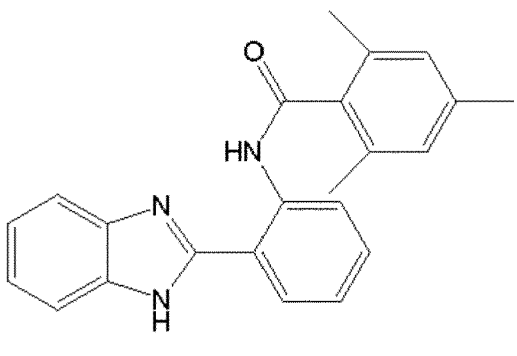

(Synthesis Example 10: Organic Compound (B248))

In 200 g of amyl alcohol, 10.0 g of dimethyl succinate, 28.1 g of 1-naphthonitrile and 15.8 g of sodium butoxide were dissolved, and the resulting mixture was refluxed for 8 hours. After cooling, the precipitate was filtered and washed with acetic acid and methanol. Thus, 14.5 g of an organic compound (B248) was obtained.

Organic Compound (B248)

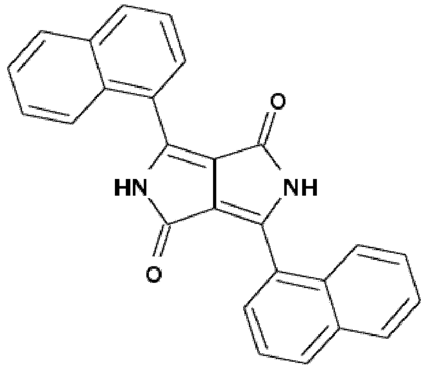

(Synthesis Example 11: Organic Compound (B242))

In 200 g of amyl alcohol, 10.0 g of dimethyl succinate, 17.1 g of 2-cyanopyridine, and 15.8 g of sodium butoxide were dissolved, and the resulting mixture was refluxed for 8 hours. After cooling, the precipitate was filtered and washed with acetic acid and methanol. Thus, 21.2 g of an organic compound (B242) was obtained.

Organic Compound (B242)

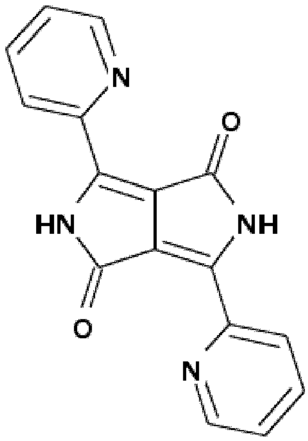

(Synthesis Example 12: Organic Compound (B254))

In 400 g of amyl alcohol, 20.0 g of dimethyl succinate, 48.0 g of 4-(dimethylamino)benzonitrile and 31.6 g of sodium hydride were dissolved, and the resulting mixture was refluxed for 8 hours. After cooling, the precipitate was filtered and washed with acetic acid and methanol, and, as a result, 13.9 g of a purple solid was obtained. Thereafter, 10 g of the thus obtained solid, 17.2 g of iodoethane and 10.3 g of sodium butoxide were dissolved in 300 g of dimethylacetamide, and the resulting mixture was refluxed for 8 hours. After cooling, the mixture was added to 1000 ml of methanol, a solid was precipitated, collected by filtration, and purified by column chromatography with silica gel. Thus, 9.46 g of an organic compound (B254) was obtained.

Organic Compound (B254)

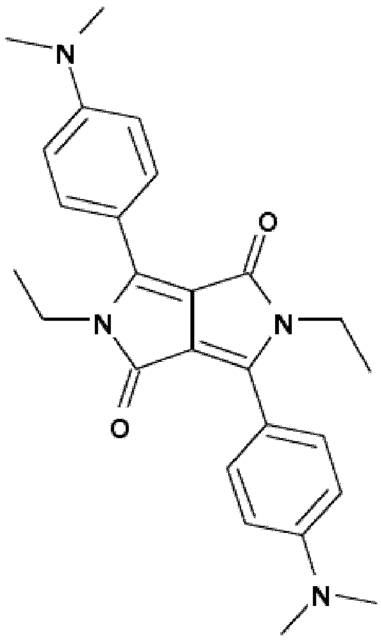

(Synthesis Example 13: Organic Compound (B250))

In 200 g of amyl alcohol, 10.0 g of dimethyl succinate, 29.9 g of 4-bromobenzonitrile and 15.8 g of sodium hydride were dissolved, and the resulting mixture was refluxed for 8 hours. After cooling, the precipitate was filtered and washed with acetic acid and methanol, and, as a result, 16.1 g of a red solid was obtained. Thereafter, 10 g of the thus obtained solid, 29.1 g of 1-thianthrenylboronic acid, and 0.65 g of tetrakis(triphenylphosphine)palladium(0) were dissolved in 100 g of xylene. The container was flushed with nitrogen gas, and the mixture was refluxed for 6 hours. After cooling, a solvent was volatilized from the reaction liquid, and purification using column chromatography with silica gel was performed. Thus, 4.53 g of an organic compound (B250) was obtained.

Organic Compound (B250)

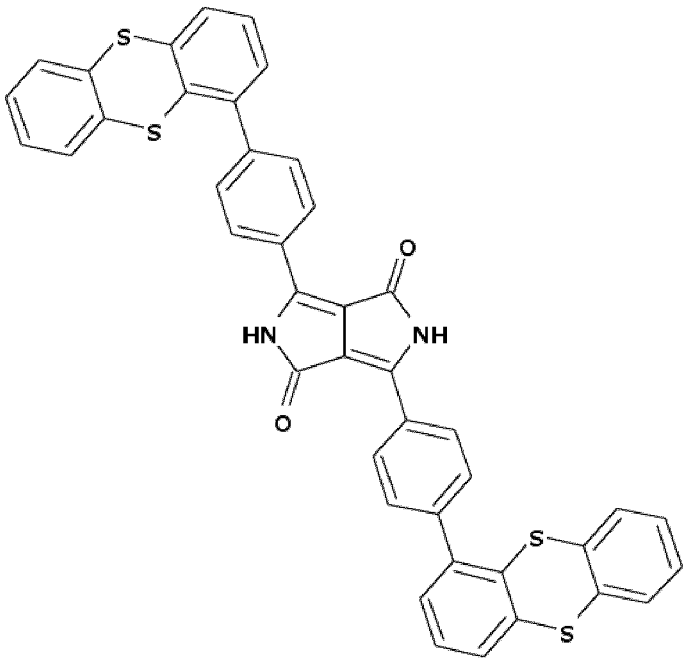

(Synthesis Example 14: Organic Compound (B257))

First, 10.0 g of rubeanic acid, 24.7 g of benzaldehyde and 100 g of phenol were mixed, and the resulting mixture was heated at 160°C for 6 hours. After cooling, the reaction liquid was added to 1000 g of methanol, the precipitated matter was collected by filtration, and washed with methanol. Thus, 17.4 g of an organic compound (B257) was obtained.

Organic compound (B257)

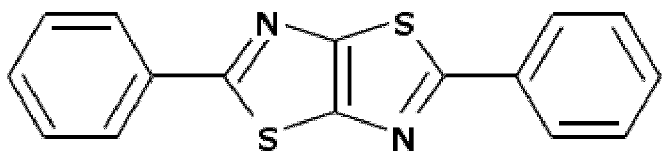

(Synthesis Example 15: Organic Compound (B277))

First, 10.0 g of rubeanic acid, 52.0 g of 3-formyl-N-ethylcarbazole and 100 g of phenol were mixed, and the resulting mixture was heated at 160°C for 6 hours. After cooling, the reaction liquid was added to 1000 g of methanol, the precipitated matter was collected by filtration, and washed with methanol. Thus, 22.5 g of an organic compound (B277) was obtained.

Organic Compound (B277)

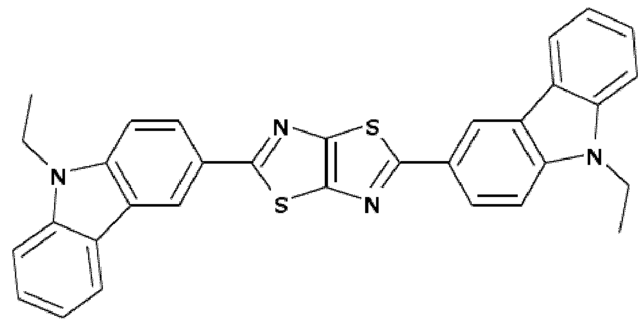

(Synthesis Example 16: Organic Compound (B351))

To a flask, 10.0 g of 2,5-diamino-1,4-benzenedithiol dihydrochloride, 40.1 g of 4-butylbenzoyl chloride, 50 g of tetrahydrofuran, and 50 g of N-methyl-2-pyrrolidone were added, and the resulting mixture was refluxed for 48 hours. After cooling, an aqueous sodium hydroxide solution was added to neutralize the reaction system, and the precipitated solid was collected by filtration. Thus, 8.2 g of an organic compound (B351) was obtained.

Organic Compound (B351)

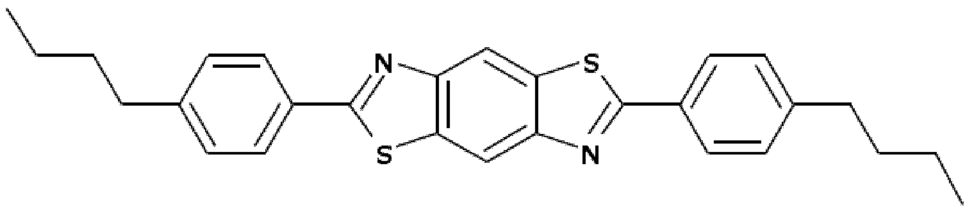

(Synthesis Example 17: Organic Compound (B339))

To a flask, 10.0 g of 2,5-diaminohydroquinone dihydrochloride, 34.4 g of thiophene-2-carbonyl chloride, 33.3 g of polyphosphoric acid, and 100 g of tetrahydrofuran were added, and the resulting mixture was refluxed for 25 hours.

After cooling, an aqueous sodium hydroxide solution was added to neutralize the reaction system, and the precipitated solid was collected by filtration. Thus, 8.0 g of an organic compound (B339) was obtained.

Organic Compound (B339)

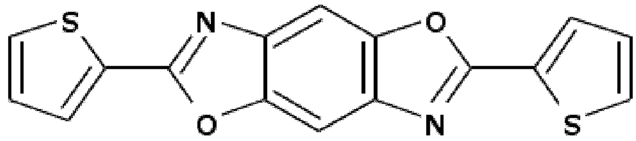

<Synthesis of Polymer with Organic Coloring Mater Introduced into Side Chain>

(Synthesis Example 8: Coloring Matter-Introduced Polymer 1)

Referring to paragraphs 0074 and 0075 of WO 2015/050113, a coloring matter-introduced polymer 1 was prepared which is an acrylic polymer having a mass-average molecular weight (Mw) of about 21,000 and having a perylene skeleton introduced into two kinds of side chains represented by the following structure.

Coloring Matter-Introduced Polymer 1

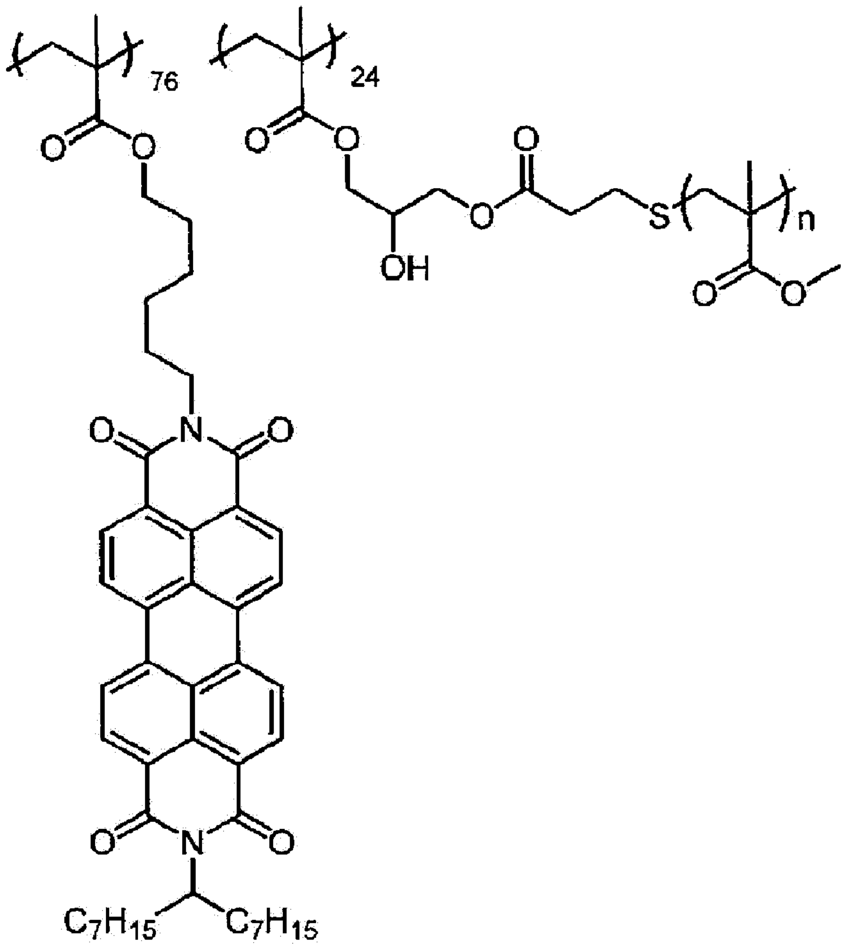

<Synthesis of Resin Component>

(Synthesis Example 9: Binder Resin 1)

First, 455.5 parts of polyester polyol ("Kuraray Polyol P-2011" manufactured by Kuraray Co., Ltd., Mn = 2,011) obtained using terephthalic acid, adipic acid and 3-methyl-1,5-pentanediol, 16.5 parts of dimethylol butanoic acid, 105.2

parts of isophorone diisocyanate, and 140 parts of toluene were charged into a reaction vessel equipped with a stirrer, a thermometer, a reflux condenser, a dropping device, and a nitrogen introduction tube, and were allowed to react in a nitrogen atmosphere at 90°C for 3 hours. To the resulting mixture, 360 parts of toluene was added to prepare a solution of urethane prepolymer having an isocyanate group. Next, 969.5 parts of the obtained solution of the urethane prepolymer having an isocyanate group was added to a mixture of 19.9 parts of isophoronediamine, 0.63 parts of di-n-butylamine, 294.5 parts of 2-propanol, and 335.5 parts of toluene, and the resulting mixture was then allowed to react at 50°C for 3 hours, and thereafter, at 70°C for 2 hours. Thereafter, vacuum drying was performed at 100°C. Thus, a binder resin 1 which was a urethane urea resin having a mass-average molecular weight (Mw) of 61,000 was obtained.

<Production of Thermoelectric Conversion Material>

[Example 1]

(Dispersion 1)

First, 0.4 parts of graphene nanoplatelets "xGNP-M-5" manufactured by XG Sciences, 0.4 parts of the organic compound (B4), and 79.2 parts of NMP each were weighed, and they were mixed together. Further, 140 parts of zirconia beads (φ 1.25 mm) were added thereto, and the mixture was shaken with a scandex for 2 hours, and subjected to filtration to remove the zirconia beads. Thus, a dispersion 1 serving as a thermoelectric conversion material was obtained.

[Examples 2 to 38 and 77 to 85]

(Dispersions 2 to 38 and 77 to 85)

With the exception of changing the components and the amounts to those shown in Table 55 and Table 56, the dispersions 2 to 38 and 77 to 85 containing a thermoelectric conversion material were prepared in the same manner as dispersion 1.

[Examples 95 to 108]

(Dispersions 200 to 213)

With the exception of changing the components and the amounts to those shown in Table 57, dispersions 200 to 213 containing a thermoelectric conversion material were prepared in the same manner as in the dispersion 1.

[Comparative Example 1]

(Dispersion 101)

With the exception of changing the organic compound (B4) of the dispersion 1 to the coloring matter-introduced polymer 1, a dispersion 101 containing the coloring matter-introduced polymer 1 was prepared in the same manner as the dispersion 14.

[Comparative Example 2]

(Dispersion 102)

First, 0.8 parts of the organic compound (B30) and 79.2 parts of NMP each were weighed, and they were mixed together. Further, 140 parts of zirconia beads (φ 1.25 mm) were added thereto, and the mixture was shaken with a scandex for 2 hours, and subjected to filtration to remove the zirconia beads. Thus, a dispersion 102 containing no electrically conductive material was obtained.

<Evaluation of Thermoelectric Conversion Materials>

The obtained dispersions 1 to 38, 77 to 85, 101, and 200 to 213 were applied to a PET film having a thickness of 75 μm that is a sheet-like substrate, using an applicator, followed by heating and drying at 120°C for 30 minutes, whereby a layered product having a thermoelectric conversion film with a film thickness of 5 μm on the PET substrate was obtained. Furthermore, about 2 g of the dispersion 102 was dropped onto a 10 cm square glass substrate, and spin

coating (2000 rpm, 10 seconds) was performed, followed by heating and drying at 120°C for 30 minutes, whereby a layered product having a thermoelectric conversion film with a film thickness of 452 nm on the glass substrate was obtained.

The obtained layered products having a thermoelectric conversion film (hereinafter may also be referred to as a coating film) were evaluated for the electroconductive properties, the Seebeck coefficient, and the power factor (PF) as follows. The results are shown in Tables 55, 56, and 57.

(Electrical Conductivity)

The obtained layered product was cut to 2.5 cm × 5 cm, and according to JIS-K 7194, the electrical conductivity was measured by the 4-terminal method using Loresta GX MCP-T700 (manufactured by Mitsubishi Chemical Analytech Co., Ltd.). The layered product using the glass substrate was cut using a glass cutter.

(Seebeck Coefficient)

The obtained layered product was cut into 3 mm × 10 mm, and using ZEM-3LW manufactured by Advance Riko, Inc., the Seebeck coefficient (μW/K) at 80°C was measured.

(Power Factor (PF))

Using the obtained electrical conductivity and the obtained Seebeck coefficient, PF (= $S^2 \cdot \sigma$) at 80°C was calculated and evaluated according to the following criteria. If the PF is at least 2.5 $\mu W/(mK^2)$, it is at a practically usable level.

A: the PF is at least 20 $\mu W/(mK^2)$ (very good)
B: the PF is at least 10 $\mu W/(mK^2)$ but lower than 20 $\mu W/(mK^2)$ (good)
C: the PF is at least 2.5 $\mu W/(mK^2)$ but lower than 10 $\mu W/(mK^2)$ (practically usable)
D: the PF is lower than 2.5 $\mu W/(mK^2)$ (practically unusable)

[Table 55]

| Table 55 | Thermoelectric Conversion Material Dispersion | Conductive Material (A) | | | Organic Compound (B) | | | NMP | Other Components | | ⊿HOMO | electrical conductivity | Seebeck Coefficient | PowerFactor PF | |
|---|---|---|---|---|---|---|---|---|---|---|---|---|---|---|---|
| | | Type | Amonunt (part) | HOMO value (eV) | Type | Amonuntt (part) | HOMO value (eV) | Amount (part) | Type | Amount (part) | eV | S/cm | μW/K | μW/(mK$^2$) | Evaluation |
| Exemple 1 | Dispersion 1 | GNP | 0.4 | -5.07 | B4 | 0.4 | -4.95 | 100% | - | 0 | 0.12 | 24 | 55.0 | 7.26 | C |
| Exemple 2 | Dispersion 2 | GNP | 0.4 | -5.07 | B30 | 0.4 | -5.49 | 100% | - | 0 | 0.42 | 18 | 64.0 | 7.37 | C |
| Exemple 3 | Dispersion 3 | GNP | 0.4 | -5.07 | B128 | 0.4 | -5.41 | 100% | - | 0 | 0.34 | 26 | 51.0 | 6.76 | C |
| Exemple 4 | Dispersion 4 | KB | 0.4 | -4.93 | B4 | 0.4 | -4.95 | 100% | - | 0 | | 24 | 59.0 | 8.35 | C |
| Exemple 5 | Dispersion 5 | KB | 0.4 | -4.93 | B30 | 0.4 | -5.49 | 100% | - | 0 | 0.35 | 19 | 62.4 | 7.40 | C |
| Exemple 6 | Dispersion 6 | KB | 0.4 | -4.93 | B166 | 0.4 | -5.28 | 100% | - | 0 | 0.24 | 18 | 63.1 | 7.17 | C |
| Exemple 7 | Dispersion 7 | Graphite | 0.4 | -5.19 | B4 | 0.4 | -4.95 | 100% | - | 0 | 0.30 | 22 | 58.8 | 7.55 | C |
| Exemple 8 | Dispersion 8 | Graphite | 0.4 | -5.19 | B30 | 0.4 | -5.49 | 100% | - | 0 | 0.22 | 22 | 62.8 | 8.68 | C |
| Exemple 9 | Dispersion 9 | Graphite | 0.4 | -5.19 | B128 | 0.4 | -5.41 | 100% | - | 0 | 0.12 | 20 | 49.6 | 4.92 | C |
| Exemple 10 | Dispersion 10 | MWCNT | 0.4 | -5.07 | B4 | 0.4 | -5.28 | 100% | - | 0 | 0.42 | 31 | 56.0 | 9.72 | C |
| Exemple 11 | Dispersion 11 | MWCNT | 0.4 | -5.07 | B30 | 0.4 | -4.95 | 100% | - | 0 | 0.34 | 34 | 61.5 | 12.86 | B |
| Exemple 12 | Dispersion 12 | MWCNT | 0.4 | -5.07 | B128 | 0.4 | -5.49 | 100% | - | 0 | 0.21 | 45 | 51.0 | 11.70 | B |
| Exemple 13 | Dispersion 13 | MWCNT | 0.4 | -5.07 | B166 | 0.4 | -5.41 | 100% | - | 0 | 0.15 | 42 | 62.4 | 16.35 | B |
| Exemple 14 | Dispersion 14 | SWCNT | 0.4 | -5.1 | B4 | 0.4 | -4.95 | 100% | - | 0 | 0.39 | 43 | 61.0 | 16.00 | B |
| Exemple 15 | Dispersion 15 | SWCNT | 0.4 | -5.1 | B30 | 0.4 | -5.49 | 100% | - | 0 | 0.31 | 44 | 65.3 | 18.55 | B |
| Exemple 16 | Dispersion 16 | SWCNT | 0.4 | -5.1 | B128 | 0.4 | -5.41 | 100% | - | 0 | 0.18 | 70 | 51.7 | 18.71 | B |
| Exemple 17 | Dispersion 17 | SWCNT | 0.4 | -5.1 | B166 | 0.4 | -5.28 | 100% | - | 0 | 0.21 | 55 | 65.3 | 23.58 | A |
| Exemple 18 | Dispersion 18 | SWCNT | 0.4 | -5.1 | B187 | 0.4 | -5.31 | 100% | - | 0 | 1.64 | 56 | 64.0 | 22.94 | A |
| Exemple 19 | Dispersion 19 | SWCNT | 0.4 | -5.1 | B192 | 0.4 | -6.74 | 100% | - | 0 | 1.29 | 85 | 48.0 | 19.58 | B |
| Exemple 20 | Dispersion 20 | SWCNT | 0.4 | -5.1 | B0 | 0.4 | -6.39 | 100% | - | 0 | 0.39 | 31 | 54.1 | 8.97 | C |
| Exemple 21 | Dispersion 21 | SWCNT | 0.4 | -5.1 | B30 | 0.02 | -5-49 | 5% | - | 0 | 0.39 | 90 | 35.0 | 11.03 | B |
| Exemple 22 | Dispersion 22 | SWCNT | 0.4 | -5.1 | B30 | 0.08 | -5.49 | 20% | - | 0 | 0.39 | 55 | 60.0 | 19.80 | B |

(continued)

| Table 55 | Thermoelectric Conversion Material Dispersion | Conductive Material (A) | | | Organic Compound (B) | | | NMP | Other Components | | ⊿HOMO | electrical conductivity | Seebeck Coefficient | PowerFactor PF | |
|---|---|---|---|---|---|---|---|---|---|---|---|---|---|---|---|
| | | Type | Amonunt (part) | HOMO value (eV) | Type | Amonuntt (part) | HOMO value (eV) | Amount (part) | Type | Amount (part) | eV | S/cm | μW/K | μW/ ($mK^2$) | Evaluation |
| Exemple 23 | Dispersion 23 | SWCNT | 0.4 | -5.1 | B30 | 0.8 | -5.49 | 200% | - | 0 | 0.39 | 25 | 71.0 | 12.60 | B |
| Exemple 24 | Dispersion 24 | SWCNT | 0.4 | -5.1 | B166 | 1.6 | -5.49 | 400% | - | 0 | 0.18 | 11 | 95.4 | 10.01 | B |
| Exemple 25 | Dispersion 25 | SWCNT | 0.4 | -5.1 | B166 | 0.02 | -5.28 | 5% | - | 0 | 0.18 | 122 | 34.0 | 14.10 | B |
| Exemple 26 | Dispersion 26 | SWCNT | 0.4 | -5.1 | B166 | 0.06 | -5.28 | 20% | - | 0 | 0.18 | 73 | 52.0 | 19.85 | B |
| Exemple 27 | Dispersion 27 | SWCNT | 0.4 | -5.1 | B166 | 0.8 | -5.28 | 200% | - | 0 | 0.18 | 22 | 75.0 | 12.38 | B |
| Exemple 28 | Dispersion 28 | SWCNT | 0.4 | -5.1 | B30 | 1.6 | -5.28 | 400% | - | 0 | 0.18 | 10 | 93.2 | 8.69 | C |
| Exemple 29 | Dispersion 29 | SWCNT | 0.4 | -5.1 | B166 | 0.4 | -5.49 | 100% | Binder Resin 1 | 0.05 | 0.39 | 25 | 55.0 | 7.56 | C |
| Exemple 30 | Dispersion 30 | SWCNT | 0.4 | -5.1 | B4 | 0.4 | -5.28 | 100% | Binder Resin 1 | 0.05 | 0.18 | 27 | 61.0 | 10.05 | B |
| Exemple 31 | Dispersion 31 | PEDOT/PSS | 0.4 | -5.28 | B30 | 0.4 | -5.49 | 100% | - | 0 | 0.21 | 45 | 54.0 | 13.12 | B |
| Exemple 32 | Dispersion 32 | PEDOT/PSS | 0.4 | -5.28 | B166 | 0.4 | -5.28 | 100% | - | 0 | 0.00 | 48 | 42.0 | 8.47 | C |
| Exemple 33 | Dispersion 33 | Ag powder | 0.4 | -4.64 | B4 | 0.4 | -495 | 20% | Binder Resin 1 | 0.05 | 0.31 | 200 | 25.0 | 12.50 | B |
| Exemple 34 | Dispersion 34 | Ag powder | 0.4 | -4.64 | B30 | 0.08 | -5.49 | 20% | Binder Resin 1 | 0.05 | 0.85 | 234 | 24.0 | 13.48 | B |
| Exemple 35 | Dispersion 35 | Ag powder | 0.4 | -4.64 | B166 | 0.08 | -5.28 | 20% | Binder Resin 1 | 0.05 | 0.64 | 216 | 26.0 | 14.60 | B |
| Exemple 36 | Dispersion 36 | Cu powder | 0.4 | -4.59 | B4 | 0.08 | -495 | 20% | Binder Resin 1 | 0.05 | .036 | 180 | 23.0 | 9.52 | C |
| Exemple 37 | Dispersion 37 | Cu powder | 0.4 | -4.59 | B30 | 0.08 | -5.49 | 20% | Binder Resin 1 | 0.05 | 0.90 | 165 | 24.0 | 9.50 | C |

(continued)

| Table 55 | Thermoelectric Conversion Material Dispersion | Conductive Material (A) | | | Organic Compound (B) | | | NMP | Other Components | | ⊿HOMO | electrical conductivity | Seebeck Coefficient | PowerFactor PF | |
|---|---|---|---|---|---|---|---|---|---|---|---|---|---|---|---|
| | | Type | Amonunt (part) | HOMO value (eV) | Type | Amonuntt (part) | HOMO value (eV) | Amount (part) | Type | Amount (part) | eV | S/cm | μW/K | μW/(mK$^2$) | Evaluation |
| Exemple 38 | Dispersion 38 | Cu powder | 0.4 | -4.59 | B166 | 0.08 | -5.28 | 20% | Binder Resin 1 | 0.05 | 0.69 | 174 | 24.0 | 10.02 | B |
| Comparative Exemple 1 | Dispersion 101 | SWCNT | 0.4 | -5.1 | Coloring Matter Introduced Polymer I | 0.4 | -6.77 | - | - | - | 1.67 | 10 | 50.0 | 2.45 | D |
| Comparative Exemple 2 | Dispersion 102 | - | 0 | - | B30 | 0.8 | -5.49 | - | - | - | - | $2.1 \times 10^{-8}$ | 25000 | 0.0 | D |

[Table 56]

| Table 56 | Thermoelectric Conversion Material Dispersion | Conductive Material (A) | | | Organic Compound (B) | | | | NMP | Other Components | | ⊿HOMO | electrical conductivity | Seebeck Coefficient | PowerFactor PF | |
|---|---|---|---|---|---|---|---|---|---|---|---|---|---|---|---|---|
| | | Type | Amount (part) | HOMO value (eV) | Type | Amount (part) | HOMO value (eV) | Amount relative to Conductive Material (A) (%) | Amount (part) | Type | Amount (part) | eV | S/cm | μW/K | μW/(mK$^2$) | Evaluation |
| Example 77 | Dispersion 77 | SWCNT | 0.4 | -5.1 | B248 | 0.4 | -5.28 | 100% | 79.2 | - | 0 | 0.18 | 48 | 65.2 | 20.40 | A |
| Example 78 | Dispersion 78 | SWCNT | 0.4 | -5.1 | B242 | 0.4 | -5.62 | 100% | 79.2 | - | 0 | 0.52 | 43 | 64.2 | 17.72 | B |
| Example 79 | Dispersion 79 | SWCNT | 0.4 | -5.1 | B254 | 0.4 | -5.03 | 100% | 79.2 | - | 0 | 0.07 | 43 | 58.8 | 14.87 | B |
| Example 80 | Dispersion 80 | SWCNT | 0.4 | -5.1 | B250 | 0.4 | -6.02 | 100% | 79.2 | - | 0 | 0.92 | 39 | 69.5 | 18.84 | B |
| Example 81 | Dispersion 81 | SWCNT | 0.4 | -5.1 | B257 | 0.4 | -5.27 | 100% | 79.2 | - | 0 | 0.17 | 49 | 64.8 | 20.58 | A |
| Example 82 | Dispersion 82 | SWCNT | 0.4 | -5.1 | B277 | 0.4 | -5.18 | 100% | 79.2 | - | 0 | 0.08 | 48 | 59.1 | 16.77 | B |
| Example 83 | Dispersion 83 | SWCNT | 0.4 | -5.1 | B351 | 0.4 | -5.52 | 100% | 79.2 | - | 0 | 0.42 | 45 | 57.9 | 15.09 | B |
| Example 84 | Dispersion 84 | SWCNT | 0.4 | -5.1 | B339 | 0.4 | -5.22 | 100% | 79.2 | - | 0 | 0.12 | 36 | 60.1 | 13.00 | B |
| Example 85 | Dispersion 85 | SWCNT | 0.4 | -5.1 | B375 | 0.4 | -5.71 | 100% | 79.2 | - | 0 | 0.61 | 53 | 54.2 | 15.57 | B |

[Table 57]

| Table 57 | Thermoelectric Conversion Material Dispersion | Conductive Material (A) | | | Organic Compound (B) | | | | NMP | Other Components | | ⊿HOMO | electrical conductivity | Seebeck Coefficient | PowerFactor PF | |
|---|---|---|---|---|---|---|---|---|---|---|---|---|---|---|---|---|
| | | Type | Amount (part) | HOMO value (eV) | Type | Amount (part) | HOMO value (eV) | Amount relative to Conductive Material (A) (%) | Amount (part) | Type | Amount (part) | eV | S/cm | $\mu$W/K | $\mu$W/(mK$^2$) | Evaluation |
| Example 95 | Dispersion 200 | SWCNT | 0.4 | -5.1 | B394 | 0.4 | -5.87 | 100% | 79.2 | - | 0 | 0.77 | 45 | 58.2 | 15.30 | B |
| Example 96 | Dispersion 201 | SWCNT | 0.4 | -5.1 | B395 | 0.4 | -5.98 | 100% | 79.2 | - | 0 | 0.88 | 43 | 59.1 | 15.02 | B |
| Example 97 | Dispersion 202 | SWCNT | 0.4 | -5.1 | B415 | 0.4 | -6.03 | 100% | 79.2 | - | 0 | 0.93 | 48 | 60.8 | 17.74 | B |
| Example 98 | Dispersion 203 | SWCNT | 0.4 | -5.1 | B442 | 0.4 | -5.57 | 100% | 79.2 | - | 0 | 0.47 | 46 | 59.9 | 16.50 | B |
| Example 99 | Dispersion 204 | SWCNT | 0.4 | -5.1 | B445 | 0.4 | -5.58 | 100% | 79.2 | - | 0 | 0.48 | 45 | 59.8 | 16.09 | B |
| Example 100 | Dispersion 205 | SWCNT | 0.4 | -5.1 | B452 | 0.4 | -5.52 | 100% | 79.2 | - | 0 | 0.42 | 25 | 52.0 | 6.76 | C |
| Example 101 | Dispersion 206 | SWCNT | 0.4 | -5.1 | B470 | 0.4 | -5.55 | 100% | 79.2 | - | 0 | 0.45 | 48 | 66.5 | 21.22 | A |
| Example 102 | Dispersion 207 | SWCNT | 0.4 | -5.1 | B497 | 0.4 | -5.33 | 100% | 79.2 | - | 0 | 0.23 | 47 | 66.4 | 20.72 | A |
| Example 103 | Dispersion 208 | SWCNT | 0.4 | -5.1 | B525 | 0.4 | -5.39 | 100% | 79.2 | - | 0 | 0.29 | 49 | -56.3 | 15.37 | B |
| Example 106 | Dispersion 211 | SWCNT | 0.4 | -5.1 | B583 | 0.4 | -6.42 | 100% | 79.2 | - | 0 | 1.32 | 37 | -60.3 | 13.45 | B |
| Example 107 | Dispersion 212 | SWCNT | 0.4 | -5.1 | B594 | 0.4 | -6.49 | 100% | 79.2 | - | 0 | 1.39 | 35 | -65.0 | 14.79 | B |
| Example 108 | Dispersion 213 | SWCNT | 0.4 | -5.1 | B595 | 0.4 | -6.51 | 100% | 79.2 | - | 0 | 1.41 | 32 | 45.0 | 6.48 | C |

The abbreviations shown in in Tables 55 and 56 are as follows.

GNP: graphene nanoplatelets "xGNP-M-5" manufactured by XGSciences
KB: Ketjen black "EC-300J" manufactured by Lion Corporation
Graphite: graphite "CPB" manufactured by Nippon Graphite Industries, Co., Ltd.
MWCNT: multi-walled carbon nanotube "100P" manufactured by Knano
SWCNT: single-walled carbon nanotube "TUBALL" manufactured by Kusumoto Chemicals, Ltd.
PEDOT/PSS: "Clevios PH1000" manufactured by by Heraeus
Ag powder: "FA-D-5" manufactured by DOWA
Cu powder: "2.5 μm-Type A" manufactured by DOWA
B375: [1,3]oxazolo[5,4-d][1,3]oxazole-2,5-dicarboxylic acid (oxazolooxazole manufactured by FCH Group)

(B375)

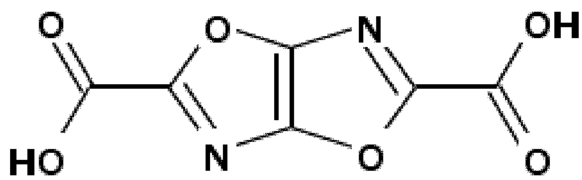

The abbreviations shown in Table 57 are as follows.

B394: 2-chlorothioxanthone (manufactured by Tokyo Chemical Industry Co., Ltd.)
B395: 2-(trifluoromethyl)thioxanthene-9-one (manufactured by Tokyo Chemical Industry Co., Ltd.)
B415: 2,4-diethylthioxanthene-9-one (manufactured by Tokyo Chemical Industry Co., Ltd.)
B442: 10-methylphenothiazine (manufactured by Tokyo Chemical Industry Co., Ltd.)
B445: 10-phenylphenothiazine (manufactured by Tokyo Chemical Industry Co., Ltd.)
B452: phenothiazine (manufactured by Tokyo Chemical Industry Co., Ltd.)
B470: 10-hexylphenothiazine (manufactured by Tokyo Chemical Industry Co., Ltd.)
B497: leucomethylene blue (manufactured by Tokyo Chemical Industry Co., Ltd.)
B525: methylene blue (manufactured by Tokyo Chemical Industry Co., Ltd.)
B568: Basic Blue 17 (manufactured by Tokyo Chemical Industry Co., Ltd.)
B569: Basic Blue 24 (manufactured by Tokyo Chemical Industry Co., Ltd.)
B583: 4,7-dimethyl-1,10-phenanthroline hydrate (manufactured by Tokyo Chemical Industry Co., Ltd.)
B594: basophenanthroline (manufactured by Tokyo Chemical Industry Co., Ltd.)
B595: bathocuproine (manufactured by Tokyo Chemical Industry Co., Ltd.)

As shown in Table 55, Table 56, and Table 57, each layered product using a dispersion containing a thermoelectric conversion material of Examples shows the compatibility between the electrical conductivity and the Seebeck coefficient, and shows a high PF. When a single-walled carbon nanotube having high electrical conductivity was used, the PF was excellent, and in particular, when a single-walled carbon nanotube and an organic compound (B) having a small ΔHOMO were combined, the PF was higher (Examples 17, 18, 77, 81, 101, and 102).
On the other hand, in Comparative Example 1 using the coloring matter-introduced polymer 1, the ΔHOMO exceeded 1.64 and the density of the coloring matter unit (a perylene skeleton) for improving the Seebeck coefficient was low, which caused a decrease in the Seebeck coefficient and resulted in a low PF. In Comparative Example 2, which did not contain a carbon material, the electrical conductivity decreased and the PF was low.

<Production of Thermoelectric Conversion Elements>

[Example 39]

(Thermoelectric Conversion Element 1)

Onto a 50 μm PET film, the dispersion 1 containing a thermoelectric conversion material prepared in Example 1 was applied, and five thermoelectric conversion films each having a shape of 5 mm × 30 mm were formed at 10 mm intervals (refer to reference sign 2 in FIG. 1). Then, using a silver paste, four silver circuits each having a shape of 5 mm × 33 mm were fabricated (refer to reference sign 3 in FIG. 1) so that the thermoelectric conversion films were

connected in series, whereby the thermoelectric conversion element 1 was obtained. As the silver paste, REXALPHA RA FS 074 manufactured by Toyochem Co., Ltd. was used.

[Examples 40 to 76 and 86 to 94, and Comparative Example 3]

(Thermoelectric Conversion Elements 2 to 38, 77 to 85 and 101)

With the exception of changing the dispersion containing the thermoelectric conversion material used in the thermoelectric conversion element 1 to the dispersion shown in Tables 58 and 59, thermoelectric conversion elements 2 to 38, 77 to 85, and 101 were manufactured in the same manner as the thermoelectric conversion element 1.

[Example 109 to 121]

(Thermoelectric Conversion Element 200 to 213)

With the exception of changing the dispersion containing the thermoelectric conversion material used in the thermoelectric conversion element 1 to the dispersion shown in Table 60, the thermoelectric conversion elements 200 to 213 were manufactured in the same manner as the thermoelectric conversion element 1.

<Evaluation of Thermoelectric Conversion Elements>

The obtained thermoelectric conversion elements were evaluated as follows. The results are shown in Tables 58, 59, and 60.

(Measurement of Electromotive Force)

Each thermoelectric conversion element was placed on a hot plate heated to 100°C while being bent (along the A-A' line shown in Figure 2) so that the thermoelectric conversion film and the silver circuit were inside. The degree of bending was adjusted so that the distance between B and B' in FIG. 2 is 10 mm. The electromotive force between the coating films was measured using a voltmeter after 10 minutes of setting the bent sample on a hot plate. The measurements were performed at room temperature (20°C). The thermoelectric properties were evaluated based on the measured values according to the following criteria.

A: the electromotive force is at least 1 mV (good)
B: the electromotive force is at least 500 μV but lower than 1 mV (practically usable)
C: the electromotive force is lower than 500 μV (poor)

[Table 58]

| Table 58 | Thermoelectric Conversion Element | Thermoelectric Conversion Material Dispersion | Electromotive Force |
|---|---|---|---|
| Example 39 | Thermoelectric Conversion Element 1 | Dispersion 1 | B |
| Example 40 | Thermoelectric Conversion Element 2 | Dispersion 2 | B |
| Example 41 | Thermoelectric Conversion Element 3 | Dispersion 3 | B |
| Example 42 | Thermoelectric Conversion Element 4 | Dispersion 4 | B |
| Example 43 | Thermoelectric Conversion Element 5 | Dispersion 5 | B |
| Example 44 | Thermoelectric Conversion Element 6 | Dispersion 6 | B |

(continued)

| Table 58 | Thermoelectric Conversion Element | Thermoelectric Conversion Material Dispersion | Electromotive Force |
|---|---|---|---|
| Example 45 | Thermoelectric Conversion Element 7 | Dispersion 7 | B |
| Example 46 | Thermoelectric Conversion Element 8 | Dispersion 8 | B |
| Example 47 | Thermoelectric Conversion Element 9 | Dispersion 9 | B |
| Example 48 | Thermoelectric Conversion Element 10 | Dispersion 10 | A |
| Example 49 | Thermoelectric Conversion Element 11 | Dispersion 11 | A |
| Example 50 | Thermoelectric Conversion Element 12 | Dispersion 12 | A |
| Example 51 | Thermoelectric Conversion Element 13 | Dispersion 13 | A |
| Example 52 | Thermoelectric Conversion Element 14 | Dispersion 14 | B |
| Example 53 | Thermoelectric Conversion Element 15 | Dispersion 1 5 | A |
| Example 54 | Thermoelectric Conversion Element 16 | Dispersion 16 | A |
| Example 55 | Thermoelectric Conversion Element 17 | Dispersion 17 | A |
| Example 56 | Thermoelectric Conversion Element 18 | Dispersion 18 | A |
| Example 57 | Thermoelectric Conversion Element 19 | Dispersion 19 | A |
| Example 58 | Thermoelectric Conversion Element 20 | Dispersion 20 | B |
| Example 59 | Thermoelectric Conversion Element 21 | Dispersion 21 | A |
| Example 60 | Thermoelectric Conversion Element 22 | Dispersion 22 | A |
| Example 61 | Thermoelectric Conversion Element 23 | Dispersion 23 | A |
| Example 62 | Thermoelectric Conversion Element 24 | Dispersion 24 | A |
| Example 63 | Thermoelectric Conversion Element 25 | Dispersion 25 | A |
| Example 64 | Thermoelectric Conversion Element 26 | Dispersion 26 | A |
| Example 65 | Thermoelectric Conversion Element 27 | Dispersion 27 | A |
| Example 66 | Thermoelectric Conversion Element 28 | Dispersion 28 | B |

(continued)

| Table 58 | Thermoelectric Conversion Element | Thermoelectric Conversion Material Dispersion | Electromotive Force |
|---|---|---|---|
| Example 67 | Thermoelectric Conversion Element 29 | Dispersion 29 | B |
| Example 68 | Thermoelectric Conversion Element 30 | Dispersion 30 | A |
| Example 69 | Thermoelectric Conversion Element 31 | Dispersion 31 | A |
| Example 70 | Thermoelectric Conversion Element 32 | Dispersion 32 | A |
| Example 71 | Thermoelectric Conversion Element 33 | Dispersion 33 | A |
| Example 72 | Thermoelectric Conversion Element 34 | Dispersion 34 | A |
| Example 73 | Thermoelectric Conversion Element 35 | Dispersion 35 | A |
| Example 74 | Thermoelectric Conversion Element 36 | Dispersion 36 | B |
| Example 75 | Thermoelectric Conversion Element 37 | Dispersion 37 | B |
| Example 76 | Thermoelectric Conversion Element 38 | Dispersion 38 | A |
| Comparative Example 3 | Thermoelectric Conversion Element 101 | Dispersion 101 | C |

[Table 59]

| Table 59 | Thermoelectric Conversion Element | Thermoelectric Conversion Material Dispersion | Electromotive Force |
|---|---|---|---|
| Example 86 | Thermoelectric Conversion Element 77 | Dispersion 77 | A |
| Example 87 | Thermoelectric Conversion Element 78 | Dispersion 78 | A |
| Example 88 | Thermoelectric Conversion Element 79 | Dispersion 79 | A |
| Example 89 | Thermoelectric Conversion Element 80 | Dispersion 80 | A |
| Example 90 | Thermoelectric Conversion Element 81 | Dispersion 81 | A |
| Example 91 | Thermoelectric Conversion Element 82 | Dispersion 82 | A |
| Example 92 | Thermoelectric Conversion Element 83 | Dispersion 83 | B |
| Example 93 | Thermoelectric Conversion Element 84 | Dispersion 84 | B |

(continued)

| Table 59 | Thermoelectric Conversion Element | Thermoelectric Conversion Material Dispersion | Electromotive Force |
|---|---|---|---|
| Example 94 | Thermoelectric Conversion Element 85 | Dispersion 85 | B |

[Table 60]

| Table 60 | Thermoelectric Conversion Element | Thermoelectric Conversion Material Dispersion | Electromotive Force |
|---|---|---|---|
| Example 109 | Thermoelectric Conversion Element 200 | Dispersion 200 | B |
| Example 110 | Thermoelectric Conversion Element 201 | Dispersion 201 | B |
| Example 111 | Thermoelectric Conversion Element 202 | Dispersion 202 | B |
| Example 112 | Thermoelectric Conversion Element 203 | Dispersion 203 | B |
| Example 113 | Thermoelectric Conversion Element 204 | Dispersion 204 | B |
| Example 114 | Thermoelectric Conversion Element 205 | Dispersion 205 | B |
| Example 115 | Thermoelectric Conversion Element 206 | Dispersion 206 | A |
| Example 116 | Thermoelectric Conversion Element 207 | Dispersion 207 | A |
| Example 117 | Thermoelectric Conversion Element 208 | Dispersion 208 | B |
| Example 118 | Thermoelectric Conversion Element 209 | Dispersion 209 | B |
| Example 119 | Thermoelectric Conversion Element 210 | Dispersion 210 | B |
| Example 120 | Thermoelectric Conversion Element 211 | Dispersion 211 | B |
| Example 121 | Thermoelectric Conversion Element 212 | Dispersion 212 | B |
| Example 122 | Thermoelectric Conversion Element 213 | Dispersion 213 | B |

As shown in Table 58, Table 59, and Table 60, the thermoelectric conversion element of each Example had superior thermoelectric properties as compared with Comparative Example 3. Accordingly, it has been found that according to an embodiment of the present invention, a thermoelectric conversion element including a dispersion containing a thermoelectric conversion material having excellent Seebeck coefficient and electroconductive properties, exhibiting a high PF, and having excellent thermoelectric properties can be realized, and a highly efficient thermoelectric conversion element can be realized.

[Second Experiment Example]

<Method for Measuring Adsorption>

The adsorption to the electrically conductive material (A) was measured by the following method. First, 55 parts of NMP and 0.001 parts of the organic compound (B) (or the organic compound (C)) were weighed, and they were mixed and completely dissolved (which is referred to as "liquid a"). Further, 0.0025 parts of the electrically conductive material (A) was added, stirring was performed for 24 hours, and a filtrate from which the electrically conductive material (A) was removed by a filter (which is also referred to as "liquid b") was obtained. Using a spectrophotometer (U-4100, manufactured by Hitachi High Technologies Corporation), the absorption spectrum of each of the liquid a and the liquid b was measured at 25°C in the wavelength range of from 300 to 800 nm. The ratio of the adsorption of the organic compound (B) (or the organic compound (C)) to the electrically conductive material (A) was calculated according to the following expression, and the evaluation of the adsorptivity was classified as follows.

Expression

the ratio of the adsorption (%) of the organic compound (B) (or the organic compound (C)) to the electrically conductive material (A) = ((the absorbance at the maximum absorption wavelength of the liquid a - the absorbance at the maximum absorption wavelength of the liquid b) ÷ the absorbance at the maximum absorption wavelength of the liquid a) × 100

(Measurement condition)

Solvent: NMP
Cell: quartz cell
Optical path length: 10 mm

(Evaluation)

AD1: the adsorption ratio is at least 0% but lower than 25%
AD2: the adsorption ratio is at least 25% but lower than 50%
AD3: the adsorption ratio is at least 50% but lower than 75%
AD4: the adsorption ratio is at least 75%

<Method for Measuring Homo and Fermi Level>

The HOMO of each of the electrically conductive material (A), the organic compound (B), and the organic compound (C) was measured by photoelectron spectroscopy (AC-2, manufactured by Riken Keiki Co., Ltd.) after fixing each single component on an electrically conductive tape adhered onto an ITO glass substrate as a measurement sample. The measured values are shown in Table 61.

<Production of Thermoelectric Conversion Materials>

[Example 1]

(Dispersion 1)

Pigment red 255 (manufactured by Tokyo Chemical Industry Co., Ltd.) in 0.2 parts, 2-isopropylthioxanthone (manufactured by Tokyo Chemical Industry Co., Ltd.) in 0.2 parts, SWCNT (manufactured by OCSiAl) in 0.4 parts, and NMP in 79.2 parts each were weighed and they were mixed together. Thus, a dispersion 1 containing a thermoelectric conversion material was obtained.

[Examples 2 to 16, Comparative Example 1]

(Dispersions 2 to 17)

With the exception of changing the components and the amounts to those shown in Table 61, dispersions 2 to 17 containing a thermoelectric conversion material was prepared in the same manner as the dispersion 1.

[Table 61]

| Table 61 | Dispersion | Conductive Material (A) | | | Organic Compound (B) | | | | Organic Compound (C) | | | | Solvent | Electromotive Force* |
|---|---|---|---|---|---|---|---|---|---|---|---|---|---|---|
| | | Type | Amount (part) | HOMO value (eV) | Type | Amount (part) | HOMO value (eV) | Adsorption to Conductive Material (A) | Type | Amount (part) | HOMO value (eV) | Adsorption to Conductive Material (A) | Amount (part) | (times) |
| Example 1 | Dispersion 1 | SWCNT | 0.4 | -5.1 | B1 | 0.2 | -5.4 | AD4 | C1 | 0.2 | -6.0 | AD1 | 79.2 | 2.0 |
| Example 2 | Dispersion 2 | Graphite | 0.4 | -5.2 | B1 | 0.2 | -5.4 | AD4 | C1 | 0.2 | -6.0 | AD1 | 79.2 | 1.5 |
| Example 3 | Dispersion 3 | GNP | 0.4 | -5.1 | B1 | 0.2 | -5.4 | AD4 | C1 | 0.2 | -6.0 | AD1 | 79.2 | 1.6 |
| Example 4 | Dispersion 4 | MWCNT | 0.4 | -5.1 | B1 | 0.2 | -5.4 | AD4 | C1 | 0.2 | -6.0 | AD1 | 79.2 | 1.8 |
| Example 5 | Dispersion 5 | SWCNT | 0.4 | -5.1 | B2 | 0.2 | -5.6 | AD2 | C2 | 0.2 | -6.2 | AD1 | 79.2 | 1.5 |
| Example 6 | Dispersion 6 | SWCNT | 0.4 | -5.1 | B1 | 0.2 | -5.4 | AD4 | C3 | 0.2 | -6.5 | AD3 | 79.2 | 2.0 |
| Example 7 | Dispersion 7 | SWCNT | 0.4 | -5.1 | B1 | 0.2 | -5.4 | AD4 | C2 | 0.2 | -6.2 | AD1 | 79.2 | 1.8 |
| Example 8 | Dispersion 8 | SWCNT | 0.4 | -5.1 | B3 | 0.2 | -5.4 | AD4 | C3 | 0.2 | -6.5 | AD3 | 79.2 | 1.7 |
| Example 9 | Dispersion 9 | SWCNT | 0.4 | -5.1 | B4 | 0.2 | -5.0 | AD4 | C4 | 0.2 | -4.9 | AD1 | 79.2 | 1.6 |
| Example 10 | Dispersion 10 | SWCNT | 0.4 | -5.1 | B4 | 0.2 | -5.0 | AD4 | C5 | 0.2 | -4.9 | AD2 | 79.2 | 1.4 |
| Example 11 | Dispersion 11 | SWCNT | 0.4 | -5.1 | B3 | 0.2 | -5.4 | AD4 | C3 | 0.02 | -6.5 | AD3 | 79.4 | 1.3 |
| Example 12 | Dispersion 12 | SWCNT | 0.4 | -5.1 | B3 | 0.08 | -5.4 | AD4 | C3 | 0.2 | -6.5 | AD3 | 79.4 | 1.4 |
| Example 13 | Dispersion 13 | SWCNT | 0.4 | -5.1 | B3 | 0.02 | -5.4 | AD4 | C3 | 0.2 | -6.5 | AD3 | 79.4 | 1.2 |
| Example 14 | Dispersion 14 | SWCNT | 0.4 | -5.1 | B3 | 0.2 | -5.4 | AD4 | C3 | 0.4 | -6.5 | AD3 | 79.0 | 1.7 |
| Example 15 | Dispersion 15 | SWCNT | 0.4 | -5.1 | B3 | 0.4 | -5.4 | AD4 | C3 | 0.2 | -6.5 | AD3 | 79.0 | 1.8 |
| Example 16 | Dispersion 16 | SWCNT | 0.4 | -5.1 | B2 | 0.4 | -5.4 | AD4 | C3 | 0.4 | -6.5 | AD3 | 78.8 | 1.8 |
| Comparative Example 1 | Dispersion 17 | SWCNT | 0.4 | -5.1 | B5 | 0.2 | -5.3 | AD1 | C6 | 0.2 | -5.6 | AD2 | 79.2 | 1 |

*) Relative value with the electromotive force of Comparative Example 1 as 1.

The abbreviations shown in Table 61 are as follows.

Conductive material (A)

GNP: graphene nanoplatelets "xGNP M5" manufactured by XG Sciences
Graphite: expanded graphite SMF manufactured by Chuetsu Graphite Works Co., Ltd.
MWCNT: multi-walled carbon nanotube "K-nanos-100 P" manufactured by KUMHO PETROCHEMICAL
SWCNT: single-walled carbon nanotube "TUBALL Nanotube" manufactured by OCSiAl Organic compounds (B) and (C)
B1: pigment red 255 (manufactured by Tokyo Chemical Industry Co., Ltd.)
B2: methylene green (manufactured by FUJIFILM Wako Pure Chemical Industries, Ltd.)
B3: 2,5-bis(2-ethylhexyl)3,6-di(2-thienyl)-2, 5-dihydropyrrolo[3,4-c]pyrrole-1,4-dione (manufactured by Tokyo Chemical Industry Co., Ltd.)
B5: N,N'-bis[4-(diphenylamino)phenyl]-N,N'-diphenylbenzidine (manufactured by Tokyo Chemical Industry Co., Ltd.)
C1: 2-isopropylthioxanthone (manufactured by Tokyo Chemical Industry Co., Ltd.)
C2: 2,2"-bi-9,9' spirobi[9H-fluorene] (manufactured by Aldrich)
C3: 2,5-diphenyl-1,3,4-oxadiazole (manufactured by Tokyo Chemical Industry Co., Ltd.)
C4: 1,4,8,11,15,18,22,25-octabutoxy-29H,31H-phthalocyanine (manufactured by Sigma-Aldrich)
C6: phloxin B (manufactured by Tokyo Chemical Industry Co., Ltd.)

<Synthesis of Organic Compounds>

(Synthesis Example 1: Organic Compound (B4))

In 400 g of amyl alcohol, 20.0 g of dimethyl succinate, 48.0 g of 4-(dimethylamino)benzonitrile and 31.6 g of sodium hydride were dissolved, and the resulting mixture was refluxed for 8 hours. After cooling, the precipitate was filtered and washed with acetic acid and methanol, and 11.8 g of a purple solid was obtained. Then, 10 g of the thus obtained solid, 15.6 g of iodomethane, and 10.3 g of sodium butoxide were dissolved in 300 g of dimethylacetamide and the obtained mixture was refluxed for 8 hours. After cooling, the mixture was added to 1000 ml of methanol, a solid was precipitated, collected by filtration, and purified by column chromatography with silica gel. Thus, 8.3 g of an organic compound (B4) was obtained.

Organic Compound (B4)

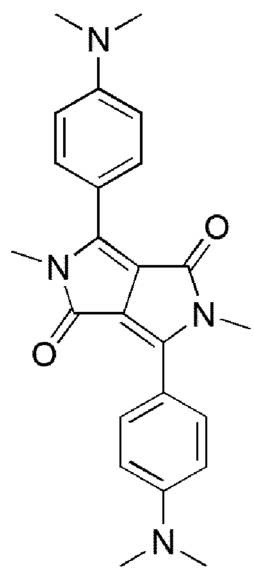

(Synthesis Example 2: Organic Compound (C5))

In 20 ml of nitrobenzene, 5.0 g of 3-aminoperylene, 15.9 g of 3-bromo-(m-tolyl)-9H-carbazole, 1.5 g of sodium

hydroxide, and 1.0 g of copper oxide were added, and the mixture was heated and stirred at 200°C for 50 hours under a nitrogen atmosphere. After cooling, the mixture was diluted with 500 ml of water and was subjected to extraction with toluene. After concentrating the extract liquid, purification was performed using column chromatography with silica gel. Thus 7.2 g of an organic compound (C5) was obtained.

Organic Compound (C5)

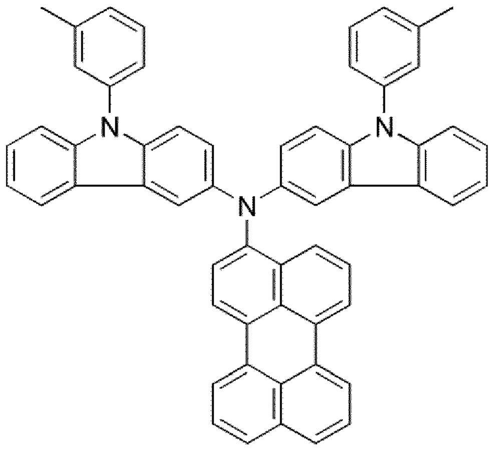

Each of the dispersions 1 to 17 was applied to a polyimide film of 50 μm in thickness, which was a sheet-like substrate, using an applicator, followed by heating and drying at 120°C for 30 minutes. Thus, a layered product having a thermoelectric conversion film of 3 μm in thickness on the polyimide substrate was obtained. The thus obtained layered product was cut to a size of 1 cm × 5 cm, and, using a silver paste, an electrode having a thickness of 10 μm and a shape of 1 cm × 1 cm was prepared so as to be electrically connected to both ends of the layered product. Thus, a thermoelectric conversion element was obtained. Each thermoelectric conversion element was placed on a hot plate heated to 80°C while being bent (along the A-A' line shown in Figure 1) so that the thermoelectric conversion film and the silver circuit were inside. The degree of bending was adjusted so that the distance between B and B' in FIG. 2 is 10 mm. The electromotive force (mV) between the coating films was measured using an electromotive force meter (KEITHLEY 2400 manufactured by Tektronix) after 10 minutes of setting the bent sample on a hot plate. The measurements were performed at 20°C. When the absolute value of the electromotive force of Comparative Example 1 is 1, the relative value with the absolute value of the electromotive force in each Example is shown in Table 61.

As shown in Table 61, the thermoelectric conversion elements of the Examples showed a high electromotive force. The mechanism of thermoelectric conversion in this experimental example is thought as follows. It is suggested that when the adsorptivity to the electrically conductive material (A) is larger in the organic compound (B) than in the organic compound (C), the organic compound (B) is preferentially adsorbed on the surface of the electrically conductive material (A) than the organic compound (C). It is suggested that, for the generation of thermoelectric conversion, the carrier movement among the electrically conductive material (A), the organic compound (B), and the organic compound (C) is required to occur, and that if the HOMO value of the organic compound (B) is closer to the HOMO value of the electrically conductive material (A) than the HOMO value of the organic compound (C), then the carrier movement between the electrically conductive material (A) and the organic compound (B) existing in the vicinity of the surface becomes smooth, and the carrier movement between the organic compound (B) and the organic compound (C) also becomes smooth, resulting in efficient carrier movement among the electrically conductive material (A), the organic compound (B), and the organic compound (C), thus enhancing the thermoelectric conversion efficiency. On the other hand, in Comparative Example 1, the adsorptivity of the organic compound (B) to the electrically conductive material (A) is greater than the adsorptivity of the organic compound (C) to the electrically conductive material (A), and the HOMO value of the organic compound (B) is further away from the HOMO value of the electrically conductive material (A) than the HOMO value of the organic compound (C). It is suggested that as a result, the requirements (1) and (2) above are satisfied, but the requirement (3) is not satisfied, and, due to this, the movement efficiency of the carrier (electron or hole) between the organic compounds (C) becomes low, thereby showing a low electromotive force.

INDUSTRIAL APPLICABILITY

Using a thermoelectric conversion material according to embodiments of the present invention, a thermoelectric conversion element achieving compatibility between the electroconductive properties and the Seebeck coefficient, and having excellent thermoelectric properties can be provided.

DESCRIPTION OF REFERENCE SIGNS

- 1: Substrate (PET film)
- 2: Thermoelectric conversion film
- 3: Circuit
- 10: Test sample of thermoelectric conversion element
- 20: Hot Plate

## Claims

1. A thermoelectric conversion material comprising at least one electrically conductive material selected from the group consisting of a carbon material, a metal material and an electrically conductive polymer, and an organic compound that is different from the electrically conductive material, wherein the electrically conductive material and the organic compound satisfy the following expression (1):

Expression (1)

$$0\ \mathrm{eV} \le \left| \text{(HOMO of the organic compound)} - \text{(HOMO of the electrically conductive material)} \right| \le 1.64\ \mathrm{eV}$$

wherein, in expression (1), HOMO represents an energy level of a highest occupied molecular orbital, provided that in a case in which the electrically conductive material is a metal material, the HOMO of the electrically conductive material represents a Fermi level of the electrically conductive material.

2. The thermoelectric conversion material according to Claim 1, wherein the HOMO of the electrically conductive material is an energy level higher than the HOMO of the organic compound.

3. The thermoelectric conversion material according to Claim 1 or 2, wherein an amount of the organic compound is not more than 400% by mass relative to a total mass of the electrically conductive material.

4. The thermoelectric conversion material according to any one of Claims 1 to 3, wherein the organic compound is a compound having any one selected from the group consisting of a perylene skeleton, a pyrrolopyrrole skeleton, a thiazolothiazole skeleton, an oxazolothiazole skeleton, an oxazolooxazole skeleton, a benzobisthiazole skeleton, a benzobisoxazole skeleton, a thiazolobenzoxazole skeleton, and a thioxanthone skeleton, a phenothiazine skeleton, and a phenanthroline skeleton, provided that the organic compound is different from a compound having a perylene carbodiimide skeleton and is different from a compound represented by the following structural formula X.

Structural Formula X

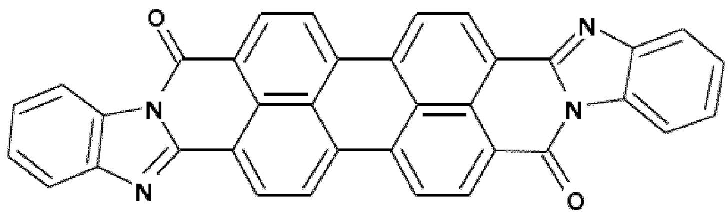

5. The thermoelectric conversion material according to any one of Claims 1 to 4, wherein the organic compound is a compound represented by any one selected from the group consisting of the following general formulae (1) to (8):

General Formula (1)

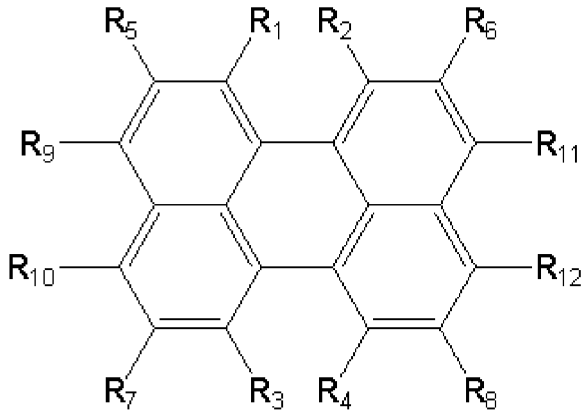

wherein, in general formula (1), each of $R_1$ to $R_{12}$ independently represents any one selected from the group consisting of a hydrogen atom, a halogen atom, a cyano group, a nitro group, a substituted or unsubstituted alkyl group, a substituted or unsubstituted alkoxy group, a substituted or unsubstituted aryloxy group, a substituted or unsubstituted alkylthio group, a substituted or unsubstituted arylthio group, a substituted or unsubstituted aryl group, a substituted or unsubstituted heterocyclic group, and a substituted or unsubstituted amino group, and adjacent two groups of $R_1$ to $R_{12}$ may be bonded to each other to form a ring;

General Formula (2)

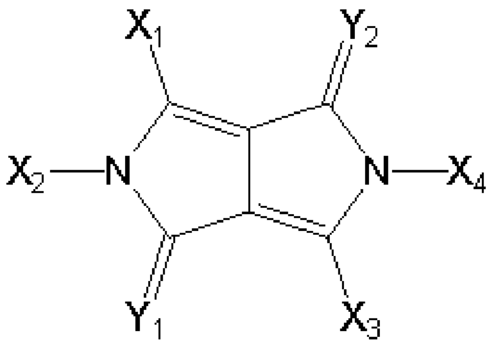

wherein, in general formula (2), each of $X_1$ to $X_4$ independently represents any one selected from the group consisting of a hydrogen atom, a substituted or unsubstituted alkyl group, a substituted or unsubstituted aryl group, and a substituted or unsubstituted heterocyclic group, and each of $Y_1$ and $Y_2$ independently represents any one selected from the group consisting of an oxygen atom, a sulfur atom, and a dicyanomethylene group;

General Formula (3)

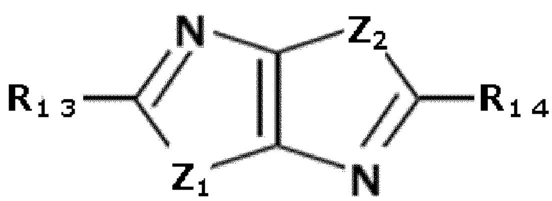

wherein, in general formula (3), each of $Z_1$ and $Z_2$ independently represents any one selected from the group consisting of an oxygen atom and a sulfur atom, and each of $R_{13}$ and $R_{14}$ independently represents any one selected from the group consisting of a hydrogen atom, a halogen atom, a cyano group, a nitro group, a carboxyl group, a substituted or unsubstituted alkyl group, a substituted or unsubstituted alkoxy group, a substituted or unsubstituted aryloxy group, a substituted or unsubstituted alkylthio group, a substituted or unsubstituted arylthio group, a substituted or unsubstituted aryl group, a substituted or unsubstituted heterocyclic group, and a substituted or unsubstituted

amino group;

General Formula (4)

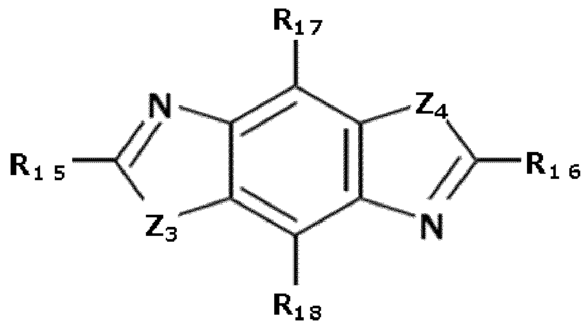

wherein, in general formula (4), each of $Z_3$ and $Z_4$ independently represents any one selected from the group consisting of an oxygen atom and a sulfur atom, and each of $R_{15}$ to $R_{18}$ independently represents any one selected from the group consisting of a hydrogen atom, a halogen atom, a cyano group, a nitro group, a carboxyl group, a substituted or unsubstituted alkyl group, a substituted or unsubstituted alkoxy group, a substituted or unsubstituted aryloxy group, a substituted or unsubstituted alkylthio group, a substituted or unsubstituted arylthio group, a substituted or unsubstituted aryl group, a substituted or unsubstituted heterocyclic group, and a substituted or unsubstituted amino group;

General Formula (5)

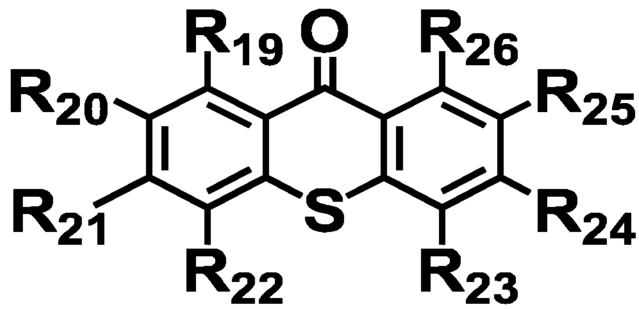

wherein, in general formula (5), each of $R_{19}$ to $R_{26}$ independently represents any one selected from the group consisting of a hydrogen atom, a halogen atom, a hydroxyl group, a sulfanyl group, a cyano group, a nitro group, a carboxyl group, an alkoxycarbonyl group, an acyl group, a substituted or unsubstituted alkyl group, a substituted or unsubstituted alkoxy group, a substituted or unsubstituted aryloxy group, a substituted or unsubstituted alkylthio group, a substituted or unsubstituted arylthio group, a substituted or unsubstituted aryl group, a substituted or unsubstituted heterocyclic group, a substituted or unsubstituted amino group, and a substituted or unsubstituted acyloxy group, provided that at least one of $R_{19}$ to $R_{26}$ is other than a hydrogen atom;

General Formula (6)

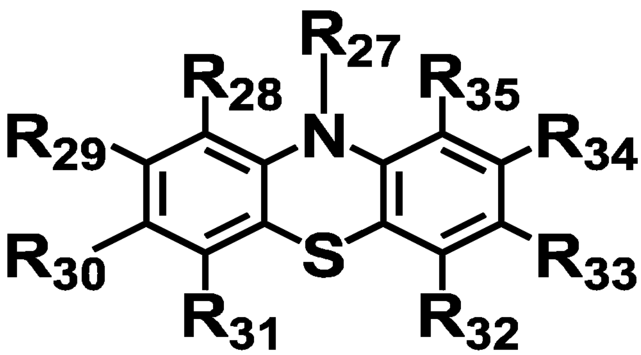

General Formula (7)

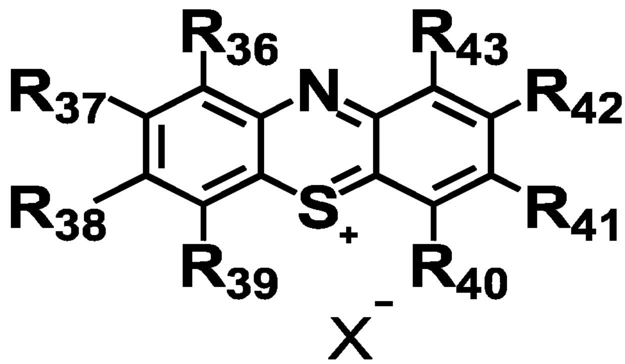

wherein, in general formula (6), $R_{27}$ represents any one selected from the group consisting of a hydrogen atom, a substituted or unsubstituted alkyl group, a substituted or unsubstituted aryl group, and a substituted or unsubstituted heterocyclic group; in general formulae (6) and (7), each of $R_{28}$ to $R_{43}$ independently represents any one selected from the group consisting of a hydrogen atom, a halogen atom, a hydroxyl group, a cyano group, a nitro group, a carboxyl group, a substituted or unsubstituted alkyl group, a substituted or unsubstituted alkoxy group, a substituted or unsubstituted aryloxy group, a substituted or unsubstituted alkylthio group, a substituted or unsubstituted arylthio group, a substituted or unsubstituted aryl group, a substituted or unsubstituted heterocyclic group, and a substituted or unsubstituted amino group; and in general formula (7), $X^-$ represents an anion;

General Formula (8)

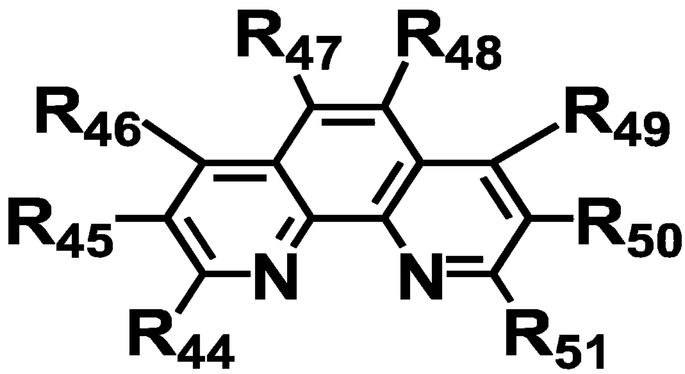

wherein, in general formula (8), each of $R_{44}$ to $R_{51}$ independently represents any one selected from the group consisting of a hydrogen atom, a halogen atom, a hydroxyl group, a cyano group, a nitro group, a carboxyl group, a sodium sulfonato group, a substituted or unsubstituted alkyl group, a substituted or unsubstituted alkoxy group, a substituted or unsubstituted aryloxy group, a substituted or unsubstituted alkylthio group, a substituted or unsubstituted arylthio group, a substituted or unsubstituted aryl group, a substituted or unsubstituted heterocyclic group, and a substituted or unsubstituted amino group, and adjacent two groups of $R_{44}$ to $R_{51}$ may be bonded to each other to form a ring.

**6.** The thermoelectric conversion material according to any one of Claims 1 to 5, wherein the electrically conductive material comprises at least one selected from the group consisting of a carbon nanotube, Ketjen black, a graphene nanoplate, and graphene.

**7.** The thermoelectric conversion material according to any one of Claims 1 to 6, wherein the electrically conductive material is a carbon nanotube.

**8.** A thermoelectric conversion material comprising an electrically conductive material, a first organic compound that is different from the electrically conductive material, and a second organic compound that is different from the electrically conductive material and that is different from the first organic compound, wherein the thermoelectric conversion material satisfies all of the following (1) to (3):

(1) 0 < ((HOMO of the first organic compound) - (HOMO of the electrically conductive material)) × ((HOMO of the second organic compound) - (HOMO of the electrically conductive material));
(2) | (HOMO of the first organic compound) - (HOMO of the electrically conductive material) | < | (HOMO of the second organic compound) - (HOMO of the electrically conductive material) |;
(3) an adsorptivity of the first organic compound to the electrically conductive material is greater than an adsorptivity of the second organic compound to the electrically conductive material;

wherein, in (1) to (3) above, HOMO represents an energy level of a highest occupied molecular orbital, provided that, in a case in which the electrically conductive material is a metal material, the HOMO of the electrically conductive material represents a Fermi level of the electrically conductive material.

**9.** The thermoelectric conversion material according to Claim 8, wherein the electrically conductive material comprises a carbon material.

**10.** The thermoelectric conversion material according to Claim 9, wherein the carbon material comprises a carbon nanotube.

**11.** A thermoelectric conversion element comprising a thermoelectric conversion film containing the thermoelectric conversion material according to any one of Claims 1 to 10, and an electrode, wherein the thermoelectric conversion film and the electrode are electrically connected to each other.

Figure 1

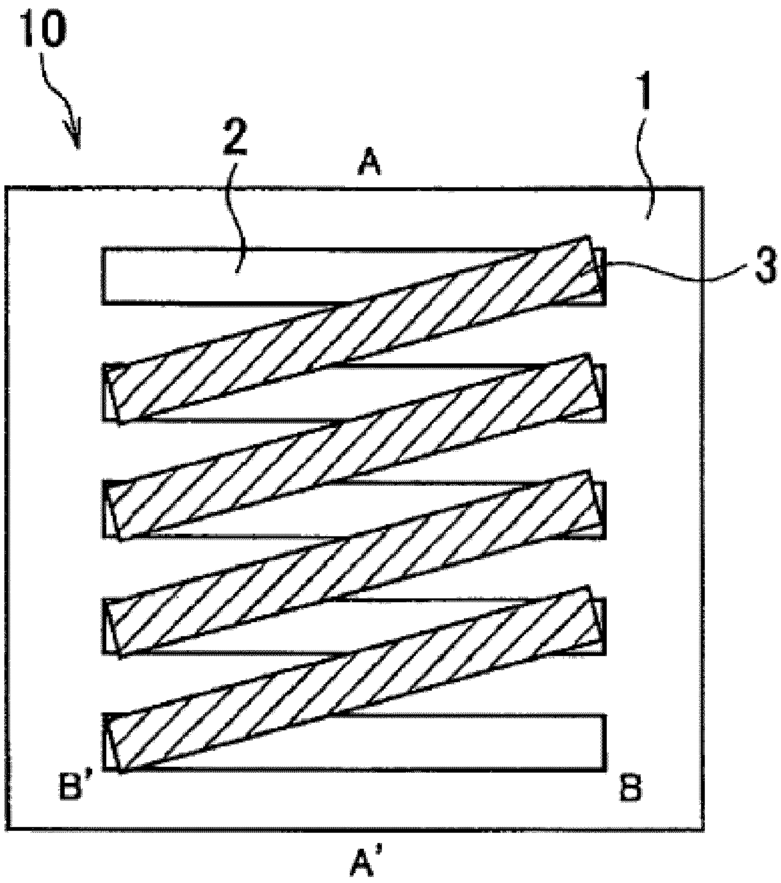


Figure 2

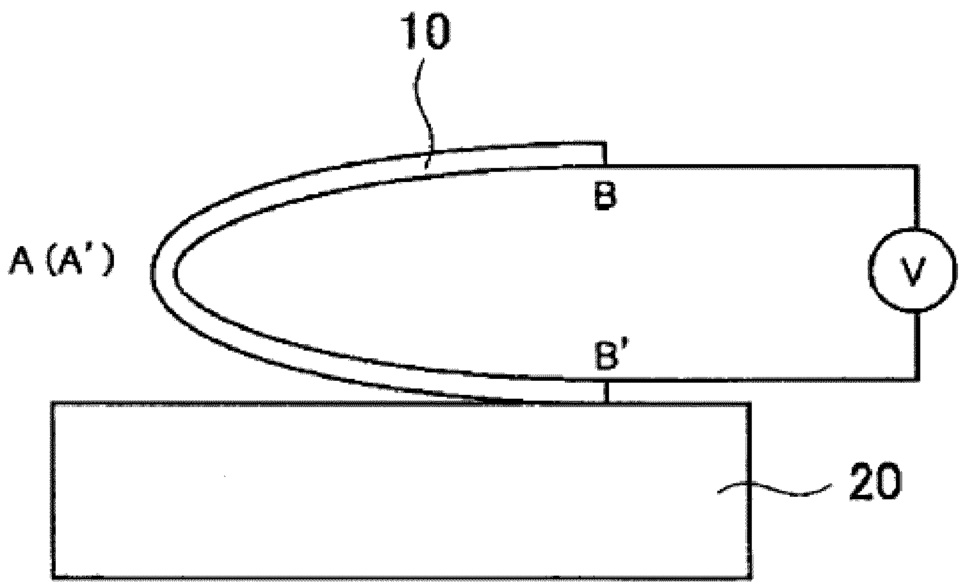

**INTERNATIONAL SEARCH REPORT**

International application No. PCT/JP2019/048905

A. CLASSIFICATION OF SUBJECT MATTER
H01L 35/24(2006.01)i; H01L 35/22(2006.01)i; H01L 51/00(2006.01)i; H01L 51/30(2006.01)i
FI: H01L35/24; H01L29/28 100Z; H01L29/28 250H; H01L35/22
According to International Patent Classification (IPC) or to both national classification and IPC

B. FIELDS SEARCHED
Minimum documentation searched (classification system followed by classification symbols)
H01L35/24; H01L35/22; H01L51/00; H01L51/30

Documentation searched other than minimum documentation to the extent that such documents are included in the fields searched

| | |
|---|---|
| Published examined utility model applications of Japan | 1922-1996 |
| Published unexamined utility model applications of Japan | 1971-2020 |
| Registered utility model specifications of Japan | 1996-2020 |
| Published registered utility model applications of Japan | 1994-2020 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X<br>A | WO 2014/133029 A1 (NARA INSTITUTE OF SCIENCE AND TECHNOLOGY) 04.09.2014 (2014-09-04) paragraphs [0043]-[0051], [0059], [0186]-[0193], fig. 4-5 | 1-3, 6-7<br>8-10 |
| Y<br>A | WO 2015/129877 A1 (NARA INSTITUTE OF SCIENCE AND TECHNOLOGY) 03.09.2015 (2015-09-03) paragraphs [0017]-[0033], [0049]-[0050], fig. 5-6 | 1-7, 11<br>8-10 |
| Y<br>A | JP 2015-092557 A (FUJIFILM CORPORATION) 14.05.2015 (2015-05-14) paragraphs [0029]-[0036], [0054] | 1-7, 11<br>8-10 |
| Y<br>A | WO 2014/156717 A1 (FUJIFILM CORPORATION) 02.10.2014 (2014-10-02) paragraphs [0027]-[0032] | 6<br>8-10 |

☐ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 03 March 2020 (03.03.2020) | 17 March 2020 (17.03.2020) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**
Information on patent family members

International application No.
PCT/JP2019/048905

| Patent Documents referred in the Report | Publication Date | Patent Family | Publication Date |
|---|---|---|---|
| WO 2014/133029 A1 | 04 Sep. 2014 | (Family: none) | |
| WO 2015/129877 A1 | 03 Sep. 2015 | CN 106165132 A<br>KR 10-2016-0127740 A<br>JP 2019-96892 A | |
| JP 2015-092557 A | 14 May 2015 | US 2016/0218268 A1<br>paragraphs [0066]-[0084], [0128]-[0132]<br>WO 2015/050077 A1 | |
| WO 2014/156717 A1 | 20 Oct. 2014 | US 2016/0013390 A1<br>paragraphs [0038]-[0045]<br>US 2017/0194547 A1<br>CN 105103316 A<br>TW 201444126 A<br>JP 2014-209531 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2015050113 A
- WO 2015129877 A


### Non-patent literature cited in the description


- **TAKENOBU KAJIKAWA.** Handbook of Thermoelectric Conversion Technology. NTS Inc, 19